# EUROPEAN PATENT APPLICATION

(11) **EP 4 252 845 A2**
(43) Date of publication of application: **04.10.2023**
(21) Application number: 23175111.6
(22) Date of filing: 05.07.2017
(51) Int. Cl.: A61P 21/00

(54) **PRE-MRNA SPLICE SWITCHING OR MODULATING OLIGONUCLEOTIDES COMPRISING BICYCLIC SCAFFOLD MOIETIES, WITH IMPROVED CHARACTERISTICS FOR THE TREATMENT OF GENETIC DISORDERS**

(30) Priority: 05.07.2016 EP 16177973
(62) Divisional of application: 17739231.3
(71) Applicant: BioMarin Technologies B.V., 2333 CH Leiden (NL)
(72) Inventor: VAN DEUTEKOM, Judith Christina Theodora, 3319 AJ Dordrecht (NL); DE VISSER, Peter Christian, 2324 DS Leiden (NL)
(74) Representative: Handley, Matthew Edward

(57) **Abstract**

The current invention provides antisense splice-switching oligonucleotides with improved characteristics that enhance clinical applicability for treating, ameliorating, preventing, and/or delaying neuromuscular disorders, such as DMD.

## Description

### Field

The invention relates to the field of antisense oligonucleotides, more specifically splice-switching oligonucleotides for the treatment of genetic disorders, more specifically neuromuscular disorders. The invention in particular relates to the use of oligonucleotides with improved characteristics enhancing clinical applicability as further defined herein.

### Background of the invention

Antisense oligonucleotides (AONs) are in (pre)clinical development for many diseases and conditions, including cancer, inflammatory conditions, cardiovascular disease and neurodegenerative and neuromuscular disorders. Their mechanism of action is aimed at various targets, such as RNaseH-mediated degradation of target RNA in the nucleus or cytoplasm, at splice-modulation (exon inclusion or skipping) in the nucleus, or at translation inhibition by steric hindrance of ribosomal subunit binding in the cytoplasm. Splice-modulating or splice-switching oligonucleotides (SSOs) were first described for correction of aberrant splicing in human β-globin pre-mRNAs (Dominski and Kole, 1993), and are currently being studied for a variety of genetic disorders including, but not limited to, cystic fibrosis (CFTR gene, Friedman et al., 1999), breast cancer (BRCA1 gene, Uchikawa et al., 2007), prostate cancer (FOLH1 gene, Williams et al., 2006), inflammatory diseases (IL-5Ralpha and MyD88 genes, Karras et al., 2001, Vickers et al., 2006), ocular albinism type 1 (OA1 gene, Vetrini et al., 2006), ataxia telangiectasia (ATM gene, Du et al., 2007), nevoid basal cell carcinoma syndrome (PTCH1 gene, Uchikawa et al., 2007), methylmalonic acidemia (MUT gene, Rincon et al., 2007), preterm labor (COX-2 gene, Tyson-Capper et al., 2006), artherosclerosis (APOB gene, Khoo et al., 2007), propionic acidemia (PCCA, PCCB genes, Rincon et al., 2007), leukemia (c-myc and WT1 genes, Renshawet al., 2004, Giles et al., 1999), dystrophic epidermolysis bullosa (COL7A1 gene, Goto et al., 2006), familial hypercholesterolemia (APOB gene, Disterer et al., 2013), laser-induced choroidal neovascularization and corneal graft rejection (KDR gene, Uehara et al., 2013), hypertrophic cardiomyopathy (MYBPC3 gene, Gedicke-Hornung et al., 2013), Usher syndrome (USH1C gene, Lentz et al., 2013), fukuyama congenital muscular dystrophy (FKTN gene, Taniguchi-Ikeda et al., 2011), laser-induced choroidal neovascularization (FLT1 gene, Owen et al., 2012), cancer (STAT3 and bcl-X genes, Zammarchi et al., 2011, Mercatante et al., 2002), and Hutchinson-Gilford progeria (LMNAgene, Osorio et al., 2011), Miyoshi myopathy (DYSF gene, Wein et al., 2010), spinocerebellar ataxia type 1 (ATXN1 gene, Gao et al., 2008), Alzheimer's disease/FTDP-17taupathies (MAPT gene, Peacey et al., 2012), myotonic dystrophy (CLC1 gene, Wheeler et al., 2007), and Huntington's disease (Evers et al., 2014). However, splice-switching AONs have progressed furthest in the treatment of the neuromuscular disorders Duchenne muscular dystrophy (DMD) an spinal muscular dystrophy (spinal muscular atrophy (SMA) type).

Duchenne muscular dystrophy (DMD) and Becker muscular dystrophy (BMD) are the most common childhood forms of muscular dystrophy. DMD is a severe, lethal neuromuscular disorder resulting in a dependency on wheelchair support before the age of 12 and patients often die before the age of thirty due to respiratory- or heart failure. It is caused by reading frame-shifting deletions (-67%) or duplications (-7%) of one or more exons, or by point mutations (-25%) in the 2.24 Mb *DMD* gene, resulting in the absence of functional dystrophin. BMD is also caused by mutations in the *DMD* gene, but these maintain the open reading frame, yield semi-functional dystrophin proteins, and result in a typically much milder phenotype and longer lifespan. During the last decade, specific modification of splicing in order to restore the disrupted reading frame of the transcript has emerged as a promising therapy for DMD (van Ommen et al., 2008; Yokota et al., 2007; van Deutekom et al., 2007; Goemans et al., 2011; Voit et al., 2014; Cirak et al., 2011). Using highly sequence-specific splice-switching antisense oligonucleotides (AONs) which bind to the exon flanking or containing the mutation and which interfere with its splicing signals, the skipping of that exon can be induced during the processing of the DMD pre-mRNA. Despite the resulting truncated transcript, the open reading frame is restored and a protein is produced which is similar to those found in BMD patients. AON-induced exon skipping provides a mutation-specific, and thus personalized, therapeutic approach for DMD patients. As the majority of the mutations cluster around exons 45 to 55, the skipping of one specific exon may be therapeutic for many patients with different mutations. The skipping of exon 51 applies to the largest subset of patients (-13%), including those with deletions of exons 45 to 50, 48 to 50, 50, or 52. The AONs applied are chemically modified to resist endonucleases, exonucleases, and RNaseH, and to promote RNA binding and duplex stability. Different AON chemistries are currently being explored for inducing corrective exon skipping for DMD, including 2'-O-methyl phosphorothioate RNA (2OMePS; Voit et al., 2014), phosphorodiamidate morpholino (PMO; Ciraket al., 2011), tricyclo DNA (tcDNA; Goyenvalle et al, 2015), and peptide nucleic acid (PNA; Gao et al., 2015). Although AONs are typically not well taken up by healthy muscle fibers, the dystrophin deficiency in DMD and the resulting pathology, characterized by activated satellite cells and damaged and thus more permeable fiber membranes, actually facilitates a better uptake. In studies in the dystrophin-deficient *mdx* mouse model, 2'-O-methyl phosphorothioate RNA oligonucleotides have indeed demonstrated an up to 10 times higher uptake in different muscle groups when compared to that in wild type mice (Heemskerk et al., 2010). Although the recent phase I/II results with both 2'-O-methyl phosphorothioate RNA and phosphorodiamidate morpholino AONs in DMD patients confirm presence of the AONs in muscle biopsies, the different chemical modifications seem to result in a differential uptake by and distribution throughout muscle. Furthermore, in both studies the levels of novel dystrophin after treatment were still limited, which challenges the field to develop oligonucleotides with improved characteristics enhancing therapeutic index and clinical applicability.

Spinal Muscular Atrophy (SMA) is an autosomal recessive disease affecting 1 in 6000 newborn children, and is caused by mutations in the survival of motor neuron gene 1 (*SMN1*). It results in progressive loss of motor neurons in the spinal cord and subsequent atrophy of voluntary muscles. Clinical severity depends on naturally occurring exon 7 inclusion levels in the almost identical *SMN2* gene and the number of copies of *SMN2.* In *SMN2,* a C>T transition in an exon 7 splicing enhancer site normally results in an unstable exon 7-skipped isoform and insufficient levels of full-length isoform (Khoo and Krainer, 2009). However, effective *SMN2* exon 7 inclusion can be achieved by blocking an intronic splicing silencer in the 5' region of intron 7 (ISS-N1; Singh *et al.,* 2006; Hua *et al*., 2008) with splice-switching AONs. On the basis of successful mouse studies (Hua *et al.,* 2010, 2011; Passini *et al.,* 2011) a candidate AON (ISIS-SMN_{Rx}, ISIS396443, or nusinersen) is currently in clinical development by IONIS Pharmaceuticals (Carlsbad, CA). In phase 1 studies, direct injection of a single 9-mg dose of ISIS-SMN_{Rx} into the intrathecal space resulted in broad distribution in the central nervous system and some motor function improvement in children with SMA (Swoboda *et al.,* 2014, Chiriboga et al., 2016). However, systemic SSO delivery may be needed to alleviate cardiac pathology and to increase peripheral motor neuron function and consequently survival.

Clinical efficacy of systemically administered AONs, such as splice-switching AONs, depends on multiple factors such as administration route, biostability, biodistribution, intra-tissue distribution, uptake by target cells, and routing to the desired intracellular location (nucleus). These factors are at least in part determined by the chemical structure of the AONs.. Part of the invention shows that certain chemical modifications in the AON scaffold can lead to AONs that show improved characteristics for possible treatment of genetic disorders.

In conclusion, to enhance the therapeutic applicability of AONs such as splice-switching AONs for genetic disorders such as DMD, BMD, or SMA, there is a need for AONs with optimized chemical characteristics.

### Description of the invention

### Oligonucleotide

In a first aspect, the invention provides an oligonucleotide comprising a 2'-substituted monomer, preferably a 2'-substituted RNA monomer, and a phosphorothioate backbone or consisting of 2'-substituted monomers, preferably of 2'-substituted RNA monomers, linked by phosphorothioate backbone linkages, and comprising a bicyclic nucleic acid (BNA) scaffold modification, and comprising a 5-methylpyrimidine base, preferably for use as a medicament for treating a disease or condition through splice modulation, such as through exon skipping, or exon inclusion, both of which are forms of splice switching. Preferred diseases in this context are Duchenne Muscular Dystrophy, Becker Muscular Dystrophy, and Spinal Muscular Atrophy.

For oligonucleotides as described in this application, when a feature of a monomer is not defined and is not apparent from context, the corresponding feature from an RNA monomer is to be assumed.

As such, in this aspect the invention provides an oligonucleotide comprising:
i)
   la) at least one 2'-substituted monomer and optionally a phosphorothioate backbone linkage, or
   Ib) only 2'-substituted monomers linked by phosphorothioate backbone linkages and/or by phosphodiester linkages,
ii) a 5-methylcytosine and/or a 5-methyluracil base and
iii) at least one monomer comprising a bicyclic nucleic acid (BNA) scaffold modification.

Preferably, said monomers are RNA monomers, or are derived from RNA monomers. Such oligonucleotides will be referred to herein as oligonucleotides according to the invention. As such, a preferred oligonucleotide according to the invention comprises:
i)
   la) at least one 2'-substituted monomer, preferably an RNA monomer or a 2'-*O*-substituted RNA monomer, and optionally a phosphorothioate backbone linkage, or
   Ib) only 2'-substituted monomers, preferably RNA monomers or 2'-*O-*substituted RNA monomers, linked by phosphorothioate backbone linkages and/or by phosphodiester linkages,
ii) a 5-methylcytosine and/or a 5-methyluracil base and
iii) at least one monomer comprising a bicyclic nucleic acid (BNA) scaffold modification,

Preferred oligonucleotides according to the invention have a length of less than 34 oligonucleotides. Said oligonucleotide may have 10, 11, 12, 13, 14, 15, 16, 17, 18, 19, 20, 21, 22, 23, 24, 25, 26, 27, 28, 29, 30, 31, 32, or 33 nucleotides. Such oligonucleotide may also be identified as an oligonucleotide having from 10 to 33 nucleotides. More preferred oligonucleotides according to the invention have a length of 16, 17, 18, 19, 20, 21, or 22 nucleotides, and may be identified as an oligonucleotide having from 16 to 22 nucleotides.

In another embodiment, the oligonucleotide according to the invention is from 16 to 25 nucleotides in length. In another embodiment, the oligonucleotide according to the invention is from 16 to 24 nucleotides in length. In another embodiment, the oligonucleotide is from 16 to 22 nucleotides in length. In another embodiment, the oligonucleotide according to the invention is 16, 18, 20, or 22 nucleotides in length. In another embodiment, the oligonucleotide according to the invention is 21, 22, 24, or 25 nucleotides in length. In another embodiment, the oligonucleotide according to the invention is 18, 22, 24, or 25 nucleotides in length.

In another embodiment, the oligonucleotide is from 16 to 25 nucleotides in length and is for skipping exons 44, 45, 51, or 53 of pre-mRNA of dystrophin. In another embodiment, the oligonucleotide is from 16 to 24 nucleotides in length and is for skipping exons 44, 51, or 53 of pre-mRNA of dystrophin. In another embodiment, the oligonucleotide is from 16 to 22 nucleotides in length and is for skipping exon 51 of pre-mRNA of dystrophin. In another embodiment, the oligonucleotide is 16, 18, 20, or 22 nucleotides in length and is for skipping exon 51 of pre-mRNA of dystrophin. In another embodiment, the oligonucleotide is 20 or 23 nucleotides in length and is for skipping exon 44 of pre-mRNA of dystrophin. In another embodiment, the oligonucleotide is 21, 22, 24, or 25 nucleotides in length and is for skipping exon 45 of pre-mRNA of dystrophin. In another embodiment, the oligonucleotide is 18, 22, 24, or 25 nucleotides in length and is for skipping exon 53 of pre-mRNA of dystrophin. Throughout this application, reference to dystrophin is preferably interpreted as reference to human dystrophin.

Encompassed by the above ((i)(la)) are oligonucleotides that comprise at least one 2'-substituted monomer, preferably a 2'-substituted RNA monomer, and no phosphorothioate backbone linkage. Such an oligonucleotide can have a backbone that only comprises phosphodiester linkages. Similarly encompassed are oligonucleotides that comprise at least one 2'-substituted monomer, preferably a 2'-substituted RNA monomer, and one or more phosphorothioate backbone linkages.

Encompassed by the above ((i)(Ib)) are oligonucleotides that comprise no other monomers than 2'-substituted RNA monomers, and that comprise only backbone linkages that are phosphorothioate backbone linkages. Similarly encompassed are oligonucleotides that comprise no other monomers than 2'-substituted RNA monomers, and that comprise only backbone linkages that are phosphodiester backbone linkages.

As known to the skilled person, an oligonucleotide such as an RNA oligonucleotide generally consists of repeating monomers. Such a monomer is most often a nucleotide or a nucleotide analogue. The most common naturally occurring nucleotides in RNA are adenosine monophosphate, cytidine monophosphate, guanosine monophosphate, thymidine monophosphate, and uridine monophosphate. These consist of a pentose sugar ribose, a 5'-linked phosphate group which is linked via a phosphate ester, and a 1'-linked base. The sugar connects the base and the phosphate, and is therefore often referred to as the scaffold of the nucleotide. A modification in the pentose sugar is therefore often referred to as a scaffold modification. For severe modifications, the original pentose sugar might be replaced in its entirety by another moiety that similarly connects the base and the phosphate. It is therefore understood that while a pentose sugar is often a scaffold, a scaffold is not necessarily a pentose sugar.

A base, sometimes called a nucleobase, is generally adenine, cytosine, guanine, thymine, or uracil, or a derivative thereof. Cytosine, thymine, and uracil are pyrimidine bases, and are generally linked to the scaffold through their 1-nitrogen. Adenine and guanine are purine bases, and are generally linked to the scaffold through their 9-nitrogen.

A nucleotide is generally connected to neighbouring nucleotides through condensation of its 5'-phosphate moiety to the 3'-hydroxyl moiety of the neighbouring nucleotide monomer. Similarly, its 3'-hydroxyl moiety is generally connected to the 5'-phosphate of a neighbouring nucleotide monomer. This forms phosphodiester bonds. The phosphodiesters and the scaffold form an alternating copolymer. The bases are grafted to this copolymer, namely to the scaffold moieties. Because of this characteristic, the alternating copolymer formed by linked monomers of an oligonucleotide is often called the backbone of the oligonucleotide. Because the phosphodiester bonds connect neighbouring monomers together, they are often referred to as backbone linkages. It is understood that when a phosphate group is modified so that it is instead an analogous moiety such as a phosphorothioate, such a moiety is still referred to as the backbone linkage of the monomer. This is referred to as a backbone linkage modification. In general terms, the backbone of an oligonucleotide is thus comprised of alternating scaffolds and backbone linkages.

In another embodiment, the nucleobase is adenine, cytosine, guanine, thymine, or uracil. In another embodiment, the nucleobase is adenine, cytosine, guanine, or uracil. In another embodiment, the nucleobase is a modified form of adenine, cytosine, guanine, thymine, or uracil. In another embodiment, the modified nucleobase is hypoxanthine, pseudouracil, pseudocytosine, 1-methylpseudouracil, orotic acid, agmatidine, lysidine, 2-thiouracil, 2-thiothymine, 5-halouracil, 5-halomethyluracil, 5-trifluoromethyluracil, 5-propynyluracil, 5-propynylcytosine, 5-aminomethyluracil, 5-hydroxymethyluracil, 5-aminomethylcytosine, 5-hydroxymethylcytosine, 7-deazaguanine, 7-deazaadenine, 7-aza-2,6-diaminopurine, 8-aza-7-deazaguanine, 8-aza-7-deazaadenine, 8-aza-7-deaza-2,6-diaminopurine, pseudoisocytidine, N4-ethylcytosine, N2-cyclopentylguanine (cPent-G), N2-cyclopentyl-2-aminopurine (cPent-AP), or N2-propyl-2-aminopurine (Pr-AP). In another embodiment, the modified nucleobase is hypoxanthine, pseudouracil, pseudocytosine, 1-methylpseudouracil, orotic acid, agmatidine, lysidine, 2-thiouracil, 2-thiothymine, 5-halouracil, 5-halomethyluracil, 5-trifluoromethyluracil, 5-propynyluracil, 5-propynylcytosine, 5-aminomethyluracil, 5-hydroxymethyluracil, 5-aminomethylcytosine, or 5-hydroxymethylcytosine.

An oligonucleotide of the invention comprises or consists of 2'-substituted phosphorothioate monomer, preferably a 2'-substituted phosphorothioate RNA monomer, 2'-substituted phosphate RNA monomer, or 2'-substituted mixed phosphate/phosphorothioate RNA monomers. Such oligonucleotide comprises a 2'-substituted RNA monomer connected through or linked by a phosphorothioate or phosphate backbone linkage, or a mixture thereof, or consists of 2'-substituted phosphorothioate RNA, 2'-substituted phosphate RNA or a mixture thereof. Preferably, such oligonucleotide consists of 2'-substituted phosphorothioate RNA monomers, 2'-substituted phosphate RNA monomers, or a mixture thereof. The 2'-substituted RNA preferably is 2'-F, 2'-*O*-methyl, or 2'-*O*-(2-methoxyethyl). The 2'-*O*-(2-methoxyethyl) moiety is often referred to as 2'-MOE. More preferably, the 2'-substituted RNA monomer is a 2'-*O*-methyl RNA monomer. Such chemistries are known to the skilled person. In a preferred embodiment of this aspect is provided an oligonucleotide according to the invention, wherein said 2'-substituted monomer is a 2'-substituted RNA monomer, a 2'-F monomer, a 2'-amino monomer, a 2'-*O*-substituted monomer, a 2'-*O*-methyl monomer, or a 2'-*O*-(2-methoxyethyl) monomer, preferably a 2'-*O*-methyl monomer. Preferably, said 2'-substituted monomer is a 2'-substituted RNA monomer, such as a 2'-*O*-methyl RNA monomer.

Throughout the application, an oligonucleotide comprising a 2'-*O*-methyl monomer, or a 2'-*O*-methyl RNA monomer and a phosphorothioate, phosphate or mixed phosphate/phosphorothioate backbone linkages may be replaced respectively by an oligonucleotide comprising a 2'-*O*-methyl phosphorothioate RNA, 2'-*O*-methyl phosphate RNA or 2'-*O*-methyl phosphate/phosphorothioate RNA. Throughout the application, an oligonucleotide consisting of 2'-*O*-methyl RNA monomers linked by or connected through phosphorothioate, phosphate or mixed phosphate/phosphorothioate backbone linkages may be replaced by an oligonucleotide consisting of 2'-*O*-methyl phosphorothioate RNA, 2'-*O-*methyl phosphate RNA or 2'-*O*-methyl phosphate/phosphorothioate RNA.

In addition, an oligonucleotide of the invention comprises a base modification that increases binding affinity to target strands, increases melting temperature of the resulting duplex of said oligonucleotide with its target, and/or decreases immunostimulatory effects, and/or increases biostability, and/or improves biodistribution and/or intra-tissue distribution, and/or cellular uptake and trafficking. In a more preferred embodiment, an oligonucleotide of the invention comprises a 5-methylpyrimidine. A 5-methylpyrimidine is selected from a 5-methylcytosine and/or a 5-methyluracil and/or a thymine, in which thymine is identical to 5-methyluracil. 'Thymine' and '5-methyluracil' may be interchanged throughout the document. Preferably, oligonucleotides of the invention comprise at least one of either a 5-methylcytosine base or a 5-methyluracil base. In a preferred embodiment of the invention therefore is provided the oligonucleotide as described above, wherein all cytosine bases are 5-methylcytosine bases, and/or wherein all uracil bases are 5-methyluracil bases. This relates to oligonucleotides that comprise 5-methylcytosine but no unsubstituted cytosine or uracil, to oligonucleotides that comprise 5-methyluracil but no unsubstituted cytosine or uracil, and to oligonucleotides that comprise both 5-methylcytosine and 5-methyluracil but no unsubstituted cytosine or uracil. It also relates to oligonucleotides that comprise 5-methylcytosine but no unsubstituted cytosine yet that comprise unsubstituted uracil, or to oligonucleotides that comprise 5-methyluracil but no unsubstituted uracil, yet that comprise unsubstituted cytosine.

An oligonucleotide of the invention comprises a scaffold modification that increases binding affinity to target strands, increases melting temperature of the resulting duplex of said oligonucleotide with its target, and/or decreases immunostimulatory effects, and/or increases biostability, and/or improves biodistribution and/or intra-tissue distribution, and/or cellular uptake and trafficking. Encompassed by the invention are those scaffold modifications that result in a bicyclic nucleic acid (BNA) monomer. A bicyclic scaffold is generally a pentose-derived scaffold that has been chemically altered to conformationally restrict the scaffold, leading to the improved effects above. Examples of bicyclic scaffolds are scaffolds where a first cycle such as a pentose cycle forms a spirane with a further cyclic moiety so that both cycles share only one atom, scaffolds where a first cycle such as a pentose cycle is fused to a further cyclic moiety so that both cycles share two adjacent atoms, and scaffolds where a first cycle such as a pentose cycle forms a bridged compound through a moiety that is linked to the first cyclic moiety at two non-adjacent atoms. Such non-adjacent atoms are referred to as bridgehead atoms. Bridged compounds comprise multiple cycles, each of which overlap over at least three atoms. A compound with two cycles wherein those cycles overlap over only two atoms is a fused compound instead. In some bridged compounds, the smallest link between two bridgehead atoms is referred to as the bridging moiety, or as the bridge moiety. In other bridged compounds, when one cycle is a characteristic cycle such as the pentose cycle of a nucleotide, the moiety that is not constitutive to that characteristic cycle is referred to as the bridging moiety. It follows that the nomenclature of bridged bicyclic compounds is context-dependent.

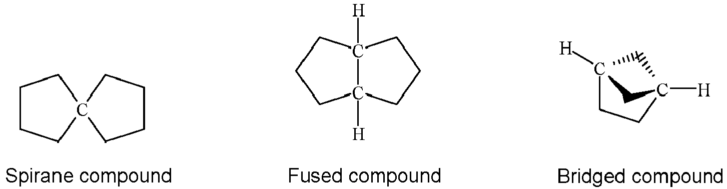

Bicyclic compounds can comprise additional cycles. A bicyclic compound according to the invention is at least bicyclic, and said two cycles constitute a spirane, a fused system, or a bridged system, or a combination thereof. The invention does not encompass scaffold modifications where two independent cycles are linked via a non-cyclic linker, so as to not form a spirane, fused compound, or bridged compound. Preferred bicyclic compounds are fused and bridged compounds. In more preferred embodiments, a bicyclic nucleic acid monomer (BNA) is a bridged nucleic acid monomer. As described herein, both a "bridged" or a "bicylic" nucleic acid monomer relates to a nucleotide with a modified scaffold that enhances the melting temperature of an oligonucleotide against an RNA target as compared to a non-BNA nucleotide-containing control oligonucleotide.

In a preferred embodiment is provided an oligonucleotide according to the invention, wherein each occurrence of said bicyclic nucleic acid (BNA) scaffold modification results in a monomer that is independently chosen from the group consisting of a conformationally restricted nucleotide (CRN) monomer, a locked nucleic acid (LNA) monomer, a xylo-LNA monomer, an α-LNA monomer, an α-L-LNA monomer, a β-D-LNA monomer, a 2'-amino-LNA monomer, a 2'-(alkylamino)-LNA monomer, a 2'-(acylamino)-LNA monomer, a 2'-N-substituted-2'-amino-LNA monomer, a 2'-thio-LNA monomer, a (2'-O,4'-C) constrained ethyl (cEt) BNA monomer, a (2'-O,4'-C) constrained methoxyethyl (cMOE) BNA monomer, a 2',4'-BNA^{NC}(N-H) monomer, a 2',4'-BNA^{NC}(N-Me) monomer, a 2',4'-BNA^{NC}(N-Bn) monomer, an ethylene-bridged nucleic acid (ENA) monomer, a carba LNA (cLNA) monomer, a 3,4-dihydro-2*H*-pyran nucleic acid (DpNA) monomer, a 2'-C-bridged bicyclic nucleotide (CBBN) monomer, a heterocyclic-bridged BNA monomer (such as triazolyl or tetrazolyl-linked), an amido-bridged BNA monomer, an urea-bridged BNA monomer, a sulfonamide-bridged BNA monomer, a bicyclic carbocyclic nucleotide monomer, a TriNA monomer, an α-L-TriNA monomer, a bicyclo DNA (bcDNA) monomer, an F-bcDNA monomer, a tricyclo DNA (tcDNA) monomer, an F-tcDNA monomer, an oxetane nucleotide monomer, a locked PMO monomer derived from 2'-amino-LNA, a guanidine-bridged nucleic acid (GuNA) monomer, a spirocyclopropylene-bridged nucleic acid (scpBNA) monomer, and derivatives thereof. It is also encompassed by the invention to introduce more than one distinct scaffold BNA modification in said oligonucleotide. More preferably, each occurrence of said BNA scaffold modification results in a monomer that is independently chosen from the group consisting of a conformationally restrained nucleotide (CRN) monomer, a locked nucleic acid (LNA) monomer, a xylo-LNA monomer, an α-L-LNA monomer, a β-D-LNA monomer, a 2'-amino-LNA monomer, a 2'-(alkylamino)-LNA monomer, a 2'-(acylamino)-LNA monomer, a 2'-*N*-substituted-2'-amino-LNA monomer, a (2'-O,4'-C) constrained ethyl (cEt) LNA monomer, a (2'-O,4'-C) constrained methoxyethyl (cMOE) BNA monomer, a 2',4'-BNA^{NC}(N-H) monomer, a 2',4'-BNA^{NC}(N-Me) monomer, an ethylene-bridged nucleic acid (ENA) monomer, a 2'-C-bridged bicyclic nucleotide (CBBN) monomer, and derivatives thereof. In preferred embodiments, the BNA scaffold modification results in an LNA monomer, an ENA monomer, a cEt BNA monomer, an oxo-CBBN monomer, a 2'-amino-LNA monomer, or a cMOE BNA monomer. In highly preferred embodiments, the BNA scaffold modification results in an LNA monomer, an ENA monomer, a cEt BNA monomer, or a cMOE BNA monomer. Most preferably, the BNA scaffold modification results in an LNA monomer.

Throughout this application, any monomer comprising a BNA scaffold modification can be replaced by any other monomer comprising a BNA modification, preferably while preserving the nucleobase or modified nucleobase thereof. In other words, the scaffold modifications could be exchanged, while preserving the sequence of the oligonucleotide. As a non-limiting example, an LNA monomer comprising an adenine could be replaced by an ENA monomer comprising an adenine.

Structural examples of monomers comprising these BNA scaffold modifications are shown below, where B is a base as defined earlier herein, X is a variable such as a heteroatom or a methylene moiety, X₂ is a hydroxyl moiety or another 2'-substitution as defined earlier herein, and L is a backbone linkage as described earlier herein. In the literature, the naming of such modifications is often arbitrary and does not follow a uniform convention - in this application, the names as provided below are intended to refer to the structures provided below. For comparison, the cyclic scaffold of a conventional RNA monomer is shown first. In the structures shown below, monomers are typically depicted as 3'-terminal monomers. When chirality is not indicated, each enantiomer is individually referenced. The invention is not limited to this kind of monomers which are provided for illustrative purposes. Heteroatoms comprised in a cyclic moiety can be substituted by other heteroatoms.

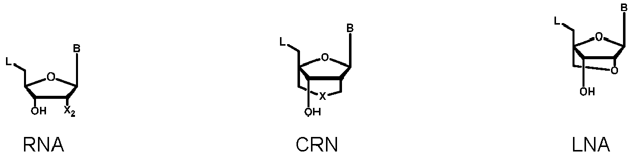

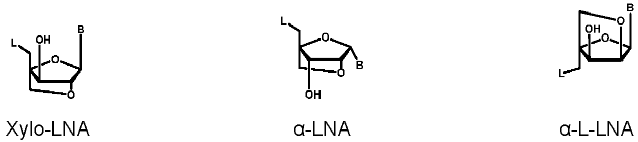

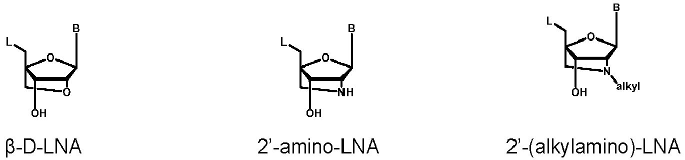

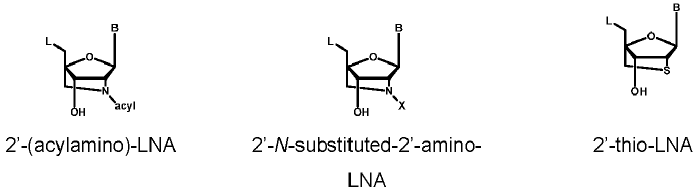

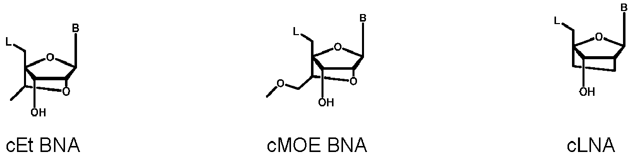

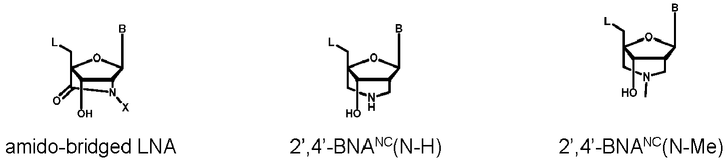

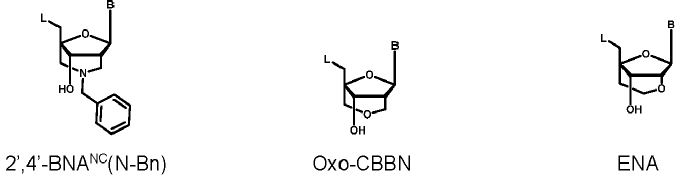

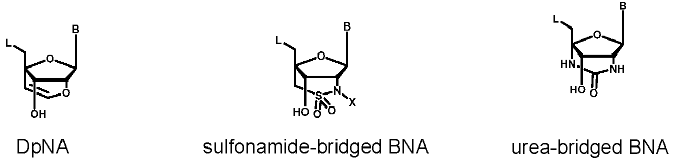

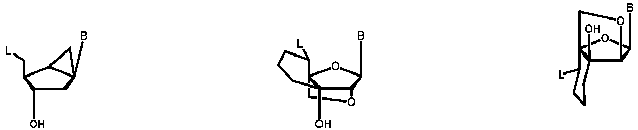

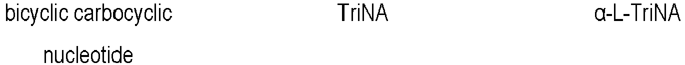

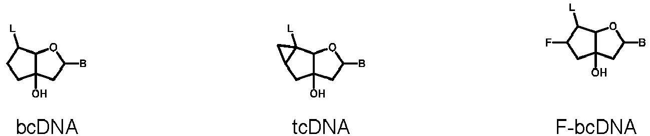

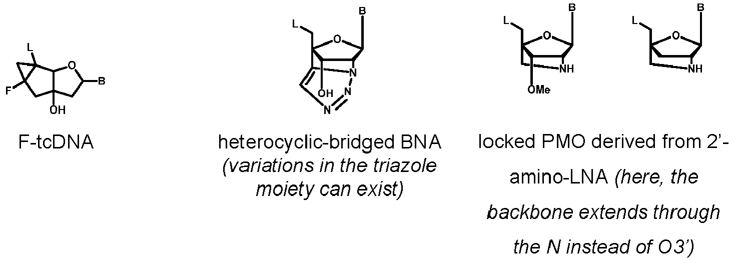

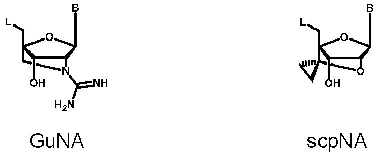

The following is a non-exhaustive overview of literature references for BNA scaffold modifications shown above: cEt (2'-*O*,4'-*C* constrained ethyl) LNA (doi: 10.1021/ja710342q), cMOE (2'-*O*,4'-*C* constrained methoxyethyl) LNA (Seth et al., J. Org. Chem. 2010, 75, 1569-1581), 2',4'-BNA^{NC}(N-H), 2',4'-BNA^{NC}(N-Me), ethylene-bridged nucleic acid (ENA) (doi: 10.1093/nass/1.1.241), carba LNA (cLNA) (doi: 10.1021/jo100170g), DpNA (Osawa et al., J. Org. Chem., 2015, 80 (21), pp 10474-10481), 2'-*C*-bridged bicyclic nucleotide (CBBN, as in *e.g.* WO 2014/145356 (MiRagen Therapeutics)), heterocyclic-bridged LNA (as in e.g. WO 2014/126229 (Mitsuoka Y et al.)), amido-bridged LNA (as in *e.g.* Yamamoto et al. Org. Biomol. Chem. 2015, 13, 3757), urea-bridged LNA (as in *e.g.* Nishida et al. Chem. Commun. 2010, 46, 5283), sulfonamide-bridged LNA (as in *e.g.* WO 2014/112463 (Obika S et al.)), bicyclic carbocyclic nucleosides (as in *e.g.* WO 2015/142910 (lonis Pharmaceuticals)), TriNA (Hanessian et al., J. Org. Chem., 2013, 78 (18), pp 9064-9075), α-L-TriNA, bicyclo DNA (bcDNA) (Bolli et al., Chem Biol. 1996 Mar;3(3):197-206), F-bcDNA (DOI: 10.1021/jo402690j), tricyclo DNA (tcDNA) (Murray et al., Nucl. Acids Res., 2012, Vol. 40, No. 13 6135-6143), F-tcDNA (doi: 10.1021/acs.joc.5b00184), an oxetane nucleotide monomer (Nucleic Acids Res. 2004, 32, 5791-5799), scpNA (Horiba et al., J. Org. Chem. 2016, doi: 10.1021/acs.joc.6b02036, GuNA (Shrestha et al., Chem. Commun. 2014, doi: 10.1039/C3CC46017G). For those not mentioned above, reference is made to WO 2011/097641 (ISIS/lonis Pharmaceuticals) and WO2016/017422 (Osaka University), which are incorporated in their entirety by reference.

An oligonucleotide of the invention comprising a BNA and a 5-methylcytosine and/or a 5-methyluracil base means that at least one of the scaffolds of said oligonucleotide has been modified by substitution with a BNA, in combination with at least one of the cytosine nucleobases of said oligonucleotide being modified by substitution of the hydrogen at the 5-position of the pyrimidine ring with a methyl group, i.e. a 5-substituted cytosine, and/or that at least one of the uracil nucleobases of said oligonucleotide has been modified by substitution of the proton at the 5-position of the pyrimidine ring with a methyl group (i.e. a 5-methyluracil), respectively. Within the context of the invention, the expression "the substitution of a hydrogen with a methyl group in position 5 of the pyrimidine ring" may be replaced by the expression "the substitution of a pyrimidine with a 5-methylpyrimidine," with pyrimidine referring to only uracil, only cytosine, or both. If said oligonucleotide comprises 1, 2, 3, 4, 5, 6, 7, 8, 9, or more cytosines and/or uracils, at least one, 2, 3, 4, 5, 6, 7, 8 9, or more cytosines and/or uracils respectively have been modified this way. Preferably all cytosines and/or uracils have been modified this way or substituted by 5-methylcytosine and/or 5-methyluracil respectively. Needless to say, the invention could therefore only be applied to oligonucleotides comprising at least one cytosine or uracil, respectively, in their sequence.

Preferably, an oligonucleotide according to the invention comprises RNA monomers, as RNA/RNA duplexes are very stable. It is preferred that an RNA oligonucleotide comprises a modification providing the RNA with an additional property, for instance resistance to endonucleases, exonucleases, and RNaseH, additional hybridisation strength, increased stability (for instance in a bodily fluid), increased or decreased flexibility, increased activity, reduced toxicity, increased intracellular transport, increased cellular uptake, tissue-specificity, etc. In addition, the mRNA complexed with the oligonucleotide of the invention is preferably not susceptible to RNaseH cleavage. Preferred modifications have been identified above.

Accordingly, the invention provides an oligonucleotide comprising a 2'-O-methyl phosphorothioate RNA monomer or consisting of 2'-O-methyl phosphorothioate RNA and comprising a BNA with or without a 5-methylpyrimidine base. Most preferably, this oligonucleotide consists of 2'-O-methyl RNA monomers connected through a phosphorothioate or phosphate backbone and all of its cytosines and/or all of its uracils, independently, have been substituted by 5-methylcytosine and/or 5-methyluracil, respectively, and at least one 2'-O-methyl scaffold has been replaced by a BNA. Thus, an oligonucleotide of the invention may have, in the addition of having at least one BNA scaffold modification:
- At least one and preferably all cytosines substituted with 5-methylcytosines,
- At least one and preferably all cytosines substituted with 5-methylcytosines and at least one uracil substituted with 5-methyluracil,
- At least one uracil substituted with 5-methyluracil.

In preferred embodiments of this aspect is provided the oligonucleotide as described above, wherein said oligonucleotide comprises 1, 2, 3, 4, 5, or 6 monomers that comprise a bicyclic nucleic acid (BNA) scaffold modification, preferably a bridged nucleic acid scaffold modification.

In these embodiments, it is preferred that at least one BNA scaffold modification is comprised in a terminal monomer of the oligonucleotide, preferably in the 5'-terminal monomer. It is most preferred that both terminal monomers comprise a BNA scaffold. As such, a more preferred embodiment of this aspect provides the oligonucleotide according to the invention wherein at least one bicyclic nucleic acid (BNA) scaffold modification is comprised in a terminal monomer of said oligonucleotide, preferably in the 5'-terminal monomer of said oligonucleotide, more preferably in both terminal monomers of said oligonucleotide. Other preferred embodiments entail that a terminal monomer and its neighbouring monomer each comprise a BNA scaffold. In such a case, the first two monomers or the last two monomers of an oligonucleotide each comprise a BNA scaffold. This can be combined in any way, so that for example the first and the last two monomers, or the first two and the last monomer all comprise a BNA scaffold. When an oligonucleotide according to the invention comprises a terminal monomer comprising a BNA scaffold, additional monomers with a BNA scaffold are preferably either at the other terminus, or adjacent to terminal monomers with a BNA scaffold.

A preferred embodiment of this aspect provides the oligonucleotide of the invention, wherein said oligonucleotide comprises BNA modifications as selected from the set consisting of:
- a single BNA scaffold modification in the monomer at the 5'-terminus,
- a single BNA scaffold modification in the monomer at the 3'-terminus,
- two BNA scaffold modifications where one is in the monomer at the 5'-terminus and the other is in the monomer at the 3'-terminus,
- two BNA scaffold modifications, one in each of the two monomers that are closest to the 5'-terminus,
- two BNA scaffold modifications, one in each of the two monomers that are closest to the 3'-terminus,
- three to seven BNA scaffold modifications, where one is in the monomer at the 5'-terminus, one is in the monomer at the 3'-terminus, and 1-5 BNA scaffold modifications are in non-terminal residues,
- three to six BNA scaffold modifications, where one is in the monomer at the 5'-terminus, one is in the monomer at the 3'-terminus, and 1-4 BNA scaffold modifications are in non-terminal residues,
- three to five BNA scaffold modifications, where one is in the monomer at the 5'-terminus, one is in the monomer at the 3'-terminus, and 1-3 BNA scaffold modifications are in non-terminal residues,
- three or four BNA scaffold modifications, where one is in the monomer at the 5'-terminus, one is in the monomer at the 3'-terminus, and one or two BNA scaffold modifications are in non-terminal residues,
- three BNA scaffold modifications, where one is in the monomer at the 5'-terminus, one is in the monomer at the 3'-terminus, and one BNA scaffold modification is in a non-terminal residue,
- four to six BNA scaffold modifications, where one is in the monomer at the 5'-terminus, one is in the monomer at the 3'-terminus, and 2-4 BNA scaffold modifications are in non-terminal residues, and
- four or five BNA scaffold modifications, where one is in the monomer at the 5'-terminus, one is in the monomer at the 3'-terminus, and 2-3 BNA scaffold modifications are in non-terminal residues.

When an oligonucleotide according to the invention comprises or consists of a sequence represented by SEQ ID NOs: 8, 14, 20, 26, 32, 38, 44, 50, 56, 62, 68, 74, 80, 86, 92, 98, 104, 110, 116, 122, 128, 134, 140, 146, 152, 158, 164, 170, 176, 182, 188, 194, 200, 206, 212, 218, 224, 230, 236, 242, 248, 254, 260, 266, 272, 278, 284, 290, 296, 302, 308, 314, 320, 326, 332, 338, 344, 350, 356, 362, 368, 374, 380, 386, 392, 398, 404, 410, 416, 422, 428, 434, 440, 446, 452, 458, 464, 470, 476, 482, 488, 494, 500, 506, 512, 518, 524, 530, 536, 542, 548, 554, 560, 566, 572, 578, 584, 590, 596, 602, 608, 614, 620, 626, 632, 638, 644, 650, 656, 662, 668, 674, 680, 686, 692, 698, 704, 710, 716, 722, 728, 734, 740, 746, 752, 758, 764, 770, 776, 782, 788, 794, 800, 806, 812, 818, 824, 830, 836, 842, 848, 854, 860, 866, 872, 878, 884, 890, 896, 902, 908, 914, 920, 926, 932, 938, 944, 950, 956, 962, 968, 974, 980, 986, 992, 998, 1004, 1010, 1016, 1022, 1028, 1034, 1040, 1046, 1052, 1058, 1064, 1070, 1076, 1082, 1088, 1094, 1100, 1106, 1112, 1118, 1124, 1130, 1136, 1142, 1148, 1154, 1160, 1166, 1172, 1178, 1184, 1190, 1196, 1202, 1208, 1214, 1220, 1226, 1232, 1238, 1244, 1250, 1256, 1262, 1268, 1274, 1280, 1286, 1292, 1298, 1304, 1310, 1316, 1322, 1328, 1334, 1340, 1346, 1352, 1358, 1364, 1370, 1376, 1382, 1388, 1394, 1400, 1406, 1412, 1418, 1424, 1430, 1436, 1442, 1448, 1454, 1460, 1466, 1472, 1478, 1484, 1490, 1496, 1502, 1508, 1514, 1520, 1526, 1532, 1538, 1544, 1550, 1556, 1562, 1568, 1574, or 1580, preferably at least one BNA scaffold modifications is comprised in said oligonucleotide.

When an oligonucleotide according to the invention comprises or consists of a sequence represented by SEQ ID NOs: 9, 15, 21, 27, 33, 39, 45, 51, 57, 63, 69, 75, 81, 87, 93, 99, 105, 111, 117, 123, 129, 135, 141, 147, 153, 159, 165, 171, 177, 183, 189, 195, 201, 207, 213, 219, 225, 231, 237, 243, 249, 255, 261, 267, 273, 279, 285, 291, 297, 303, 309, 315, 321, 327, 333, 339, 345, 351, 357, 363, 369, 375, 381, 387, 393, 399, 405, 411, 417, 423, 429, 435, 441, 447, 453, 459, 465, 471, 477, 483, 489, 495, 501, 507, 513, 519, 525, 531, 537, 543, 549, 555, 561, 567, 573, 579, 585, 591, 597, 603, 609, 615, 621, 627, 633, 639, 645, 651, 657, 663, 669, 675, 681, 687, 693, 699, 705, 711, 717, 723, 729, 735, 741, 747, 753, 759, 765, 771, 777, 783, 789, 795, 801, 807, 813, 819, 825, 831, 837, 843, 849, 855, 861, 867, 873, 879, 885, 891, 897, 903, 909, 915, 921, 927, 933, 939, 945, 951, 957, 963, 969, 975, 981, 987, 993, 999, 1005, 1011, 1017, 1023, 1029, 1035, 1041, 1047, 1053, 1059, 1065, 1071, 1077, 1083, 1089, 1095, 1101, 1107, 1113, 1119, 1125, 1131, 1137, 1143, 1149, 1155, 1161, 1167, 1173, 1179, 1185, 1191, 1197, 1203, 1209, 1215, 1221, 1227, 1233, 1239, 1245, 1251, 1257, 1263, 1269, 1275, 1281, 1287, 1293, 1299, 1305, 1311, 1317, 1323, 1329, 1335, 1341, 1347, 1353, 1359, 1365, 1371, 1377, 1383, 1389, 1395, 1401, 1407, 1413, 1419, 1425, 1431, 1437, 1443, 1449, 1455, 1461, 1467, 1473, 1479, 1485, 1491, 1497, 1503, 1509, 1515, 1521, 1527, 1533, 1539, 1545, 1551, 1557, 1563, 1569, or 1575, preferably only the 5'-terminal monomer of said oligonucleotide comprises a BNA scaffold modification.

When an oligonucleotide according to the invention comprises or consists of a sequence represented by SEQ ID NOs: 10, 16, 22, 28, 34, 40, 46, 52, 58, 64, 70, 76, 82, 88, 94, 100, 106, 112, 118, 124, 130, 136, 142, 148, 154, 160, 166, 172, 178, 184, 190, 196, 202, 208, 214, 220, 226, 232, 238, 244, 250, 256, 262, 268, 274, 280, 286, 292, 298, 304, 310, 316, 322, 328, 334, 340, 346, 352, 358, 364, 370, 376, 382, 388, 394, 400, 406, 412, 418, 424, 430, 436, 442, 448, 454, 460, 466, 472, 478, 484, 490, 496, 502, 508, 514, 520, 526, 532, 538, 544, 550, 556, 562, 568, 574, 580, 586, 592, 598, 604, 610, 616, 622, 628, 634, 640, 646, 652, 658, 664, 670, 676, 682, 688, 694, 700, 706, 712, 718, 724, 730, 736, 742, 748, 754, 760, 766, 772, 778, 784, 790, 796, 802, 808, 814, 820, 826, 832, 838, 844, 850, 856, 862, 868, 874, 880, 886, 892, 898, 904, 910, 916, 922, 928, 934, 940, 946, 952, 958, 964, 970, 976, 982, 988, 994, 1000, 1006, 1012, 1018, 1024, 1030, 1036, 1042, 1048, 1054, 1060, 1066, 1072, 1078, 1084, 1090, 1096, 1102, 1108, 1114, 1120, 1126, 1132, 1138, 1144, 1150, 1156, 1162, 1168, 1174, 1180, 1186, 1192, 1198, 1204, 1210, 1216, 1222, 1228, 1234, 1240, 1246, 1252, 1258, 1264, 1270, 1276, 1282, 1288, 1294, 1300, 1306, 1312, 1318, 1324, 1330, 1336, 1342, 1348, 1354, 1360, 1366, 1372, 1378, 1384, 1390, 1396, 1402, 1408, 1414, 1420, 1426, 1432, 1438, 1444, 1450, 1456, 1462, 1468, 1474, 1480, 1486, 1492, 1498, 1504, 1510, 1516, 1522, 1528, 1534, 1540, 1546, 1552, 1558, 1564, 1570, or 1576, preferably only the 3'-terminal monomer of said oligonucleotide comprises a BNA scaffold modification.

When an oligonucleotide according to the invention comprises or consists of a sequence represented by SEQ ID NOs: 11, 17, 23, 29, 35, 41, 47, 53, 59, 65, 71, 77, 83, 89, 95, 101, 107, 113, 119, 125, 131, 137, 143, 149, 155, 161, 167, 173, 179, 185, 191, 197, 203, 209, 215, 221, 227, 233, 239, 245, 251, 257, 263, 269, 275, 281, 287, 293, 299, 305, 311, 317, 323, 329, 335, 341, 347, 353, 359, 365, 371, 377, 383, 389, 395, 401, 407, 413, 419, 425, 431, 437, 443, 449, 455, 461, 467, 473, 479, 485, 491, 497, 503, 509, 515, 521, 527, 533, 539, 545, 551, 557, 563, 569, 575, 581, 587, 593, 599, 605, 611, 617, 623, 629, 635, 641, 647, 653, 659, 665, 671, 677, 683, 689, 695, 701, 707, 713, 719, 725, 731, 737, 743, 749, 755, 761, 767, 773, 779, 785, 791, 797, 803, 809, 815, 821, 827, 833, 839, 845, 851, 857, 863, 869, 875, 881, 887, 893, 899, 905, 911, 917, 923, 929, 935, 941, 947, 953, 959, 965, 971, 977, 983, 989, 995, 1001, 1007, 1013, 1019, 1025, 1031, 1037, 1043, 1049, 1055, 1061, 1067, 1073, 1079, 1085, 1091, 1097, 1103, 1109, 1115, 1121, 1127, 1133, 1139, 1145, 1151, 1157, 1163, 1169, 1175, 1181, 1187, 1193, 1199, 1205, 1211, 1217, 1223, 1229, 1235, 1241, 1247, 1253, 1259, 1265, 1271, 1277, 1283, 1289, 1295, 1301, 1307, 1313, 1319, 1325, 1331, 1337, 1343, 1349, 1355, 1361, 1367, 1373, 1379, 1385, 1391, 1397, 1403, 1409, 1415, 1421, 1427, 1433, 1439, 1445, 1451, 1457, 1463, 1469, 1475, 1481, 1487, 1493, 1499, 1505, 1511, 1517, 1523, 1529, 1535, 1541, 1547, 1553, 1559, 1565, 1571, or 1577, preferably only both the 5'-terminal monomer and the 3'-terminal monomer of said oligonucleotide comprise a BNA scaffold modification.

When an oligonucleotide according to the invention comprises or consists of a sequence represented by SEQ ID NOs: 12, 18, 24, 30, 36, 42, 48, 54, 60, 66, 72, 78, 84, 90, 96, 102, 108, 114, 120, 126, 132, 138, 144, 150, 156, 162, 168, 174, 180, 186, 192, 198, 204, 210, 216, 222, 228, 234, 240, 246, 252, 258, 264, 270, 276, 282, 288, 294, 300, 306, 312, 318, 324, 330, 336, 342, 348, 354, 360, 366, 372, 378, 384, 390, 396, 402, 408, 414, 420, 426, 432, 438, 444, 450, 456, 462, 468, 474, 480, 486, 492, 498, 504, 510, 516, 522, 528, 534, 540, 546, 552, 558, 564, 570, 576, 582, 588, 594, 600, 606, 612, 618, 624, 630, 636, 642, 648, 654, 660, 666, 672, 678, 684, 690, 696, 702, 708, 714, 720, 726, 732, 738, 744, 750, 756, 762, 768, 774, 780, 786, 792, 798, 804, 810, 816, 822, 828, 834, 840, 846, 852, 858, 864, 870, 876, 882, 888, 894, 900, 906, 912, 918, 924, 930, 936, 942, 948, 954, 960, 966, 972, 978, 984, 990, 996, 1002, 1008, 1014, 1020, 1026, 1032, 1038, 1044, 1050, 1056, 1062, 1068, 1074, 1080, 1086, 1092, 1098, 1104, 1110, 1116, 1122, 1128, 1134, 1140, 1146, 1152, 1158, 1164, 1170, 1176, 1182, 1188, 1194, 1200, 1206, 1212, 1218, 1224, 1230, 1236, 1242, 1248, 1254, 1260, 1266, 1272, 1278, 1284, 1290, 1296, 1302, 1308, 1314, 1320, 1326, 1332, 1338, 1344, 1350, 1356, 1362, 1368, 1374, 1380, 1386, 1392, 1398, 1404, 1410, 1416, 1422, 1428, 1434, 1440, 1446, 1452, 1458, 1464, 1470, 1476, 1482, 1488, 1494, 1500, 1506, 1512, 1518, 1524, 1530, 1536, 1542, 1548, 1554, 1560, 1566, 1572, or 1578, preferably only the two most 5'-terminal monomers of said oligonucleotide both comprise a BNA scaffold modification.

When an oligonucleotide according to the invention comprises or consists of a sequence represented by SEQ ID NOs: 13, 19, 25, 31, 37, 43, 49, 55, 61, 67, 73, 79, 85, 91, 97, 103, 109, 115, 121, 127, 133, 139, 145, 151, 157, 163, 169, 175, 181, 187, 193, 199, 205, 211, 217, 223, 229, 235, 241, 247, 253, 259, 265, 271, 277, 283, 289, 295, 301, 307, 313, 319, 325, 331, 337, 343, 349, 355, 361, 367, 373, 379, 385, 391, 397, 403, 409, 415, 421, 427, 433, 439, 445, 451, 457, 463, 469, 475, 481, 487, 493, 499, 505, 511, 517, 523, 529, 535, 541, 547, 553, 559, 565, 571, 577, 583, 589, 595, 601, 607, 613, 619, 625, 631, 637, 643, 649, 655, 661, 667, 673, 679, 685, 691, 697, 703, 709, 715, 721, 727, 733, 739, 745, 751, 757, 763, 769, 775, 781, 787, 793, 799, 805, 811, 817, 823, 829, 835, 841, 847, 853, 859, 865, 871, 877, 883, 889, 895, 901, 907, 913, 919, 925, 931, 937, 943, 949, 955, 961, 967, 973, 979, 985, 991, 997, 1003, 1009, 1015, 1021, 1027, 1033, 1039, 1045, 1051, 1057, 1063, 1069, 1075, 1081, 1087, 1093, 1099, 1105, 1111, 1117, 1123, 1129, 1135, 1141, 1147, 1153, 1159, 1165, 1171, 1177, 1183, 1189, 1195, 1201, 1207, 1213, 1219, 1225, 1231, 1237, 1243, 1249, 1255, 1261, 1267, 1273, 1279, 1285, 1291, 1297, 1303, 1309, 1315, 1321, 1327, 1333, 1339, 1345, 1351, 1357, 1363, 1369, 1375, 1381, 1387, 1393, 1399, 1405, 1411, 1417, 1423, 1429, 1435, 1441, 1447, 1453, 1459, 1465, 1471, 1477, 1483, 1489, 1495, 1501, 1507, 1513, 1519, 1525, 1531, 1537, 1543, 1549, 1555, 1561, 1567, 1573, or 1579, preferably only the two most 3'-terminal monomers of said oligonucleotide both comprise a BNA scaffold modification.

When an oligonucleotide according to the invention comprises or consists of a sequence represented by a SEQ ID NO that is not SEQ ID NO: 1580, said oligonucleotide preferably comprises 5-methylcytosines instead of cytosines, and said oligonucleotide preferably comprises at least one 2'-O-methyl phosphorothioate monomer, more preferably comprises only 2'-O-methyl phosphorothioate monomers. When an oligonucleotide according to the invention comprises or consists of a sequence represented by SEQ ID NO: 1580, said oligonucleotide preferably comprises cytosines instead of 5-methylcytosines, and said oligonucleotide preferably comprises at least one 2'-*O*-methyl phosphorothioate monomer, more preferably comprises only 2'-*O*-methyl phosphorothioate monomers. Whenever a SEQ ID NO references T or U and said monomer comprises a BNA scaffold modification, said monomer (i.e. said reference) can optionally be replaced by U or T, respectively. Whenever a SEQ ID NO references C or 5-methyl-C and said monomer comprises a BNA scaffold modification, said reference can optionally be replaced by 5-methyl-C or C, respectively.

Throughout this application, a BNA scaffold modification can always be comprised in an oligonucleotide unless explicitly stated otherwise. However, for the sake of legibility, this is not always explicitly spelt out. This means that whenever an oligonucleotide is said to comprise or consist of only a particular kind of monomer, this does not exclude the presence of BNA scaffold modifications in cases where a BNA scaffold modification is mentioned as being present. For example, an oligonucleotide that consists of only 2'-O-methyl RNA monomers can nonetheless comprise a monomer with a BNA scaffold modification. This will be apparent from context (for example, when an AON is said to consist exclusively of one monomer, yet still also comprise a BNA scaffold modification.)

In preferred embodiments of this aspect is provided the oligonucleotide according to the invention, wherein said oligonucleotide is complementary, preferably reverse complementary to or binds to or targets or hybridizes with at least a part of an exon and/or non-exon region, preferably wherein said oligonucleotide comprises or consists of a sequence which is complementary to or binds or targets or hybridizes at least a part of an exon recognition sequence (ERS), an exonic splicing silencer (ESS), an intronic splicing silencer (ISS), an SR protein binding site, or another splicing element, signal, or structure. When such an oligonucleotide is complementary, it is understood that it can also be reverse complementary. In this application, the term "complementary" encompasses both forward complementary and reverse complementary sequences, as will be apparent to a skilled person from the context.

In this context, preferred sequences to which the oligonucleotide according to the invention is complementary, or to which it binds, or which it targets, or which it hybridizes with, are dystrophin exons, such as dystrophin pre-mRNA exons 2 to 78. Preferred dystrophin exons are exons 2 to 78, more preferably exons 10 to 60, most preferably exons 44 to 55 and preferred non-exon regions are introns 1 to 78. More preferred exons to which the oligonucleotide is complementary, or to which it binds, or which it targets, or with which it hybridizes, are dystrophin pre-mRNA exons 44, 45, 51, 52, 53 and 55. Preferred exons to which the oligonucleotide is complementary, or to which it binds, or which it targets, or with which it hybridizes, are dystrophin pre-mRNA exons 44, 45, 51, 52, 53 and 55. Most preferably, such an oligonucleotide hybridizes with at least a part of a dystrophin pre-mRNA exon chosen from exons 44, 45, 51, 52, 53 and 55, and has a length of 10 to 33 nucleotides, more preferably of 16 to 22 nucleotides. Accordingly, in a preferred embodiment is provided an oligonucleotide according to the invention wherein said oligonucleotide is complementary, preferably reverse complementary to at least part of an exon and/or non-exon region, wherein said at least part of an exon and/or non-exon region has a length of 10 to 33 nucleotides, preferably of 16 to 22 nucleotides. More preferably, said at least part of an exon and/or non-exon region has a length of at least 10, 11, 12, 13, 14, 15, 16, 17, 18, 19, 20, 21, 22, 23 ,24, 25, 26, 27, 28, 29, 30, 31, 32, or 33 nucleotides. Accordingly, it is preferred that said at least part of an exon and/or non-exon region has a length of at most 33, 32, 31, 30, 29, 28, 27, 26, 25, 24, 23, 22, 21, 20, 19, 18, 17, 16, 15, 14, 13, 12, 11, or 10 nucleotides. Most preferred lengths are 16, 17, 18, 19, 20, 21, or 22 nucleotides.

Additionally, in this context, other preferred sequences to which the oligonucleotide according to the invention is complementary, or to which it binds, or which it targets, or which it hybridizes with, are SMN2 splicing regulatory elements, preferably such as those in introns 6 and 7, more preferably such as the splicing silencer ISS-N1 in intron 7.

Accordingly, in a preferred embodiment is provided an oligonucleotide according to the invention, wherein said exon and/or non-exon region is in a DMD gene or in an SMN gene. An SMN gene can be an SMN1 gene or an SMN2 gene, preferably an SMN2 gene.

Preferably, an oligonucleotide of the invention is represented by a nucleotide sequence comprising or consisting of a sequence that is capable of binding to, targeting or being complementary to a part of an exon of dystrophin pre-mRNA. Said binding or targeted part may be at least 50% of the length of the oligonucleotide of the invention, or at least 60%, or at least 70%, or at least 80%, or at least 90% or at least 95%, or 98% and up to 100%. An oligonucleotide may be represented by a nucleotide sequence, said nucleotide sequence comprising a sequence that binds, targets, or is complementary to at least a part of dystrophin pre-mRNA as defined herein and additional flanking sequences. In a more preferred embodiment, the length of said binding or targeted part of said oligonucleotide is of at least 10, 11, 12, 13, 14, 15, 16, 17, 18, 19, 20, 21, 22, 23, 24, 25, 26, 27, 28, 29, 30, 31, 32, or 33 nucleotides. Several types of flanking sequences may be used. Preferably, flanking sequences are used to modify the binding of a protein to said oligonucleotide, or to modify a thermodynamic property of said oligonucleotide, more preferably to modify target RNA binding affinity. In another preferred embodiment, additional flanking sequences are complementary to sequences of the dystrophin pre-mRNA which are not present in said exon. Such flanking sequences are preferably capable of binding to or targeting sequences comprising or consisting of the branchpoint and/or the splice site acceptor or donor consensus sequences of said exon. In a preferred embodiment, such flanking sequences are capable of binding to or targeting sequences comprising or consisting of sequences of an intron of the dystrophin pre-mRNA which is adjacent to said exon.

Preferred oligonucleotides according to the invention are those wherein said oligonucleotide is represented by a nucleotide sequence comprising or consisting of SEQ ID NO: 8-1580 or SEQ ID NO: 1592-1607, or by a nucleotide sequence comprising or consisting of a fragment of SEQ ID NO: 8-1580 or SEQ ID NO: 1592-1607, preferably wherein said oligonucleotide is represented by a nucleotide sequence comprising or consisting of SEQ ID NO:453-613 or SEQ ID NO: 1592-1605 or SEQ ID NO: 1607, or by a nucleotide sequence comprising or consisting of a fragment of SEQ ID NO: 453-613 or SEQ ID NO 1592-1605 or SEQ ID NO: 1607, more preferably wherein said oligonucleotide is represented by a nucleotide sequence comprising or consisting of SEQ ID NO: 453, 455, 456, 459, 461, 462, 465, 467, 468, 471, 473, 474, 486, 483, 1592, 1593, 1594, 1595, 1596, 1597, 1598, 1599, 1600, 1601, 1602, 1603, 1604, 1605, or 1607 or by a nucleotide sequence comprising or consisting of a fragment of SEQ ID NO: 453, 455, 456, 459, 461, 462, 465, 467, 468, 471, 473, 474, 486, 483 1592, 1593, 1594, 1595, 1596, 1597, 1598, 1599, 1600, 1601, 1602, 1603, 1604, 1605, or 1607. Within the context of the invention, a fragment of a SEQ ID NO preferably means a nucleotide sequence comprising or consisting of at least 10 contiguous nucleotides from said SEQ ID NO.

More preferred oligonucleotides according to the invention are those wherein said oligonucleotide is represented by a nucleotide sequence comprising or consisting of SEQ ID NO: 8-1580 or SEQ ID NO: 1592-2099 or SEQ ID NO: 3000-6048, or by a nucleotide sequence comprising or consisting of a fragment of SEQ ID NO: 8-1580 or SEQ ID NO: 1592-2099 or SEQ ID NO: 3000-6048, more preferably wherein said oligonucleotide is represented by a nucleotide sequence comprising or consisting of SEQ ID NOs: 455, 459, 4528, 4531, 4532, 4533, 4535, 4542, 4548, or 4568, or by a nucleotide sequence comprising or consisting of a fragment of SEQ ID NOs: 455, 459, 4528, 4531, 4532, 4533, 4535, 4542, 4548, or 4568.

More preferred oligonucleotides according to the invention are those wherein said oligonucleotide is represented by a nucleotide sequence comprising or consisting of SEQ ID NO: 8-1580 or SEQ ID NO: 1592-2099 or SEQ ID NO: 3000-6048, or by a nucleotide sequence comprising or consisting of a fragment of SEQ ID NO: 8-1580 or SEQ ID NO: 1592-2099 or SEQ ID NO: 3000-6048, preferably wherein said oligonucleotide is represented by a nucleotide sequence comprising or consisting of SEQ ID NO: 453, 455, 456, 459, 461, 462, 465, 467, 468, 471, 473, 474, 483, or 486, or by a nucleotide sequence comprising or consisting of a fragment of SEQ ID NO: 453, 455, 456, 459, 461, 462, 465, 467, 468, 471, 473, 474, 483, or 486, more preferably wherein said oligonucleotide is represented by a nucleotide sequence comprising or consisting of SEQ ID NO: 452-613, 4528-4572, or by a nucleotide sequence comprising or consisting of a fragment of SEQ ID NO: 452-613, 4528-4572, most preferably wherein said oligonucleotide is represented by a nucleotide sequence comprising or consisting of SEQ ID NO: 455, 459, 4528, 4531, 4532, 4533, 4535, 4542, 4548, or 4568, or by a nucleotide sequence comprising or consisting of a fragment of SEQ ID NO: 455, 459, 4528, 4531, 4532, 4533, 4535, 4542, 4548, or 4568.

Preferred AONs are those wherein said oligonucleotide induces pre-mRNA splicing modulation, preferably said pre-mRNA splicing modulation alters production or composition of protein, which preferably comprises exon skipping or exon inclusion, wherein said pre-mRNA splicing modulation most preferably comprises exon skipping. This pre-mRNA splicing modulation is preferably used in the context of a therapeutic application as later defined herein.

The objective of pre-mRNA splicing modulation can be to alter production of protein, most often the protein the RNA codes for. This production can be altered through increase or decrease of the level of said production. This production can also be altered through alteration of the composition of the protein that is actually produced, for example when pre-mRNA splicing modulation results in inclusion or exclusion of one or more exons, and in a protein that has a different amino acid sequence. Preferably, such a protein with a different amino acid sequence has more functionality, or has a better functionality, or has at least one altered property, than the protein that is produced as a result of the disease or condition.

In the case of DMD, pre-mRNA splicing modulation can be applied to skip one or more specific exons in the dystrophin pre-mRNA in order to restore the open reading frame of the transcript and to induce the expression of a shorter but (more) functional dystrophin protein, with the ultimate goal to be able to interfere with the course of the disease. Similar strategies allow interference with the course of BMD. In the case of SMA pre-mRNA splicing modulation can be applied to enhance inclusion of exon 7 in the SMN2 gene and to increase levels of the survival of motor neuron protein, which decreases loss of motor neurons in the spinal cord and subsequent atrophy of voluntary muscles. As such, in a preferred embodiment is provided an oligonucleotide according to the invention, wherein said oligonucleotide induces pre-mRNA splicing modulation, wherein said pre-mRNA splicing modulation alters production of protein that is related to a disease or a condition, preferably wherein said disease or condition is Duchenne Muscular Dystrophy (DMD), Becker Muscular Dystrophy (BMD), or Spinal Muscular Atrophy (SMA).

Preferably, an AON is for use in the splicing modulation of a therapeutic pre-mRNA. An AON is preferably an oligonucleotide which is complementary to a specific sequence of the dystrophin or SMN2 pre-mRNA derived from the coding strand of a DNA of an individual. This oligonucleotide binds to or targets said sequence of said pre-mRNA. In the context of the invention, a therapeutic pre-mRNA may be called a diseased pre-mRNA of a gene involved in a genetic disease. Modulation of the splicing of a therapeutic pre-mRNA therefore allows the treatment of said genetic disease.

In the case of DMD or of BMD, pre-mRNA splicing modulation can be applied to skip one or more specific exons in the dystrophin pre-mRNA in order to restore the open reading frame of the transcript and to induce the expression of a shorter but (more) functional dystrophin protein, with the ultimate goal to be able to delay or even stop progression of the disease.

In a preferred embodiment, an oligonucleotide of the invention is used for inducing exon-skipping in the dystrophin pre-mRNA in a cell, in an organ, in a tissue and/or in an individual. Exon-skipping results in a mature dystrophin mRNA that does not contain a skipped exon and thus, when said exon codes for amino acids, can lead to the expression of a shorter protein product. The skipping of at least one exon is preferably induced by the binding of an AON to specific exon-internal sequences comprising splicing regulatory elements, the splice sites and/or intronic branchpoint sequences.

In a preferred embodiment, the invention also encompasses oligonucleotides as described above that are suitable for the skipping of multiple exons, sometimes referred to as multiskipping. Such an oligonucleotide according to the invention is capable of binding to a region of a first exon and to a region of a second exon within the same pre-mRNA, wherein said region of said second exon has at least 50% identity with said region of said first exon. These oligonucleotides are preferably capable of inducing the skipping of said first exon and said second exon of said pre-mRNA. Preferably, the skipping of additional exon(s) is also induced, wherein said additional exon(s) is/are preferably located in between said first and said second exon. The resulting transcript of said pre-mRNA, wherein said exons are skipped, is in-frame. More details of such oligonucleotides are provided in WO2014007620.

As defined herein a DMD pre-mRNA preferably means a pre-mRNA of a DMD gene coding for a dystrophin protein. A mutated DMD pre-mRNA corresponds to a pre-mRNA of a BMD or DMD patient with a mutation when compared to a wild type DMD pre-mRNA of a non-affected person, resulting in (reduced levels of) an aberrant protein (BMD), or in the absence of functional dystrophin (DMD). A DMD pre-mRNA is also named a dystrophin pre-mRNA. A DMD gene may also be named a dystrophin gene. Dystrophin and DMD may be used interchangeably throughout the application.

A patient is preferably intended to mean a patient having DMD or BMD as later defined herein or a patient susceptible to develop DMD or BMD due to his or her genetic background. In the case of a DMD patient, an oligonucleotide used will preferably correct one mutation as present in the DMD gene of said patient and create a protein that will look like a BMD protein: said protein will preferably be a functional or semi-functional dystrophin as later defined herein. In the case of a BMD patient, an oligonucleotide as used will preferably correct one mutation as present in the BMD gene of said patient and create a dystrophin which will be more functional than the dystrophin which was originally present in said BMD patient.

As defined herein, a functional dystrophin is preferably a wild type dystrophin corresponding to a protein having the amino acid sequence as identified in SEQ ID NO: 1. As defined herein, a semi-functional dystrophin is preferably a BMD-like dystrophin corresponding to a protein having an acting binding domain in its N terminal part (first 240 amino acids at the N terminus), a cysteine-rich domain (amino acid 3361 till 3685) and a C terminal domain (last 325 amino acids at the C terminus) each of these domains being present in a wild type dystrophin as known to the skilled person. The amino acids indicated herein correspond to amino acids of the wild type dystrophin being represented by SEQ ID NO:1. In other words, a functional or a semi-functional dystrophin is a dystrophin which exhibits at least to some extent an activity of a wild type dystrophin. "At least to some extent" preferably means at least 10%, 20%, 30%, 40%, 50%, 60%, 70%, 80%, 90%, 95% or 100% of a corresponding activity of a wild type functional dystrophin. In this context, an activity of a functional dystrophin is preferably binding to actin and to the dystrophin-associated glycoprotein complex (DGC or DAPC) (Ehmsen J et al, 2002).

Binding of dystrophin to actin and to the DGC or DAPC complex may be visualized by either co-immunoprecipitation using total protein extracts or immunofluorescence analysis of cross-sections using various antibodies reacting with the different members of the complex, from a control (non-DMD) biopsy of one from a muscle suspected to be dystrophic, pre- and/or post-treatment, as known to the skilled person.

Individuals or patients suffering from Duchenne muscular dystrophy typically have a mutation in the gene encoding dystrophin (the DMD or dystrophin gene) that prevents synthesis of the complete protein, i.e. a premature stop codon prevents the synthesis of the C-terminus. In Becker muscular dystrophy the dystrophin gene also comprises a mutation compared to the wild type but the mutation does typically not result in a premature stop codon and the C-terminus is typically synthesized. As a result a functional or semi-functional dystrophin protein is synthesized that has at least the same activity in kind as the wild type protein, although not necessarily the same amount of activity. The genome of a BMD patient typically encodes a dystrophin protein comprising the N terminal part (first 240 amino acids at the N terminus), a cysteine-rich domain (amino acid 3361 till 3685) and a C-terminal domain (last 325 amino acids at the C-terminus) but in the majority of cases its central rod shaped domain is shorter than the one of a wild type dystrophin (Monaco et al., 1988). Antisense oligonucleotide-induced exon skipping for the treatment of DMD is typically directed to overcome a premature stop in the pre-mRNA by skipping an exon, preferably in the central rod-domain shaped domain, to correct the open reading frame and allow synthesis of remainder of the dystrophin protein including the C-terminus, albeit that the protein is somewhat smaller as a result of a smaller rod domain. In a preferred embodiment, an individual having DMD and being treated by an oligonucleotide as defined herein will be provided a dystrophin which exhibits at least to some extent an activity of a wild type dystrophin. More preferably, if said individual is a Duchenne patient or is suspected to be a Duchenne patient, a functional or a semi-functional dystrophin is a dystrophin of an individual having BMD: typically said dystrophin is able to interact with both actin and the DGC or DAPC, but its central rod shaped domain may be shorter than the one of a wild type dystrophin (Monaco et al. , 1988). The central rod domain of wild type dystrophin comprises 24 spectrin-like repeats. For example, a central rod shaped domain of a dystrophin as provided herein may comprise 5 to 23, 10 to 22 or 12 to 18 spectrin-like repeats as long as it can bind to actin and to DGC.

Alleviating one or more symptom(s) of Duchenne Muscular Dystrophy or Becker Muscular Dystrophy in an individual using an oligonucleotide of the invention may be assessed by any of the following assays: prolongation of time to loss of walking, improvement of muscle strength, improvement of the ability to lift weight, improvement of the time taken to rise from the floor, improvement in the nine-meter walking time, improvement in the time taken for four-stairs climbing, improvement of the leg function grade, improvement of the pulmonary function, improvement of cardiac function, improvement of the quality of life. Each of these assays is known to the skilled person. As an example, the publication of Manzur et al. (2008), gives an extensive explanation of each of these assays. For each of these assays, as soon as a detectable improvement or prolongation of a parameter measured in an assay has been found, it will preferably mean that one or more symptoms of Duchenne Muscular Dystrophy or Becker Muscular Dystrophy has been alleviated in an individual using an oligonucleotide of the invention. Detectable improvement or prolongation is preferably a statistically significant improvement or prolongation as described in Hodgetts et al. (2006). Alternatively, the alleviation of one or more symptom(s) of Duchenne Muscular Dystrophy or Becker Muscular Dystrophy may be assessed by measuring an improvement of a muscle fiber function, integrity and/or survival. In a preferred method, one or more symptom(s) of a DMD or a BMD patient is/are alleviated and/or one or more characteristic(s) of one or more muscle cells from a DMD or a BMD patient is/are improved. Such symptoms or characteristics may be assessed at the cellular, tissue level or on the patient self.

An alleviation of one or more characteristics of a muscle cell from a patient may be assessed by any of the following assays on a myogenic cell or muscle cell from a patient: reduced calcium uptake by muscle cells, decreased collagen synthesis, altered morphology, altered lipid biosynthesis, decreased oxidative stress, and/or improved muscle fiber function, integrity, and/or survival. These parameters are usually assessed using immunofluorescence and/or histochemical analyses of cross sections of muscle biopsies.

The improvement of muscle fiber function, integrity and/or survival may be assessed using at least one of the following assays: a detectable decrease of creatine kinase in blood, a detectable decrease of necrosis of muscle fibers in a biopsy cross-section of a muscle suspected to be dystrophic, and/or a detectable increase of the homogeneity of the diameter of muscle fibers in a biopsy cross-section of a muscle suspected to be dystrophic. Each of these assays is known to the skilled person.

Creatine kinase may be detected in blood as described in Hodgetts et al. (2006). A detectable decrease in creatine kinase may mean a decrease of 5%, 10%, 20%, 30%, 40%, 50%, 60%, 70%, 80%, 90% or more compared to the concentration of creatine kinase in a same DMD or BMD patient before treatment.

A detectable decrease of necrosis of muscle fibers is preferably assessed in a muscle biopsy, more preferably as described in Hodgetts et al. (2006), using biopsy cross-sections. A detectable decrease of necrosis may be a decrease of 5%, 10%, 20%, 30%, 40%, 50%, 60%, 70%, 80%, 90% or more of the area wherein necrosis has been identified using biopsy cross-sections. The decrease is measured by comparison to the necrosis as assessed in a same DMD or BMD patient before treatment.

A detectable increase of the homogeneity of the diameter of a muscle fiber is preferably assessed in a muscle biopsy cross-section, more preferably as described in Hodgetts et al. (2006). The increase is measured by comparison to the homogeneity of the diameter of a muscle fiber in a same DMD or BMD patient before treatment

Preferably, an oligonucleotide of the invention provides said individual with a functional or a semi-functional dystrophin protein (typically in the case of DMD) and is able to, for at least in part decrease the production of an aberrant dystrophin protein in said individual (typically in the case of BMD).

Decreasing the production of an aberrant dystrophin mRNA, or aberrant dystrophin protein, preferably means that 90%, 80%, 70%, 60%, 50%, 40%, 30%, 20%, 10%, 5% or less of the initial amount of aberrant dystrophin mRNA, or aberrant dystrophin protein, is still detectable by RT-PCR (mRNA) or immunofluorescence or western blot analysis (protein). An aberrant dystrophin mRNA or protein is also referred to herein as a less functional (compared to a wild type functional dystrophin protein as earlier defined herein) or a non-functional dystrophin mRNA or protein. A non-functional dystrophin protein is preferably a dystrophin protein which is not able to bind actin and/or members of the DGC protein complex. A non-functional dystrophin protein or dystrophin mRNA does typically not have, or does not encode a dystrophin protein with an intact C-terminus of the protein. The detection of a functional or semi-functional dystrophin mRNA or protein may be done as for an aberrant dystrophin mRNA or protein.

Once a DMD patient is provided with a functional or a semi-functional dystrophin protein, at least part of the cause of DMD is taken away. Hence, it would then be expected that the symptoms of DMD are at least partly alleviated, or that the rate with which the symptoms worsen is decreased, resulting in a slower decline. The enhanced skipping frequency also increases the level of functional or a semi-functional dystrophin protein produced in a muscle cell of a DMD or BMD individual.

Spinal muscle atrophy (SMA) is a genetic disorder, often fatal, which results from the loss of the SMN protein encoded by the Survival Motor Neuron SMN gene. The SMN genes, SMN1 and SMN2, are located on chromosome 5 and SMA is caused by the loss of SMN1 from both chromosomes. SMN2, while it is almost identical to SMN1, is less effective at providing the SMN protein. SMN1 encodes a ubiquitously expressed 38 kDa SMN protein that is necessary for snRNP assembly, an essential process for cell survival. SMN1 and SMN2 differ by a critical C to T mutation at position 6 of exon 7 (C6U in the transcript of SMN2). C6U does not change the coding sequence, but is sufficient to cause exon 7 skipping in SMN2. This leads to an unstable truncated protein, SMNΔ7. The severity of SMA is affected by the efficiency with which SMN2, of which there are several copies, produces its SMN protein. In SMA patients, SMN2 generally fails to compensate for the loss of SMN1 because of exon 7 skipping, producing the unstable truncated protein, SMNΔ7, which cannot ensure cell survival. At present, available treatment for SMA consists of prevention and management of the secondary effect of chronic motor unit loss. There are no drug therapies available for the treatment or prevention of SMA. Antisense technology for splice switching can be used to provide new therapeutics for SMA treatment. Effective agents can alter splicing of SMN2 pre-mRNAs and are likely to be therapeutically useful. Another molecular basis of SMA can be the point mutation (E134K).

Preferred AONs enhance the level of exon 7-containing SMN2 mRNA relative to exon 7-deleted SMN2 mRNA in a cell. Preferred AONs are preferably of adequate length and complementarity (more preferably as defined later herein) so that the AONs specifically hybridize to a region within the SMN2 gene, such that the level of exon 7-containing SMN2 mRNA relative to exon-deleted SMN2 mRNA in the cell is enhanced. Preferred AONs comprise or consist of SEQ ID NO: 1400-1579. A more preferred AON comprises or consists of SEQ ID NO: 1490. In the case of SMA, pre-mRNA splicing modulation can be applied to include one or more exons, preferably exon 7, in the SMN2 pre-mRNA in order to increase functional SMN2 levels by increasing expression of exon 7-containing SMN2 mRNA or protein. This has the ultimate goal to be able to delay or even stop progression of the disease.

In a preferred embodiment, an AON is used for inducing exon 7-inclusion in an SMN pre-mRNA, preferably the SMN2 pre-mRNA, in a cell, in an organ, in a tissue and/or in an individual. Exon-inclusion preferably results in a mature SMN mRNA that contains an otherwise skipped exon 7 and thus can lead to the expression of a more functional protein product. The inclusion of at least one exon, preferably exon 7, is preferably induced by the binding of an AON to specific sequences, preferably intron-internal sequences, comprising splicing regulatory elements, splice sites, and/or intronic branchpoint sequences.

As defined herein an SMN1 pre-mRNA preferably means a pre-mRNA of an SMN1 gene coding for an SMN protein. As defined herein an SMN2 pre-mRNA preferably means a pre-mRNA of an SMN2 gene coding for an SMN protein. In cases where the pre-mRNA of subjects suffering SMA is discussed, an SMN2 pre-mRNA is sometimes also named an SMN pre-mRNA; this is because in SMA patients no SMN1 gene is present, and thus all SMN pre-mRNA is SMN2 pre-mRNA. An SMN2 gene may also be named an SMN gene in such a case.

A patient is preferably intended to mean a patient having SMA as defined herein or a patient susceptible to develop SMA due to his or her genetic background. In the case of an SMA patient, an oligonucleotide used will preferably promote inclusion of exon 7 as present in the SMN2 gene of said patient and create a functional SMN protein and not an SMNΔ7 protein: said protein will preferably be a functional or semi-functional SMN as later defined herein. In the case of an SMA patient, an oligonucleotide as used will preferably suppress or reduce the effect of one mutation as present in the SMN2 gene of said patient and create increased levels of an SMN protein which will be more functional than the SMN protein, often the SMNΔ7 protein, which was originally present in said SMA patient. Preferably, the ratio of SMN protein to SMNΔ7 protein shifts in favour of the functional SMN protein. Preferred molar ratios of SMN protein to SMNΔ7 protein that are detected after treatment are 5:4, 5:3, 5:2, 5:1, or 10:1. Most preferably, SMNΔ7 protein can no longer be detected, or only in trace amounts.

As defined herein, a functional SMN protein is preferably a wild type SMN corresponding to a protein having the amino acid sequence as identified in SEQ ID NO: 1581. Preferably, a functional SMN protein comprises exon 7. This exon 7 is identified in SEQ ID NO: 1584. In other words, a functional or a semi-functional SMN protein is an SMN protein which exhibits at least to some extent an activity of a wild type SMN protein. "At least to some extent" preferably means at least 10%, 20%, 30%, 40%, 50%, 60%, 70%, 80%, 90%, 95% or 100% of a corresponding activity of a wild type functional SMN protein. In this context, preferred activities of a functional SMN protein relate to telomerase regeneration, to transcriptional regulation, and to cellular trafficking, as known to a skilled person. More preferred activities of a functional SMN protein are the formation of functional snRNP assemblies, and the interaction with Sm proteins (Smith proteins).

Functional SMN binds to the Arg and Gly-rich C-terminal tails of the Sm D1 and D3 proteins (Selenko et al., 2001). In vitro binding assays can be performed to study this interaction, for example through expression of C-terminal tails of Sm D1 and Sm D3 as glutathione-S-transferase (GST) fusion proteins and subsequent pull-down experiments with isolated SMN protein. Less functional SMN will show less or no interaction. Such assays can be performed using total protein extracts. Alternately, exon 7-included SMN can be detected through immunofluorescence analysis of biopsy cross-sections using various antibodies interacting with the region coded by exon 7, or with part of that region, or with folds only present in SMN comprising exon 7. Comparison with non-SMA (control) biopsies can be appropriate, as known to a skilled person.

In a preferred embodiment, an individual having SMA and being treated by an AON as defined herein will be provided an SMN protein which exhibits at least to some extent an activity of a wild type SMN protein, as typically encoded by the SMN1 gene. More preferably, if said individual is an SMA patient or is suspected to be an SMA patient, a functional SMN protein is an SMN protein with exon 7 included, as typically encoded by the SMN1 gene.

Alleviating one or more symptom(s) of SMA in an individual using an AON may be assessed by any of the following assays: improvement in weight gain of a subject, improvement in motor activity of a subject, and increased survival time, either of a subject, or of motor neuron cells, and increased production of functional SMN. Each of these parameters are known to the skilled person, and can be assayed routinely. For each of these assays, as soon as a detectable improvement or prolongation of a parameter measured in an assay has been found, it will preferably mean that one or more symptoms of SMA has been alleviated in an individual using an oligonucleotide according to the invention. Detectable improvement or prolongation is preferably a statistically significant improvement or prolongation. Alternatively, the alleviation of one or more symptom(s) of SMA may be assessed by measuring an improvement of a muscle function, integrity, and/or survival. In a preferred method, one or more symptom(s) of an SMA patient is/are alleviated and/or one or more characteristic(s) of one or more muscle cells from an SMA patient is/are improved. Such symptoms or characteristics may be assessed at the cellular, tissue level or on the patient's self.

An alleviation of one or more characteristics of a motor neuron cell from a patient may be assessed by any of the following assays on a cell from a patient: reduced calcium uptake, decreased snRNP production, decreased collagen synthesis, altered morphology, altered lipid biosynthesis, decreased oxidative stress, and/or improved muscle function, integrity, and/or survival. These parameters are usually assessed using immunofluorescence and/or histochemical analyses of cross sections of biopsies, such as muscle biopsies.

A detectable increase in motor neuron survival is preferably assessed in a muscle biopsy, using biopsy cross-sections. A detectable increase of survival may be an increase of 5%, 10%, 20%, 30%, 40%, 50%, 60%, 70%, 80%, 90% or more as compared to untreated control samples, or to known or previous rates of survival. The increase is measured by comparison to the survival as assessed in a same SMA patient before treatment.

A detectable increase in motor neuron survival can be assessed as known to the skilled person, for example using methods as described in Lunn et al., 2004.

Preferably, an AON provides said individual with a functional or a semi-functional SMN protein and is able to, for at least in part, decrease the production of an aberrant SMN protein such as SMNΔ7 in said individual.

Decreasing the production of an aberrant SMN mRNA, or aberrant SMN protein, preferably means that 90%, 80%, 70%, 60%, 50%, 40%, 30%, 20%, 10%, 5% or less of the initial amount of aberrant SMN mRNA, or aberrant SMN protein, is still detectable by RT-PCR (mRNA) or immunofluorescence or western blot analysis (protein). An aberrant SMN mRNA or protein is also referred to herein as a less functional (compared to a wild type functional SMN protein as earlier defined herein) or a non-functional SMN mRNA or protein. A non-functional SMN protein is preferably an SMN protein which is not able to bind Sm proteins and/or which does not promote, or which hampers, snRNP assembly. A non-functional SMN protein or SMN mRNA does typically not have, or does not encode, the amino acid sequence encoded by exon 7. The detection of a functional or semi-functional SMN mRNA or protein may be done as for an aberrant SMN mRNA or protein.

Once a SMA patient is provided with a functional or a semi-functional SMN protein, at least part of the cause of SMA is taken away. Hence, it would then be expected that the symptoms of SMA are at least partly alleviated, or that the rate with which the symptoms worsen is decreased, resulting in a slower decline. The enhanced inclusion frequency also increases the level of functional or semi-functional SMN protein produced in a cell of an SMA individual.

Exons and introns contain one or more specific sequences comprising splicing regulatory elements which have shown to be effective targets for antisense oligonucleotides. One embodiment therefore provides an oligonucleotide for providing said individual with a functional or semi-functional dystrophin or SMN protein wherein said oligonucleotide comprises a sequence which is specifically binding and/or blocking these splicing regulatory elements in a dystrophin or SMN2 pre-mRNA exon or intron. In addition, since an exon will only be included into the resulting mRNA when both the splice sites are recognized by the spliceosome complex, splice sites are other targets for an oligonucleotide of the invention. One embodiment therefore provides an oligonucleotide for providing said individual with a functional or semi-functional dystrophin or SMN protein wherein said oligonucleotide comprises a sequence which is specifically binding and/or blocking one of or both the splice sites of an exon of a dystrophin or SMN2 pre-mRNA. Usually a splice site of an exon comprises 1, 2, 3, or more nucleotides present in said exon and 1, 2, 3, or more nucleotides present in an adjacent or neighboring intron. In one embodiment an oligonucleotide is used which is solely binding to an intron region of a dystrophin or SMN2 pre-mRNA. This is however not necessary: it is also possible to use an oligonucleotide which targets, or binds an intron-specific sequence as well as exon-specific sequence. Of course, an oligonucleotide is not necessarily binding to the entire sequence of a dystrophin or SMN2 exon or intron. Oligonucleotides which are specifically binding part of such exon or intron are preferred. An oligonucleotide is used, said oligonucleotide is preferably complementary to, binds to or targets at least part of an exon and/or intron, said part having at least 10, 11, 12, 13, 14, 15, 16, 17, 18, 19, 20, 21, 22, 23, 24, 25, 26, 27, 28, 29, 30, 31, 32, or 33 nucleotides.

Splicing of a pre-mRNA occurs via two sequential transesterification reactions involving an intronic branch point and a splice site of an adjacent intron. Hence, an oligonucleotide is preferably used for exon skipping, wherein said oligonucleotide comprises a sequence which is binding to such branch point and/or splice site. Preferably said splice site and/or branch point is present in a dystrophin pre-mRNA.

Since splice sites contain consensus sequences, the use of an oligonucleotide part or a functional equivalent thereof comprising a sequence which is capable of binding to a splice site involves the risk of promiscuous hybridization. Hybridization of said oligonucleotide to other splice sites than the sites of the exon to be skipped could easily interfere with the accuracy of the splicing process. To overcome these and other potential problems related to the use of an oligonucleotide which is binding to a splice site, most preferred embodiment provides an oligonucleotide for providing said individual with a functional or a semi-functional dystrophin or SMN protein, wherein said oligonucleotide or a functional equivalent thereof, binding to a specific part of a dystrophin pre-mRNA exon or SMN2 pre-mRNA exon or intron. Exons contain coding sequences which are typically more specific that the non-coding intron sequences. Preferably, said oligonucleotide binding to a specific part of a dystrophin pre-mRNA exon is capable of specifically blocking, interfering and/or inhibiting a splicing regulatory sequence and/or structure of the anticipated exon(s) in said dystrophin or SMN2 pre-mRNA. Interfering with such splicing regulatory sequence and/or structure has the advantage that such elements are located within the exon. The risk for sequence-related off-target effects is therefore limited. In the case of exon skipping, by providing an oligonucleotide for the interior of the exon to be skipped, it is possible to mask the exon from the splicing apparatus. The failure of the splicing apparatus to recognize the exon to be skipped thus leads to exclusion of the exon from the final mRNA. In the case of exon inclusion, by providing an oligonucleotide for blocking for example an intronic splicing silencer (ISS), increase in exon inclusion is achieved. The hybridization of AONs to the ISS region can displace trans-acting negative repressors and/or can unwind a cis-acting RNA stem-loop that interferes with the binding of U1 small nuclear RNA at the 5' splice site of the exon to be included These embodiments do not interfere directly with the enzymatic process of the splicing machinery (the joining of the exons). It is thought that this allows the method to be more specific and/or reliable.

Within the context of the invention, an oligonucleotide of the invention may comprise a functional equivalent of an oligonucleotide. A functional equivalent of an oligonucleotide preferably means an oligonucleotide as defined herein wherein one or more nucleotides have been substituted and wherein an activity of said functional equivalent is retained to at least some extent. Preferably, an activity of said oligonucleotide comprising a functional equivalent of an oligonucleotide is providing a functional or a semi-functional dystrophin or SMN protein. Said activity of said oligonucleotide comprising a functional equivalent of an oligonucleotide is therefore preferably assessed by quantifying the amount of a functional or a semi-functional dystrophin or SMN protein. A functional or semi-functional dystrophin is herein preferably defined as being a dystrophin able to bind actin and members of the DGC (or DAPC) protein complex. The assessment of said activity of said functional equivalent of an oligonucleotide is preferably done by RT-PCR and sequencing (on RNA level; for detection of specific exon skipping (DMD) or inclusion (SMA)), or by immunofluorescence and Western blot analyses (on protein level: for detection of protein restoration). Said activity is preferably retained to at least some extent when it represents at least 50%, or at least 60%, or at least 70% or at least 80% or at least 90% or at least 95% or more of corresponding activity of said oligonucleotide the functional equivalent derives from. Throughout this application, when the word oligonucleotide is used it may be replaced by a functional equivalent thereof as defined herein. Throughout this application, when the word oligonucleotide is used it may be replaced by an antisense oligonucleotide as defined herein unless otherwise indicated.

Hence, the use of an oligonucleotide according to the invention or a functional equivalent thereof, comprising a 2'-O-methyl monomer, preferably a 2'-O-methyl RNA monomer, or consisting of 2'-O-methyl RNA and optionally comprising phosphorothioate, and comprising at least one BNA scaffold modification with or without a 5-methylpyrimidine (i.e. a 5-methylcytosine and/or a 5-methyluracil) base and being represented by a nucleotide sequence comprising or consisting of a sequence which is complementary to, binds or targets or hybridizes with a dystrophin or SMN2 pre-mRNA exon or intron is assumed to have a positive effect on at least one of the parameters of said oligonucleotide, as has already been defined herein, when compared to their counterparts which do not comprise any BNA scaffold modification with/or without 5-methylcytosine and/or 5-methyluracil as indicated earlier herein, and is therefore assumed to exhibit an improved therapeutic result in a DMD or a BMD or an SMA cell of a patient and/or in a DMD or a BMD or an SMA patient. Such a therapeutic result may be characterized by alleviating one or more symptom(s) of DMD or BMD or SMA. Such a therapeutic result may also or alternately be characterized by:
- reducing the rate of increase or worsening of one or more of said symptoms, and/or
- alleviating one or more characteristics of a muscle cell from a patient and/or
- providing said individual with a functional or semi-functional dystrophin or SMN protein and/or
- decreasing loss of motor neurons in the spinal cord, and/or
- decreasing atrophy of voluntary muscles, and/or- at least in part decreasing the production of an aberrant dystrophin protein in said individual.
Each of these features has already been defined herein.

Preferably, an oligonucleotide is represented by a nucleotide sequence which comprises or consists of a sequence which is binding to, targeting or being complementary to at least a part of dystrophin or SMN2 pre-mRNA, said oligonucleotide having a length of at least 10 nucleotides. However, the length of said oligonucleotide may be at least 11, 12, 13, 14, 15, 16, 17, 18, 19, 20, 21, 22, 23, 24, 25, 26, 27, 28, 29, 30, 31, 32, or 33 nucleotides.

One preferred embodiment provides an oligonucleotide for providing said individual with a functional or a semi-functional dystrophin or SMN protein, said oligonucleotide or a functional equivalent thereof, being represented by a sequence which comprises:
- a sequence which binds, targets, hybridizes or is complementary to a region of a dystrophin or SMN2 pre-mRNA exon that is hybridized to another part of a dystrophin or SMN2 pre-mRNA exon (closed structure), and
- a sequence which binds, targets, hybridizes or is complementary to a region of a dystrophin or SMN2 pre-mRNA exon that is not hybridized in said dystrophin or SMN2 pre-mRNA (open structure).

For this embodiment, reference is made to the WO 2004/083446 patent application. RNA molecules exhibit strong secondary structures, mostly due to base pairing of complementary or partly complementary stretches within the same RNA. It has long since been thought that structures in the RNA play a role in the function of the RNA. Without being bound by theory, it is believed that the secondary structure of the RNA of an exon plays a role in structuring the splicing process. Through its structure, an exon is recognized as a part that needs to be included in the mRNA. In an embodiment, an oligonucleotide is capable of interfering with the structure of the exon and therefore capable of interfering with the splicing apparatus of said exon, masking the exon from the splicing apparatus and thereby inducing the skipping of said exon. It has been found that many oligonucleotides indeed comprise this capacity, some more efficient than others. Without being bound by theory it is thought that the overlap with an open structure improves the invasion efficiency of the oligonucleotide (i.e. increases the efficiency with which the oligonucleotide can enter the structure), whereas the overlap with the closed structure subsequently increases the efficiency of interfering with the secondary structure of the RNA of the exon. It is found that the length of the partial complementarity to both the closed and the open structure is not extremely restricted. We have observed high efficiencies with compounds comprising oligonucleotides with variable lengths of complementarity in either structure. The term (reverse) complementarity is used herein to refer to a stretch of nucleic acids that can hybridise to another stretch of nucleic acids under physiological conditions. An antisense strand is generally said to be complementary to a matching sense strand. In this context, an antisense oligonucleotide is complementary to its target. Hybridization conditions are later defined herein. It is thus not absolutely required that all the bases in the region of complementarity are capable of pairing with bases in the opposing strand. For instance, when designing an antisense oligonucleotide, one may want to incorporate for instance a residue that does not base pair with the base on the complementary strand. Mismatches may to some extent be allowed, if under the circumstances in the cell, the stretch of nucleotides is capable of hybridizing to the complementary part.

In a preferred embodiment a complementary part of an antisense oligonucleotide (either to said open or to said closed structure) comprises at least 3, and more preferably at least 4 consecutive nucleotides. The complementary regions are preferably designed such that, when combined, they are specific for an exon in a pre-mRNA. Such specificity may be created with various lengths of complementary regions as this depends on the actual sequences in other (pre-)mRNA in the system. The risk that also one or more other pre-mRNA will be able to hybridise to an oligonucleotide decreases with increasing size of said oligonucleotide. It is clear that an antisense oligonucleotide comprising mismatches in the region of complementarity but that retain the capacity to hybridise to the targeted region(s) in the pre-mRNA, can be used in the present invention. However, preferably at least the complementary parts do not comprise such mismatches as these typically have a higher efficiency and a higher specificity than oligonucleotide having such mismatches in one or more complementary regions. It is thought that higher hybridisation strengths, (i.e. increasing number of interactions with the opposing strand) are favourable in increasing the efficiency of the process of interfering with the splicing machinery of the system.

Preferably, for AONs that are suitable for inducing single-exon skipping, the complementarity is from 90 to 100%. In general this allows for 1 or 2 mismatch(es) in an oligonucleotide of 20 nucleotides or 1 to 4 mismatches in an oligonucleotide of 40 nucleotides. Therefore, we may have 1, 2, 3, 4, 5 mismatches in an oligonucleotide of 10 to 50 nucleotides. Preferably, 0, 1 or 2 mismatches are present in an oligonucleotide of 10 to 50 nucleotides.

For so-called multiskipping AONs (AONs that are capable of binding to a region of a first exon and to a region of another exon (i.e. a second exon) within the same pre-mRNA, wherein said region of said second exon has at least 50% identity with said region of said first exon) there is preferably at least 80% complementary to said region of said first exon and at least 45% complementary to said region of said second exon. More preferably, said antisense oligonucleotide is at least 85%, 90%, 95% or 100% complementary to said region of said first and at least 50%, 55%, 60%, 65%, 70%, 75% ,80%, 85%, 90%, 95% or 100% complementary to said region of said second exon. The complementarity is preferably but not necessarily assessed over the whole length of the oligonucleotide.

For such so-called multiskipping AONs, a region of a first exon may be at least 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, or up to 90, 100, 110, 120, 130, 140, 150, 160, 170, 180, 190, 200, or more nucleotides. A region of a first exon may also be defined as being at least 1%, 5%, 10%, 20%, 30%, 40%, 50%, 60%, 70%, 80%, 90% or 100% of the length of said exon. A region of a first exon may be called an identity region.

For such so-called multiskipping AONs, a region of a second exon may be at least 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79,80 or up to 90, 100, 110, 120, 130, 140, 150, 160, 170, 180, 190, 200, or more nucleotides. A region of a second exon may be defined as being at least 1%, 5%, 10%, 20%, 30%, 40%, 50%, 60%, 70%, 80%, 90% or 100% of the length of said exon. A region of a second exon may be called an identity region. Preferred additional characteristics of so-called multiskipping AONs are described in WO2014007620.

The structure (i.e. open and closed structures) is best analyzed in the context of the pre-mRNA wherein the exon resides. Such structure may be analyzed in the actual RNA. However, it is currently possible to predict the secondary structure of an RNA molecule (at lowest energy costs) quite well using structure-modeling programs. Non-limiting examples of a suitable program are RNA structure version 4.5 or RNA mfold version 3.5 (Zuker et al., 2003). A person skilled in the art will be able to predict, with suitable reproducibility, a likely structure of an exon, given a nucleotide sequence. Best predictions are obtained when providing such modeling programs with both said exon and flanking intron sequences. It is typically not necessary to model the structure of the entire pre-mRNA.

The open and closed structure to which the oligonucleotide of an oligonucleotide is directed, are preferably adjacent to one another. It is thought that in this way the annealing of the oligonucleotide to the open structure induces opening of the closed structure whereupon annealing progresses into this closed structure. Through this action the previously closed structure assumes a different conformation. However, when potential (cryptic) splice acceptor and/or donor sequences are present within the targeted exon, occasionally a new exon inclusion signal is generated defining a different (neo) exon, i.e. with a different 5' end, a different 3' end, or both. This type of activity is within the scope of the present invention as the targeted exon is excluded from the mRNA. The presence of a new exon, containing part of the targeted exon, in the mRNA does not alter the fact that the targeted exon, as such, is excluded. The inclusion of a neo-exon can be seen as a side effect which occurs only occasionally. There are two possibilities when exon skipping is used to restore (part of) an open reading frame of dystrophin that is disrupted as a result of a mutation. One is that the neo-exon is functional in the restoration of the reading frame, whereas in the other case the reading frame is not restored. When selecting a compound comprising an oligonucleotide for restoring dystrophin reading frames by means of exon-skipping it is of course clear that under these conditions only those compounds comprising those oligonucleotide are selected that indeed result in exon-skipping that restores the dystrophin open reading frame, with or without a neo-exon.

Further provided is an oligonucleotide for providing said individual with a functional or a semi-functional dystrophin protein, wherein said oligonucleotide or a functional equivalent thereof is an oligonucleotide as described above, i.e. it comprises a 2'-O-methyl monomer or consists of 2'-O-methyl monomers, preferably 2'-O-methyl RNA monomers, and optionally comprises phosphorothioate, and further comprises a BNA scaffold with or without a 5-methylpyrimidine (i.e. a 5-methylcytosine, and/or a 5-methyluracil) and is represented by a nucleotide sequence comprising a sequence that is complementary to or binds or targets or hybridizes with a binding site for a serine-arginine (SR) protein in RNA of an exon of a dystrophin pre-mRNA. In WO 2006/112705 patent application we have disclosed the presence of a correlation between the effectivity of an exon-internal antisense oligonucleotide in inducing exon skipping and the presence of a (for example by ESEfinder) predicted SR binding site in the target pre-mRNA site of said AON. Therefore, in one embodiment an oligonucleotide is generated comprising determining a (putative) binding site for an SR (Ser-Arg) protein in RNA of a dystrophin exon and producing a corresponding compound comprising oligonucleotide that is complementary to, binds or targets or hybridizes with said RNA and that at least partly overlaps said (putative) binding site. The term "at least partly overlaps" is defined herein as to comprise an overlap of only a single nucleotide of an SR binding site as well as multiple nucleotides of said binding site as well as a complete overlap of said binding site. This embodiment preferably further comprises determining from a secondary structure of said RNA, a region that is hybridized to another part of said RNA (closed structure) and a region that is not hybridized in said structure (open structure), and subsequently generating an oligonucleotide that at least partly overlaps said (putative) binding site and that overlaps at least part of said closed structure and overlaps at least part of said open structure. In this way we increase the chance of obtaining an oligonucleotide that is capable of interfering with the exon inclusion from the pre-mRNA into mRNA. It is possible that a first selected SR-binding region does not have the requested open-closed structure in which case another (second) SR protein binding site is selected which is then subsequently tested for the presence of an open-closed structure. This process is continued until a sequence is identified which contains an SR protein binding site as well as a(n) (partly overlapping) open-closed structure. This sequence is then used to design an oligonucleotide which is complementary to said sequence.

Such a method for generating an antisense oligonucleotide is also performed by reversing the described order, i.e. first generating an oligonucleotide comprising determining, from a secondary structure of RNA from a dystrophin exon, a region that assumes a structure that is hybridised to another part of said RNA (closed structure) and a region that is not hybridised in said structure (open structure), and subsequently generating an oligonucleotide, of which at least a part of said oligonucleotide is complementary to said closed structure and of which at least another part of said oligonucleotide is complementary to said open structure. This is then followed by determining whether an SR protein binding site at least overlaps with said open/closed structure. In this way the method of WO 2004/083446 is improved. In yet another embodiment the selections are performed simultaneously.

Without wishing to be bound by any theory it is currently thought that use of an oligonucleotide directed to or targeting an SR protein binding site results in (at least partly) impairing the binding of an SR protein to the binding site of an SR protein which results in disrupted or impaired splicing.

Preferably, an open/closed structure and an SR protein binding site partly overlap and even more preferred an open/closed structure completely overlaps an SR protein binding site or an SR protein binding site completely overlaps an open/closed structure. This allows for an improved disruption of exon inclusion.

Besides consensus splice site and branchpoint intronic sequences, many (if not all) exons contain splicing regulatory sequences such as but not limited to exonic splicing enhancer (ESE) sequences to facilitate the recognition of genuine splice sites by the spliceosome (Cartegni et al., 2002; and Cartegni et al., 2003). A subgroup of splicing factors, called the SR proteins, can bind to these ESEs and recruit other splicing factors, such as U1 and U2AF to (weakly defined) splice sites. The binding sites of the four most abundant SR proteins (SF2/ASF, SC35, SRp40 and SRp55) have been analyzed in detail and these results are implemented in ESEfinder, a web source that predicts potential binding sites for these SR proteins (Cartegni et al., 2002; and Cartegni et al., 2003). In embodiments where the AON is for exon skipping, there is a correlation between the effectiveness of an AON and the presence/absence of an SF2/ASF, SC35 and SRp40 binding site in the site targeted by said AON. In a preferred embodiment, the invention thus provides an oligonucleotide as described above, which is complementary to or targets or binds a binding site for a SR protein. Preferably, said SR protein is SF2/ASF or SC35 or SRp40. In embodiments where the AON is for exon inclusion, there is a correlation between the effectiveness of an AON and the presence of a U1 small nuclear RNA binding site or a heterogeneous nuclear ribonucleoprotein (hnRNP) binding site or a small nuclear ribonucleoprotein (snRNP) in the site targeted by said AON. In a preferred embodiment, the invention provides an oligonucleotide as described above, which is complementary to or targets or binds a binding site for a snRNA, such as U1 small nuclear RNA, a snRNP, or a hnRNP.

In one embodiment a DMD patient is provided with a functional or a semi-functional dystrophin protein by using an oligonucleotide as described above, or a functional equivalent thereof, i.e. an oligonucleotide comprising a 2'-*O*-methyl monomer, preferably a 2'-O-methyl RNA monomer, or consisting of 2'-*O*-methyl RNA and comprising at least one BNA scaffold with or without a 5-methylpyrimidine (i.e. a 5-methylcytosine, and/or a 5-methyluracil) base and being capable of specifically binding or targeting a regulatory RNA sequence which is required for the correct splicing of a dystrophin exon in a transcript. Several cis-acting RNA sequences are required for the correct splicing of exons in a transcript. In particular, elements such as an exonic splicing enhancer (ESE), an exon recognition sequence (ERS), and/or an exonic splicing silencer (ESS) and/or an intronic splicing silencer (ISS) are identified to regulate specific and efficient splicing of constitutive and alternative exons. Using a sequence-specific antisense oligonucleotide (AON) that binds to, targets or is complementary to the elements, their regulatory function is disturbed so that the exon is skipped or included, as shown for DMD or SMA. Hence, in one preferred embodiment, an oligonucleotide or a functional equivalent thereof is used which is complementary to, binds or targets an exonic splicing enhancer (ESE), an exon recognition sequence (ERS), and/or an exonic splicing silencer (ESS) and/or an intronic splicing silencer (ISS).

In a preferred embodiment, an oligonucleotide of the invention that is suitable for inducing the skipping of a single exon comprises or consists of a sequence that is complementary to or binds or targets or hybridizes with at least a part of dystrophin pre-mRNA exons 44, 45, 46, 47, 48, 49, 50, 51, 52, 53, 54, or 55, said part having at least 10 nucleotides. However, said part may also have at least 11, 12, 13, 14, 15, 16, 17, 18, 19, 20, 21, 22, 23, 24, 25, 26, 27, 28, 29, 30, 31, 32, or, 33 nucleotides. For the dystrophin exons identified above, we provide a stretch of nucleotides (SEQ ID NO: 2 to 7) from said exon to which an oligonucleotide preferably binds or is complementary to or targets or hybridizes with.

In a preferred embodiment, an oligonucleotide of the invention that is suitable for so-called multiskipping as defined earlier herein induces the skipping of the following dystrophin exons: exons 8 to 19, exons 9 to 22, exons 9 to 30, exons 10 to 18, exons 10 to 30, exons 10 to 42, exons 10 to 47, exons 10 to 57, exons 10 to 60, exons 11 to 23, exons 13 to 30, exons 23 to 42, exons 34 to 53, exons 40 to 53, exons 44 to 56, exons 45 to 51, exons 45 to 53, exons 45 to 55, exons 45 to 60 or exons 56 to 60. Preferably, such a so-called multiskipping oligonucleotide according to the invention comprises or consists of a sequence that is capable of binding to, targeting, hybridizing and/or is reverse-complementary to a region of a first exon of the dystrophin pre-mRNA such that the reverse-complementary part is at least 30% of the length of said oligonucleotide of the invention, more preferably at least 40%, even more preferably at least 50%, even more preferably at least 60%, even more preferably at least 70%, even more preferably at least 80%, even more preferably at least 90% or even more preferably at least 95%, or even more preferably 98% and most preferably up to 100%. In this context, a first exon is preferably exon 8, 9, 10, 11, 13, 23, 34, 40, 44, 45, or 56 of dystrophin pre-mRNA as defined herein. Said oligonucleotide may comprise additional flanking sequences. In a more preferred embodiment, the length of said reverse-complementary part of said oligonucleotide is at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides. Several types of flanking sequences may be used. Preferably, flanking sequences are used to modify the binding of a protein to said oligonucleotide, or to modify a thermodynamic property of said oligonucleotide, more preferably to modify target RNA binding affinity. In another preferred embodiment, additional flanking sequences are reverse-complementary to sequences of the dystrophin pre-mRNA which are not present in said exon.

A preferred oligonucleotide comprises:
i)
   la) at least one 2'-substituted monomer, preferably an RNA monomer or a 2'-O-substituted RNA monomer, and optionally a phosphorothioate backbone linkage, or
   Ib) only 2'-substituted monomers, preferably RNA monomers or 2'-O-substituted RNA monomers, linked by phosphorothioate backbone linkages and/or by phosphodiester linkages,
ii) a 5-methylcytosine and/or a 5-methyluracil base and
iii) at least one monomer comprising a bicyclic nucleic acid (BNA) scaffold modification,
and binds or is complementary to or targets or hybridizes with a continuous stretch of at least 10 and up to 33 nucleotides within at least one of the following exon nucleotide sequences selected from SEQ ID NO: 2 to 7, more preferably from:

5’-
GCGAUUUGACAGAUCUGUUGAGAAAUGGCGGCGUUUUCAUUAUGAUAUAAAGAUAUU
UAAUCAGUGGCUAACAGAAGCUGAACAGUUUCUCAGAAAGACACAAAUUCCUGAGAAU
UGGGAACAUGCUAAAUACAAAUGGUAUCUUAAG-3’ (SEQ ID NO: 2) for skipping or at
least skipping of exon 44;

5'-
GAACUCCAGGAUGGCAUUGGGCAGCGGCAAACUGUUGUCAGAACAUUGAAUGCAACU
GGGGAAGAAAUAAUUCAGCAAUCCUCAAAAACAGAUGCCAGUAUUCUACAGGAAAAAU
UGGGAAGCCUGAAUCUGCGGUGGCAGGAGGUCUGCAAACAGCUGUCAGACAGAAAAA
AGAG-3' (SEQ ID NO: 3) for skipping or at least skipping of exon 45;

5'-
CUCCUACUCAGACUGUUACUCUGGUGACACAACCUGUGGUUACUAAGGAAACUGCCA
UCUCCAAACUAGAAAUGCCAUCUUCCUUGAUGUUGGAGGUACCUGCUCUGGCAGAUU
UCAACCGGGCUUGGACAGAACUUACCGACUGGCUUUCUCUGCUUGAUCAAGUUAUAA
AAUCACAGAGGGUGAUGGUGGGUGACCUUGAGGAUAUCAACGAGAUGAUCAUCAAGC
AGAAG-3' (SEQ ID NO: 4) for skipping or at least skipping of exon 51;

5’-
GCAACAAUGCAGGAUUUGGAACAGAGGCGUCCCCAGUUGGAAGAACUCAUUACCGCU
GCCCAAAAUUUGAAAAACAAGACCAGCAAUCAAGAGGCUAGAACAAUCAUUACGGAUC
GAA-3’ (SEQ ID NO: 5) for skipping or at least skipping of exon 52;

5'-
UUGAAAGAAUUCAGAAUCAGUGGGAUGAAGUACAAGAACACCUUCAGAACCGGAGGCA
ACAGUUGAAUGAAAUGUUAAAGGAUUCAACACAAUGGCUGGAAGCUAAGGAAGAAGCU
GAGCAGGUCUUAGGACAGGCCAGAGCCAAGCUUGAGUCAUGGAAGGAGGGUCCCUAU
ACAGUAGAUGCAAUCCAAAAGAAAAUCACAGAAACCAAG-3' (SEQ ID NO: 6) for skipping
or at least skipping of exon 53;

5’-
GGUGAGUGAGCGAGAGGCUGCUUUGGAAGAAACUCAUAGAUUACUGCAACAGUUCCC
CCUGGACCUGGAAAAGUUUCUUGCCUGGCUUACAGAAGCUGAAACAACUGCCAAUGU
CCUACAGGAUGCUACCCGUAAGGAAAGGCUCCUAGAAGACUCCAAGGGAGUAAAAGA
GCUGAUGAAACAAUGGCAA-3’ (SEQ ID NO: 7) for skipping or at least skipping of exon 55.

Another preferred oligonucleotide comprises:
i)
   la) at least one 2'-substituted monomer, preferably an RNA monomer or a 2'-O-substituted RNA monomer, and optionally a phosphorothioate backbone linkage, or
   Ib) only 2'-substituted monomers, preferably RNA monomers or 2'-O-substituted RNA monomers, linked by phosphorothioate backbone linkages and/or by phosphodiester linkages,
ii) a 5-methylcytosine and/or a 5-methyluracil base and
iii) at least one monomer comprising a bicyclic nucleic acid (BNA) scaffold modification,
and comprises a continuous stretch of at least 10 and up to 33 nucleotides of at least one of the following nucleotide sequences selected from SEQ ID NO: 6065 to 6070, more preferably from:

5'-
CUUAAGAUACCAUUUGUAUUUAGCAUGUUCCCAAUUCUCAGGAAUUUGUGUCUUUCU
GAGAAACUGUUCAGCUUCUGUUAGCCACUGAUUAAAUAUCUUUAUAUCAUAAUGAAAA
CGCCGCCAUUUCUCAACAGAUCUGUCAAAUCGC-3' (SEQ ID NO: 6065) for skipping at
least exon 44,

5'-
CUCUUUUUCUGUCUGACAGCUGUUUGCAGACCUCCUGCCACCGCAGAUUCAGGCUU
CCCAAUUUUUCCUGUAGAAUACUGGCAUCUGUUUUUGAGGAUUGCUGAAUUAUUUCU
UCCCCAGUUGCAUUCAAUGUUCUGACAACAGUUUGCCGCUGCCCAAUGCCAUCCUGG
AGUUC-3' (SEQ ID NO: 6066) for skipping at least exon 45,

5’-
CUUCUGCUUGAUGAUCAUCUCGUUGAUAUCCUCAAGGUCACCCACCAUCACCCUCUG
UGAUUUUAUAACUUGAUCAAGCAGAGAAAGCCAGUCGGUAAGUUCUGUCCAAGCCCG
GUUGAAAUCUGCCAGAGCAGGUACCUCCAACAUCAAGGAAGAUGGCAUUUCUAGUUU
GGAGAUGGCAGUUUCCUUAGUAACCACAGGUUGUGUCACCAGAGUAACAGUCUGAGU
AGGAG-3’ (SEQ ID NO: 6067) for skipping at least exon 51,

5'-
UUCGAUCCGUAAUGAUUGUUCUAGCCUCUUGAUUGCUGGUCUUGUUUUUCAAAUUUU
GGGCAGCGGUAAUGAGUUCUUCCAACUGGGGACGCCUCUGUUCCAAAUCCUGCAUUG
UUGC-3' (SEQ ID NO: 6068) for skipping at least exon 52,

5'-
CUUGGUUUCUGUGAUUUUCUUUUGGAUUGCAUCUACUGUAUAGGGACCCUCCUUCCA
UGACUCAAGCUUGGCUCUGGCCUGUCCUAAGACCUGCUCAGCUUCUUCCUUAGCUUC
CAGCCAUUGUGUUGAAUCCUUUAACAUUUCAUUCAACUGUUGCCUCCGGUUCUGAAG
GUGUUCUUGUACUUCAUCCCACUGAUUCUGAAUUCUUUCAA-3' (SEQ ID NO: 6069) for
skipping at least exon 53,

5'-
UUGCCAUUGUUUCAUCAGCUCUUUUACUCCCUUGGAGUCUUCUAGGAGCCUUUCCUU
ACGGGUAGCAUCCUGUAGGACAUUGGCAGUUGUUUCAGCUUCUGUAAGCCAGGCAAG
AAACUUUUCCAGGUCCAGGGGGAACUGUUGCAGUAAUCUAUGAGUUUCUUCCAAAGC
AGCCUCUCGCUCACUCACC-3' (SEQ ID NO: 6070) for skipping at least exon 55.

In preferred embodiments, the oligonucleotide according to the invention comprises or consists of a nucleotide sequence represented by SEQ ID NOs: 8-271 or SEQ ID NOs: 1608-2099 or SEQ ID NOs: 3000-3184. These oligonucleotides are preferably for skipping dystrophin pre-mRNA exon 44.

In preferred embodiments, the oligonucleotide according to the invention comprises or consists of a nucleotide sequence represented by SEQ ID NOs: 272-451 or SEQ ID NOs: 3185-4527. These oligonucleotides are preferably for skipping dystrophin pre-mRNA exon 45.

In preferred embodiments, the oligonucleotide according to the invention comprises or consists of a nucleotide sequence represented by SEQ ID NOs: 452-613 or SEQ ID NOs: 4528-4572. In more preferred embodiments, the oligonucleotide according to the invention comprises or consists of a nucleotide sequence represented by SEQ ID NOs: 455, 459, 4528, 4531, 4532, 4533, 4535, 4542, 4548, or 4568. These oligonucleotides are preferably for skipping dystrophin pre-mRNA exon 51.

In preferred embodiments, the oligonucleotide according to the invention comprises or consists of a nucleotide sequence represented by SEQ ID NOs: 842-1159 or SEQ ID NOs: 4573-6048. These oligonucleotides are preferably for skipping dystrophin pre-mRNA exon 53.

In preferred embodiments, the oligonucleotide according to the invention comprises or consists of a nucleotide sequence represented by SEQ ID NO: 614-841. These oligonucleotides are preferably for skipping dystrophin pre-mRNA exon 52.

In preferred embodiments, the oligonucleotide according to the invention comprises or consists of a nucleotide sequence represented by SEQ ID NO: 1160-1399. These oligonucleotides are preferably for skipping dystrophin pre-mRNA exon 55.

SEQ ID NOs: 6065-6070 represent reverse complementary sequences to SEQ ID NOs: 2-7. In a more preferred embodiment, the oligonucleotide according to the invention has a length from 10 to 33 nucleotides and comprises:
i) only 2'-substituted monomers, preferably RNA monomers or 2'-O-substituted RNA monomers, linked by phosphorothioate backbone linkages and/or by phosphodiester linkages,
ii) a 5-methylcytosine base, and
iii) at least one monomer comprising a bicyclic nucleic acid (BNA) scaffold modification,
and comprises a continuous stretch of at least 10 and up to 33 nucleotides of at least one of the nucleotide sequences selected from SEQ ID NO: 6065 to 6070, preferably SEQ ID NO: 6067.

In an even more preferred embodiment, the oligonucleotide according to the invention has a length from 10 to 33 nucleotides and comprises:
i) only 2'-substituted monomers, preferably 2'-O-substituted RNA monomers, linked by phosphorothioate backbone linkages,
ii) a 5-methylcytosine base, and
iii) at least one monomer comprising a bicyclic nucleic acid (BNA) scaffold modification,
and comprises a continuous stretch of at least 10 and up to 33 nucleotides of at least one of the nucleotide sequences selected from SEQ ID NO: 6065 to 6070, preferably SEQ ID NO: 6067. In more preferred embodiments, such an oligonucleotide has at least two monomers comprising a BNA scaffold modification.

In these embodiments, it is preferred that said continuous stretch is of at least 16 to 26 nucleotides, or from 16 to 25 nucleotides in length. In another embodiment, said continuous stretch is from 16 to 24 nucleotides in length. In another embodiment, said continuous stretch is from 16 to 22 nucleotides in length. In another embodiment, said continuous stretch is 16, 18, 20, or 22 nucleotides in length. In another embodiment, said continuous stretch is 21, 22, 24, or 25 nucleotides in length. In another embodiment, said continuous stretch is 18, 22, 24, or 25 nucleotides in length. The oligonucleotide of the invention preferably consists of said continuous stretch.

More preferred oligonucleotides comprise at least one 2'-substituted monomer and optionally a phosphorothioate backbone linkage, or only 2'-substituted monomers linked by phosphorothioate backbone linkages and/or by phosphodiester linkages, comprise a 5-methylcytosine and/or a 5-methyluracil base and comprise at least one monomer comprising a bicyclic nucleic acid (BNA) scaffold modification, and are represented by a nucleotide sequence comprising or consisting of SEQ ID NO: 8-1580 or SEQ ID NO: 1592-1607, or by a nucleotide sequence comprising or consisting of a fragment of SEQ ID NO: 8 -1580 or SEQ ID NO: 1592-1607. More preferred oligonucleotides comprise at least one 2'-substituted monomer and optionally a phosphorothioate backbone linkage, or only 2'-substituted monomers linked by phosphorothioate backbone linkages and/or by phosphodiester linkages, comprise a 5-methylcytosine and/or a 5-methyluracil base and comprise at least one monomer comprising a bicyclic nucleic acid (BNA) scaffold modification, and are represented by a nucleotide sequence comprising or consisting of SEQ ID NO: 8-1580 or SEQ ID NO: 1592-2099 or SEQ ID NO: 3000-6048, or by a nucleotide sequence comprising or consisting of a fragment of SEQ ID NO: 8 - 1580 or SEQ ID NO: 1592-2099 or SEQ ID NO: 3000-6048. Such oligonucleotides preferably have a length of 16-30 nucleotides, more preferably of 16-24 nucleotides, most preferably of 19, 22, or 22 nucleotides. More preferred oligonucleotides are as described above, and are represented by a nucleotide sequence comprising or consisting of SEQ ID NO: 9-13, 15-19, 21-25, 27-31, 33-37, 39-43, 45-49,51-55, 57-61, 63-67, 69-73, 75-79, 81-85, 87-91, 93-97, 99-103, 105-109, 111-115, 117-121, 123-127, 129-133, 135-139, 141-145, 147-151, 153-157, 159-163, 165-169, 171-175, 177-181, 183-187, 189-193, 195-199, 201-205, 207-211, 213-217, 219-223, 225-229, 231-235, 237-241, 243-247, 249-253, 255-259, 261-265, 267-271, 273-277, 279-283, 285-289, 291-295, 297-301, 303-307, 309-313, 315-319, 321-325, 327-331, 333-337, 339-343, 345-349, 351-355, 357-361, 363-367, 369-373, 375-379, 381-385, 387-391, 393-397, 399-403, 405-409, 411-415, 417-421, 423-427, 429-433, 435-439, 441-445, 447-451, 453-457, 459-463, 465-469, 471-475, 477-481, 483-487, 489-493, 495-499, 501-505, 507-511, 513-517, 519-523, 525-529, 531-535, 537-541, 543-547, 549-553, 555-559, 561-565, 567-571, 573-577, 579-583, 585-589, 591-595, 597-601, 603-607, 609-613, 615-619, 621-625, 627-631, 633-637, 639-643, 645-649, 651-655, 657-661, 663-667, 669-673, 675-679, 681-685, 687-691, 693-697, 699-703, 705-709, 711-715, 717-721, 723-727, 729-733, 735-739, 741-745, 747-751, 753-757, 759-763, 765-769, 771-775, 777-781, 783-787, 789-793, 795-799, 801-805, 807-811, 813-817, 819-823, 825-829, 831-835, 837-841, 843-847, 849-853, 855-859, 861-865, 867-871, 873-877, 879-883, 885-889, 891-895, 897-901, 903-907, 909-913, 915-919, 921-925, 927-931, 933-937, 939-943, 945-949, 951-955, 957-961, 963-967, 969-973, 975-979, 981-985, 987-991, 993-997, 999-1003, 1005-1009, 1011-1015, 1017-1021, 1023-1027, 1029-1033, 1035-1039, 1041-1045, 1047-1051, 1053-1057, 1059-1063, 1065-1069, 1071-1075, 1077-1081, 1083-1087, 1089-1093, 1095-1099, 1101-1105, 1107-1111, 1113-1117, 1119-1123, 1125-1129, 1131-1135, 1137-1141, 1143-1147, 1149-1153, 1155-1159, 1161-1165, 1167-1171, 1173-1177, 1179-1183, 1185-1189, 1191-1195, 1197-1201, 1203-1207, 1209-1213, 1215-1219, 1221-1225, 1227-1231, 1233-1237, 1239-1243, 1245-1249, 1251-1255, 1257-1261, 1263-1267, 1269-1273, 1275-1279, 1281-1285, 1287-1291, 1293-1297, 1299-1303, 1305-1309, 1311-1315, 1317-1321, 1323-1327, 1329-1333, 1335-1339, 1341-1345, 1347-1351, 1353-1357, 1359-1363, 1365-1369, 1371-1375, 1377-1381, 1383-1387, 1389-1393, 1395-1399, 1401-1405, 1407-1411, 1413-1417, 1419-1423, 1425-1429, 1431-1435, 1437-1441, 1443-1447, 1449-1453, 1455-1459, 1461-1465, 1467-1471, 1473-1477, 1479-1483, 1485-1489, 1491-1495, 1497-1501, 1503-1507, 1509-1513, 1515-1519, 1521-1525, 1527-1531, 1533-1537, 1539-1543, 1545-1549, 1551-1555, 1557-1561, 1563-1567, 1569-1573, 1575-1579, 1592-2099, or 3000-6048 or by a nucleotide sequence comprising or consisting of a fragment of SEQ ID NO: 9-13, 15-19, 21-25, 27-31, 33-37, 39-43, 45-49,51-55, 57-61, 63-67, 69-73, 75-79, 81-85, 87-91, 93-97, 99-103, 105-109, 111-115, 117-121, 123-127, 129-133, 135-139, 141-145, 147-151, 153-157, 159-163, 165-169, 171-175, 177-181, 183-187, 189-193, 195-199, 201-205, 207-211, 213-217, 219-223, 225-229, 231-235, 237-241, 243-247, 249-253, 255-259, 261-265, 267-271, 273-277, 279-283, 285-289, 291-295, 297-301, 303-307, 309-313, 315-319, 321-325, 327-331, 333-337, 339-343, 345-349, 351-355, 357-361, 363-367, 369-373, 375-379, 381-385, 387-391, 393-397, 399-403, 405-409, 411-415, 417-421, 423-427, 429-433, 435-439, 441-445, 447-451, 453-457, 459-463, 465-469, 471-475, 477-481, 483-487, 489-493, 495-499, 501-505, 507-511, 513-517, 519-523, 525-529, 531-535, 537-541, 543-547, 549-553, 555-559, 561-565, 567-571, 573-577, 579-583, 585-589, 591-595, 597-601, 603-607, 609-613, 615-619, 621-625, 627-631, 633-637, 639-643, 645-649, 651-655, 657-661, 663-667, 669-673, 675-679, 681-685, 687-691, 693-697, 699-703, 705-709, 711-715, 717-721, 723-727, 729-733, 735-739, 741-745, 747-751, 753-757, 759-763, 765-769, 771-775, 777-781, 783-787, 789-793, 795-799, 801-805, 807-811, 813-817, 819-823, 825-829, 831-835, 837-841, 843-847, 849-853, 855-859, 861-865, 867-871, 873-877, 879-883, 885-889, 891-895, 897-901, 903-907, 909-913, 915-919, 921-925, 927-931, 933-937, 939-943, 945-949, 951-955, 957-961, 963-967, 969-973, 975-979, 981-985, 987-991, 993-997, 999-1003, 1005-1009, 1011-1015, 1017-1021, 1023-1027, 1029-1033, 1035-1039, 1041-1045, 1047-1051, 1053-1057, 1059-1063, 1065-1069, 1071-1075, 1077-1081, 1083-1087, 1089-1093, 1095-1099, 1101-1105, 1107-1111, 1113-1117, 1119-1123, 1125-1129, 1131-1135, 1137-1141, 1143-1147, 1149-1153, 1155-1159, 1161-1165, 1167-1171, 1173-1177, 1179-1183, 1185-1189, 1191-1195, 1197-1201, 1203-1207, 1209-1213, 1215-1219, 1221-1225, 1227-1231, 1233-1237, 1239-1243, 1245-1249, 1251-1255, 1257-1261, 1263-1267, 1269-1273, 1275-1279, 1281-1285, 1287-1291, 1293-1297, 1299-1303, 1305-1309, 1311-1315, 1317-1321, 1323-1327, 1329-1333, 1335-1339, 1341-1345, 1347-1351, 1353-1357, 1359-1363, 1365-1369, 1371-1375, 1377-1381, 1383-1387, 1389-1393, 1395-1399, 1401-1405, 1407-1411, 1413-1417, 1419-1423, 1425-1429, 1431-1435, 1437-1441, 1443-1447, 1449-1453, 1455-1459, 1461-1465, 1467-1471, 1473-1477, 1479-1483, 1485-1489, 1491-1495, 1497-1501, 1503-1507, 1509-1513, 1515-1519, 1521-1525, 1527-1531, 1533-1537, 1539-1543, 1545-1549, 1551-1555, 1557-1561, 1563-1567, 1569-1573, 1575-1579, 1592-2099, or 3000-6048. Within the context of the invention, a fragment of SEQ ID NO: 8-1580 or SEQ ID NO: 1592-2099 or SEQ ID NO: 3000-6048 preferably means a nucleotide sequence comprising or consisting of at least 10 contiguous nucleotides from said SEQ ID NO.

More preferred oligonucleotides comprise at least one 2'-substituted monomer, preferably an RNA monomer, and optionally a phosphorothioate backbone linkage, or only 2'-substituted monomers linked by phosphorothioate backbone linkages and/or by phosphodiester linkages, comprise a 5-methylcytosine and/or a 5-methyluracil base and comprise at least one monomer comprising a bicyclic nucleic acid (BNA) scaffold modification, and are represented by a nucleotide sequence comprising or consisting of SEQ ID NO: 8-1580 or SEQ ID NO: 1592-2099 or SEQ ID NO: 3000-6048, or by a nucleotide sequence comprising or consisting of fragment of SEQ ID NO: 8-1580 or SEQ ID NO: 1592-2099 or SEQ ID NO: 3000-6048, and have a length of 10, 11, 12, 13, 14, 15, 16, 17, 18, 19, 20, 21, 22, 23, 24, 25, 26, 27, 28, 29, 30, 31, 32 or 33 nucleotides.

Preferred sequences include SEQ ID NO: 452-613 and SEQ ID NO: 1592-1605 and SEQ ID NO: 1607, more preferably SEQ ID NO: 453-457, 459-463, 465-469, 471-475, 477-481, 483-487, 489-493, 495-499, 501-505, 507-511, 513-517, 519-523, 525-529, 531-535, 537-541, 543-547, 549-553, 555-559, 561-565, 567-571, 573-577, 579-583, 585-589, 591-595, 597-601, 603-607, 609-613, 1592-1605, and 1607, and even more preferably SEQ ID NO: 453, 455, 456, 459, 461, 462, 465, 467, 468, 471, 473, 474, 483, 486, 1592, 1593, 1594, 1595, 1596, 1597, 1598, 1599, 1600, 1601, 1602, 1603, 1604, 1605, or 1607. The most preferred sequences are SEQ ID NO: 455, 459, 4528, 4531, 4532, 4533, 4535, 4542, 4548, and 4568.

In a preferred embodiment, an oligonucleotide is represented by a nucleotide sequence comprising or consisting of SEQ ID NO: 8-271 or SEQ ID NO:1608-2099 or SEQ ID NO: 3000-3184 and is for skipping exon 44 of the pre-mRNA of dystrophin, and comprises one or more of the following:
- at least one 2'-substituted monomer
- at least one phosphorothioate backbone linkage;
- only 2'-substituted monomers;
- only phosphorothioate backbone linkages;
- only 2'-substituted monomers linked by phosphorothioate backbone linkages;
- a 5-methylcytosine and/or a 5-methyluracil base;
- only 5-methylcytosine bases instead of cytosine bases;
- at least one monomer comprising a bicyclic nucleic acid (BNA) scaffold modification.

Preferably, said oligonucleotide comprises only 2'-substituted monomers, only phosphorothioate backbone linkages, and at least one monomer comprising a BNA scaffold modification More preferably, said oligonucleotide is represented by a nucleotide sequence comprising or consisting of SEQ ID NO: 9-13, 15-19, 21-25, 27-31, 33-37, 39-43, 45-49,51-55, 57-61, 63-67, 69-73, 75-79, 81-85, 87-91, 93-97, 99-103, 105-109, 111-115, 117-121, 123-127, 129-133, 135-139, 141-145, 147-151, 153-157, 159-163, 165-169, 171-175, 177-181, 183-187, 189-193, 195-199, 201-205, 207-211, 213-217, 219-223, 225-229, 231-235, 237-241, 243-247, 249-253, 255-259, 261-265, or 267-271. Preferably, said oligonucleotide has a length of 10 to 33 nucleotides, most preferably of 16 to 22 nucleotides.

In a preferred embodiment, an oligonucleotide is represented by a nucleotide sequence comprising or consisting of SEQ ID NO: 272-451 or SEQ ID NO:3185-4527 and is for skipping exon 45 of the pre-mRNA of dystrophin, and comprises one or more of the following:
- at least one 2'-substituted monomer
- at least one phosphorothioate backbone linkage;
- only 2'-substituted monomers;
- only phosphorothioate backbone linkages;
- only 2'-substituted monomers linked by phosphorothioate backbone linkages;
- a 5-methylcytosine and/or a 5-methyluracil base;
- only 5-methylcytosine bases instead of cytosine bases;
- at least one monomer comprising a bicyclic nucleic acid (BNA) scaffold modification.

Preferably, said oligonucleotide comprises only 2'-substituted monomers, only phosphorothioate backbone linkages, and at least one monomer comprising a BNA scaffold modification. More preferably, said oligonucleotide is represented by a nucleotide sequence comprising or consisting of SEQ ID NO: 273-277, 279-283, 285-289, 291-295, 297-301, 303-307, 309-313, 315-319, 321-325, 327-331, 333-337, 339-343, 345-349, 351-355, 357-361, 363-367, 369-373, 375-379, 381-385, 387-391, 393-397, 399-403, 405-409, 411-415, 417-421, 423-427, 429-433, 435-439, 441-445, or 447-451. Preferably, said oligonucleotide has a length of 10 to 33 nucleotides, most preferably of 16 to 22 nucleotides.

In a preferred embodiment, an oligonucleotide is represented by a nucleotide sequence comprising or consisting of SEQ ID NO: 452-613 or SEQ ID NO 1592-1605 or SEQ ID NO: 4528-4572 and is for skipping exon 51 of the pre-mRNA of dystrophin, and comprises one or more of the following:
- at least one 2'-substituted monomer
- at least one phosphorothioate backbone linkage;
- only 2'-substituted monomers;
- only phosphorothioate backbone linkages;
- only 2'-substituted monomers linked by phosphorothioate backbone linkages;
- a 5-methylcytosine and/or a 5-methyluracil base;
- only 5-methylcytosine bases instead of cytosine bases;
- at least one monomer comprising a bicyclic nucleic acid (BNA) scaffold modification.

Preferably, said oligonucleotide comprises only 2'-substituted monomers, only phosphorothioate backbone linkages, and at least one monomer comprising a BNA scaffold modification. More preferably, said oligonucleotide is represented by a nucleotide sequence comprising or consisting of SEQ ID NO: 453-457, 459-463, 465-469, 471-475, 477-481, 483-487, 489-493, 495-499, 501-505, 507-511, 513-517, 519-523, 525-529, 531-535, 537-541, 543-547, 549-553, 555-559, 561-565, 567-571, 573-577, 579-583, 585-589, 591-595, 597-601, 603-607, 609-613, 1592-1605, or 1607, even more preferably SEQ ID NO: 453, 455, 456, 459, 461, 462, 465, 467, 468, 471, 473, 474, 483, 486, 1592, 1593, 1594, 1595, 1596, 1597, 1598, 1599, 1600, 1601, 1602, 1603, 1604, 1605, or 1607 most preferably SEQ ID NO: 1592, 1593, 1594, 1595, 1596, 1597, 1598, 1599, 1600, 1601, 1602, 1603, 1604, 1605, or 1607, even more preferably SEQ ID NO: 455, 459, 4528, 4531, 4532, 4533, 4535, 4542, 4548, and 4568. Preferably, said oligonucleotide has a length of 10 to 33 nucleotides, most preferably of 16 to 22 nucleotides.

Accordingly, in a preferred embodiment, an oligonucleotide according to the invention is represented by a nucleotide sequence comprising or consisting of SEQ ID NO: 1592 (TCAAGGAAGAUGGCAUUUCU) and is for skipping exon 51 of the pre-mRNA of dystrophin, comprises a BNA scaffold modification in the 5'-terminal monomer and in no other monomers, comprises 5-methylcytosine instead of cytosine, comprises only phosphorothioate linkages, and further comprises only 2'-O-methyl RNA monomers.

Accordingly, in a preferred embodiment, an oligonucleotide according to the invention is represented by a nucleotide sequence comprising or consisting of SEQ ID NO: 1593 (TCAAGGAAGAUGGCAUUUCT) and is for skipping exon 51 of the pre-mRNA of dystrophin, comprises a BNA scaffold modification in the 5'-terminal monomer and in the 3'-terminal monomer and in no other monomers, comprises 5-methylcytosine instead of cytosine, comprises only phosphorothioate linkages, and further comprises only 2'-O-methyl RNA monomers.

Accordingly, in a preferred embodiment, an oligonucleotide according to the invention is represented by a nucleotide sequence comprising or consisting of SEQ ID NO: 1594 (TCAAGGAAGAUGGCAUUUCU) and is for skipping exon 51 of the pre-mRNA of dystrophin, comprises a BNA scaffold modification in the 5'-terminal monomer and in its neighbouring monomer and in no other monomers, comprises 5-methylcytosine instead of cytosine, comprises only phosphorothioate linkages, and further comprises only 2'-O-methyl RNA monomers.

Accordingly, in a preferred embodiment, an oligonucleotide according to the invention is represented by a nucleotide sequence comprising or consisting of SEQ ID NO: 1595 (TCAAGGAAGAUGGCAUUUCUAG) and is for skipping exon 51 of the pre-mRNA of dystrophin, comprises a BNA scaffold modification in the 5'-terminal monomer and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 1596 (TCAAGGAAGAUGGCAUUUCUAG) and is for skipping exon 51 of the pre-mRNA of dystrophin, comprises a BNA scaffold modification in the 5'-terminal monomer and in the 3'-terminal monomer and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 1597 (TCAAGGAAGAUGGCAUUUCUAG) and is for skipping exon 51 of the pre-mRNA of dystrophin, comprises a BNA scaffold modification in the 5'-terminal monomer and in its neighbouring monomer and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 1598 (AAGGAAGAUGGCAUUUCU) and is for skipping exon 51 of the pre-mRNA of dystrophin, comprises a BNA scaffold modification in the 5'-terminal monomer and in no other monomers, comprises 5-methylcytosine instead of cytosine, comprises only phosphorothioate linkages, and further comprises only 2'-O-methyl RNA monomers.

Accordingly, in a preferred embodiment, an oligonucleotide according to the invention is represented by a nucleotide sequence comprising or consisting of SEQ ID NO: 1599 (AAGGAAGAUGGCAUUUCT) and is for skipping exon 51 of the pre-mRNA of dystrophin, comprises a BNA scaffold modification in the 5'-terminal monomer and in the 3'-terminal monomer and in no other monomers, comprises 5-methylcytosine instead of cytosine, comprises only phosphorothioate linkages, and further comprises only 2'-O-methyl RNA monomers.

Accordingly, in a preferred embodiment, an oligonucleotide according to the invention is represented by a nucleotide sequence comprising or consisting of SEQ ID NO: 1600 (AAGGAAGAUGGCAUUUCU) and is for skipping exon 51 of the pre-mRNA of dystrophin, comprises a BNA scaffold modification in the 5'-terminal monomer and in its neighbouring monomer and in no other monomers, comprises 5-methylcytosine instead of cytosine, comprises only phosphorothioate linkages, and further comprises only 2'-O-methyl RNA monomers.

Accordingly, in a preferred embodiment, an oligonucleotide according to the invention is represented by a nucleotide sequence comprising or consisting of SEQ ID NO: 1601 (GGAAGAUGGCAUUUCU) and is for skipping exon 51 of the pre-mRNA of dystrophin, comprises a BNA scaffold modification in the 5'-terminal monomer and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 1602 (GGAAGAUGGCAUUUCT) and is for skipping exon 51 of the pre-mRNA of dystrophin, comprises a BNA scaffold modification in the 5'-terminal monomer and in the 3'-terminal monomer and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 1603 (GGAAGAUGGCAUUUCU) and is for skipping exon 51 of the pre-mRNA of dystrophin, comprises a BNA scaffold modification in the 5'-terminal monomer and in its neighbouring monomer and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 1604 (TCAAGGAAGAUGGCAU) and is for skipping exon 51 of the pre-mRNA of dystrophin, comprises a BNA scaffold modification in the 5'-terminal monomer and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 1605 (TCAAGGAAGAUGGCAU) and is for skipping exon 51 of the pre-mRNA of dystrophin, comprises a BNA scaffold modification in the 5'-terminal monomer and in its neighbouring monomer and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 1607 (CUCCAACAUCAAGGAAGAUGGCAUUUCUAG) and is for skipping exon 51 of the pre-mRNA of dystrophin, comprises no BNA scaffold modification in any monomers, comprises cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 1607 (CUCCAACAUCAAGGAAGAUGGCAUUUCUAG) and is for skipping exon 51 of the pre-mRNA of dystrophin, comprises at least one BNA scaffold modification in any monomer, preferably only in either the 5'terminal monomer, the 3'-terminal monomer, both the 5'-terminal and in the 3'-terminal monomer, the two most 5'-terminal monomers, or the two most 3'-terminal monomers, comprises cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 455 (TCAAGGAAGAUGGCAUUUCT) and is for skipping exon 51 of the pre-mRNA of dystrophin, comprises a BNA scaffold modification in the 5'-terminal monomer and in the 3'-terminal monomer and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 459 (TCAAGGAAGAUGGCAUUUCUAG) and is for skipping exon 51 of the pre-mRNA of dystrophin, comprises a BNA scaffold modification in the 5'-terminal monomer and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 4528 (TCAAGGAAGAUGGCAUUUCUAG) and is for skipping exon 51 of the pre-mRNA of dystrophin, comprises a BNA scaffold modification in the 5'-terminal monomer, its neighbouring monomer, and its 3'-terminal monomer, and in no other monomers, comprises 5-methylcytosine instead of cytosine, comprises only phosphorothioate linkages, and further comprises only 2'-O-methyl RNA monomers.

Accordingly, in a preferred embodiment, an oligonucleotide according to the invention is represented by a nucleotide sequence comprising or consisting of SEQ ID NO: 4531 (TCAAGGAAGAUGGCAUUUCUAG) and is for skipping exon 51 of the pre-mRNA of dystrophin, comprises a BNA scaffold modification in the 5'-terminal monomer, its neighbouring monomer, in the 13^{th} monomer from the 5'-terminus, and in its 3'-terminal monomer, and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 4532 (TCAAGGAAGAUGGCAUUUCUAG) and is for skipping exon 51 of the pre-mRNA of dystrophin, comprises a BNA scaffold modification in the 5'-terminal monomer, its neighbouring monomer, in the 9^{th} monomer from the 5'-terminus, and in its 3'-terminal monomer, and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 4533 (TCAAGGAAGAUGGCAUUUCUAG) and is for skipping exon 51 of the pre-mRNA of dystrophin, comprises a BNA scaffold modification in the 5'-terminal monomer, its neighbouring monomer, in the 9^{th} monomer from the 5'-terminus, in the 13^{th} monomer from the 5'-terminus, and in its 3'-terminal monomer, and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 4535 (TCAAGGAAGAUGGCAUUUCT) and is for skipping exon 51 of the pre-mRNA of dystrophin, comprises a BNA scaffold modification in the 5'-terminal monomer, in its neighbouring monomer, and in its 3'-terminal monomer, and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 4542 (TCAAGGAAGAUGGCAUUUCT) and is for skipping exon 51 of the pre-mRNA of dystrophin, comprises a BNA scaffold modification in the 5'-terminal monomer, in its 13^{th} monomer from the 5'-terminus, and in its 3'-terminal monomer, and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 4548 (CAAGGAAGAUGGCAUUUCT) and is for skipping exon 51 of the pre-mRNA of dystrophin, comprises a BNA scaffold modification in the 5'-terminal monomer, in its neighbouring monomer, in its 8^{th} monomer from the 5'-terminus, in its 12^{th} monomer from the 5'-terminus, and in its 3'-terminal monomer, and in no other monomers, comprises 5-methylcytosine instead of cytosine, comprises only phosphorothioate linkages, and further comprises only 2'-*O*-methyl RNA monomers.

Accordingly, in a preferred embodiment, an oligonucleotide according to the invention is represented by a nucleotide sequence comprising or consisting of SEQ ID NO: 4568 (GGUAAGUUCUGUCCAAGC) and is for skipping exon 51 of the pre-mRNA of dystrophin, comprises a BNA scaffold modification in the 5'-terminal monomer, in its neighbouring monomer, in its 6^{th} monomer from the 5'-terminus, and in its 3'-terminal monomer, and in no other monomers, comprises 5-methylcytosine instead of cytosine, comprises only phosphorothioate linkages, and further comprises only 2'-*O*-methyl RNA monomers.

In a preferred embodiment, an oligonucleotide is represented by a nucleotide sequence comprising or consisting of SEQ ID NO: 614-841 and is for skipping exon 52 of the pre-mRNA of dystrophin, and comprises one or more of the following:
- at least one 2'-substituted monomer
- at least one phosphorothioate backbone linkage;
- only 2'-substituted monomers;
- only phosphorothioate backbone linkages;
- only 2'-substituted monomers linked by phosphorothioate backbone linkages;
- a 5-methylcytosine and/or a 5-methyluracil base;
- only 5-methylcytosine bases instead of cytosine bases;
- at least one monomer comprising a bicyclic nucleic acid (BNA) scaffold modification.

Preferably, said oligonucleotide comprises only 2'-substituted monomers, only phosphorothioate backbone linkages, and at least one monomer comprising a BNA scaffold modification. More preferably, said oligonucleotide is represented by a nucleotide sequence comprising or consisting of SEQ ID NO: 615-619, 621-625, 627-631, 633-637, 639-643, 645-649, 651-655, 657-661, 663-667, 669-673, 675-679, 681-685, 687-691, 693-697, 699-703, 705-709, 711-715, 717-721, 723-727, 729-733, 735-739, 741-745, 747-751, 753-757, 759-763, 765-769, 771-775, 777-781, 783-787, 789-793, 795-799, 801-805, 807-811, 813-817, 819-823, 825-829, 831-835, or 837-841. Preferably, said oligonucleotide has a length of 10 to 33 nucleotides, most preferably of 16 to 22 nucleotides.

In a preferred embodiment, an oligonucleotide is represented by a nucleotide sequence comprising or consisting of SEQ ID NO: 842-1159 or SEQ ID NO:4573-6048 and is for skipping exon 53 of the pre-mRNA of dystrophin, and comprises one or more of the following:
- at least one 2'-substituted monomer
- at least one phosphorothioate backbone linkage;
- only 2'-substituted monomers;
- only phosphorothioate backbone linkages;
- only 2'-substituted monomers linked by phosphorothioate backbone linkages;
- a 5-methylcytosine and/or a 5-methyluracil base;
- only 5-methylcytosine bases instead of cytosine bases;
- at least one monomer comprising a bicyclic nucleic acid (BNA) scaffold modification.

Preferably, said oligonucleotide comprises only 2'-substituted monomers, only phosphorothioate backbone linkages, and at least one monomer comprising a BNA scaffold modification. More preferably, said oligonucleotide is represented by a nucleotide sequence comprising or consisting of SEQ ID NO: 843-847, 849-853, 855-859, 861-865, 867-871, 873-877, 879-883, 885-889, 891-895, 897-901, 903-907, 909-913, 915-919, 921-925, 927-931, 933-937, 939-943, 945-949, 951-955, 957-961, 963-967, 969-973, 975-979, 981-985, 987-991, 993-997, 999-1003, 1005-1009, 1011-1015, 1017-1021, 1023-1027, 1029-1033, 1035-1039, 1041-1045, 1047-1051, 1053-1057, 1059-1063, 1065-1069, 1071-1075, 1077-1081, 1083-1087, 1089-1093, 1095-1099, 1101-1105, 1107-1111, 1113-1117, 1119-1123, 1125-1129, 1131-1135, 1137-1141, 1143-1147, 1149-1153, or 1155-1159. Preferably, said oligonucleotide has a length of 10 to 33 nucleotides, most preferably of 16 to 22 nucleotides.

In a preferred embodiment, an oligonucleotide is represented by a nucleotide sequence comprising or consisting of SEQ ID NO: 1160-1399 and is for skipping exon 55 of the pre-mRNA of dystrophin, and comprises one or more of the following:
- at least one 2'-substituted monomer
- at least one phosphorothioate backbone linkage;
- only 2'-substituted monomers;
- only phosphorothioate backbone linkages;
- only 2'-substituted monomers linked by phosphorothioate backbone linkages;
- a 5-methylcytosine and/or a 5-methyluracil base;
- only 5-methylcytosine bases instead of cytosine bases;
- at least one monomer comprising a bicyclic nucleic acid (BNA) scaffold modification.

Preferably, said oligonucleotide comprises only 2'-substituted monomers, only phosphorothioate backbone linkages, and at least one monomer comprising a BNA scaffold modification. More preferably, said oligonucleotide is represented by a nucleotide sequence comprising or consisting of SEQ ID NO: 1161-1165, 1167-1171, 1173-1177, 1179-1183, 1185-1189, 1191-1195, 1197-1201, 1203-1207, 1209-1213, 1215-1219, 1221-1225, 1227-1231, 1233-1237, 1239-1243, 1245-1249, 1251-1255, 1257-1261, 1263-1267, 1269-1273, 1275-1279, 1281-1285, 1287-1291, 1293-1297, 1299-1303, 1305-1309, 1311-1315, 1317-1321, 1323-1327, 1329-1333, 1335-1339, 1341-1345, 1347-1351, 1353-1357, 1359-1363, 1365-1369, 1371-1375, 1377-1381, 1383-1387, 1389-1393, or 1395-1399. Preferably, said oligonucleotide has a length of 10 to 33 nucleotides, most preferably of 16 to 22 nucleotides.

In a preferred embodiment, an oligonucleotide is represented by a nucleotide sequence comprising or consisting of SEQ ID NO: 1400-1579 and is for including exon 7 of the pre-mRNA of SMN2, and comprises one or more of the following:
- at least one 2'-substituted monomer
- at least one phosphorothioate backbone linkage;
- only 2'-substituted monomers;
- only phosphorothioate backbone linkages;
- only 2'-substituted monomers linked by phosphorothioate backbone linkages;
- a 5-methylcytosine and/or a 5-methyluracil base;
- only 5-methylcytosine bases instead of cytosine bases;
- at least one monomer comprising a bicyclic nucleic acid (BNA) scaffold modification.

Preferably, said oligonucleotide comprises only 2'-substituted monomers, only phosphorothioate backbone linkages, and at least one monomer comprising a BNA scaffold modification. Most preferably, said oligonucleotide is represented by a nucleotide sequence comprising or consisting of SEQ ID NO: 1606, wherein it comprises no BNA scaffold modification, or comprising or consisting of SEQ ID NO: 1490, wherein it comprises BNA scaffold modifications in any monomer, preferably only in either the 5'terminal monomer, the 3'-terminal monomer, both the 5'-terminal and in the 3'-terminal monomer, the two most 5'-terminal monomers, or the two most 3'-terminal monomers. Preferably, said oligonucleotide has a length of 10 to 33 nucleotides, most preferably of 16 to 22 nucleotides.

In a preferred embodiment, an oligonucleotide is represented by a nucleotide sequence comprising or consisting of SEQ ID NO: 1400-1441 and is for targeting intron 6 of the pre-mRNA of SMN2, and comprises one or more of the following:
- at least one 2'-substituted monomer
- at least one phosphorothioate backbone linkage;
- only 2'-substituted monomers;
- only phosphorothioate backbone linkages;
- only 2'-substituted monomers linked by phosphorothioate backbone linkages;
- a 5-methylcytosine and/or a 5-methyluracil base;
- only 5-methylcytosine bases instead of cytosine bases;
- at least one monomer comprising a bicyclic nucleic acid (BNA) scaffold modification.

Preferably, said oligonucleotide comprises only 2'-substituted monomers, only phosphorothioate backbone linkages, and at least one monomer comprising a BNA scaffold modification. More preferably, said oligonucleotide is represented by a nucleotide sequence comprising or consisting of SEQ ID NO: 1401-1405, 1407-1411, 1413-1417, 1419-1423, 1425-1429, 1431-1435, 1437-1441. Preferably, said oligonucleotide has a length of 10 to 33 nucleotides, most preferably of 16 to 22 nucleotides.

In a preferred embodiment, an oligonucleotide is represented by a nucleotide sequence comprising or consisting of SEQ ID NO: 1442-1579 and is for targeting intron 7 of the pre-mRNA of SMN2, and comprises one or more of the following:
- at least one 2'-substituted monomer
- at least one phosphorothioate backbone linkage;
- only 2'-substituted monomers;
- only phosphorothioate backbone linkages;
- only 2'-substituted monomers linked by phosphorothioate backbone linkages;
- a 5-methylcytosine and/or a 5-methyluracil base;
- only 5-methylcytosine bases instead of cytosine bases;
- at least one monomer comprising a bicyclic nucleic acid (BNA) scaffold modification.

Preferably, said oligonucleotide comprises only 2'-substituted monomers, only phosphorothioate backbone linkages, and at least one monomer comprising a BNA scaffold modification. More preferably, said oligonucleotide is represented by a nucleotide sequence comprising or consisting of SEQ ID NO: 1443-1447, 1449-1453, 1455-1459, 1461-1465, 1467-1471, 1473-1477, 1479-1483, 1485-1489, 1491-1495, 1497-1501, 1503-1507, 1509-1513, 1515-1519, 1521-1525, 1527-1531, 1533-1537, 1539-1543, 1545-1549, 1551-1555, 1557-1561, 1563-1567, 1569-1573, 1575-1579. Most preferably, said oligonucleotide is represented by a nucleotide sequence comprising or consisting of SEQ ID NO: 1490, wherein it comprises BNA scaffold modifications in any monomer, preferably only in either the 5'terminal monomer, the 3'-terminal monomer, both the 5'-terminal and in the 3'-terminal monomer, the two most 5'-terminal monomers, or the two most 3'-terminal monomers. Preferably, said oligonucleotide has a length of 10 to 33 nucleotides, more preferably of 16 to 22 nucleotides, most preferably 18 monomers.

In a preferred embodiment, an oligonucleotide according to the invention is represented by a nucleotide sequence comprising or consisting of SEQ ID NO: 1606 (UCACUUUCAUAAUGCUGG) and is for targeting intron 7 of the pre-mRNA of SMN2, comprises no BNA scaffold modification in any monomers, comprises 5-methylcytosine instead of cytosine, comprises only phosphorothioate linkages, and further comprises only 2'-O-methyl RNA monomers.

In preferred embodiments the oligonucleotide according to the invention is one wherein said oligonucleotide has an improved parameter by comparison to a corresponding oligonucleotide that does not comprise a bicyclic nucleic acid (BNA) scaffold modification. We discovered that the presence of a BNA with 5-methylcytosine or 5-methyluracil in an oligonucleotide of the invention has a positive effect on at least one of the parameters of said oligonucleotides. In this context, parameters may include: binding affinity and/or kinetics, exon skipping activity, biostability, (intra-tissue) distribution, cellular uptake and/or trafficking, and/or immunogenicity of said oligonucleotide, as explained below.

Binding affinity and kinetics depend on the thermodynamic properties of the oligonucleotide. These are at least in part determined by the melting temperature of said oligonucleotide (Tm; calculated with e.g. the oligonucleotide properties calculator (accessible via the internet at for example www.unc.edu/~cail/biotool/oligo/index.html or at for example eu.idtdna.com/analyzer/Applications/OligoAnalyzer/) for single stranded RNA using the basic Tm and the nearest neighbor model), and/or the free energy of the oligonucleotide-target exon complex (using RNA structure version 4.5 or RNA mfold version 3.5). If a Tm is increased, the exon skipping activity typically increases, but when a Tm is too high, the oligonucleotide is expected to become less sequence-specific. An acceptable Tm and free energy depend on the sequence of the oligonucleotide. Therefore, it is difficult to give preferred ranges for each of these parameters.

Exon skipping activity is preferably measured by analysing total RNA isolated from oligonucleotide-treated muscle cell cultures or muscle tissue by reverse transcriptase quantitative or digital droplet polymerase chain reaction (RT-qPCR or RT-ddPCR) using DMD gene-specific primers flanking the targeted exon as described (Aartsma-Rus et al., 2003, Spitali et al., 2013). The ratio of shorter transcript fragments, representing transcripts in which the targeted exon is skipped, to the total of transcript products is assessed (calculated as percentage of exon skipping induced by an oligonucleotide). Shorter fragments may also be sequenced to determine the correctness and specificity of the targeted exon skipping.

In certain embodiments, RNA modulation activity may be an increase or decrease in an amount of a nucleic acid or protein. In certain embodiments, such activity may be a change in the ratio of splice variants of a nucleic acid or protein. Detection and/or measuring of antisense activity may be direct or indirect. In certain embodiments, antisense activity is assessed by observing a phenotypic change in a cell or animal.

As used herein and explained above, "modulation" can refer to a perturbation of amount or quality of a function or activity when compared to the function or activity prior to modulation. For example, modulation includes the change, either an increase (stimulation or induction) or a decrease (inhibition or reduction) in gene expression. As a further example, modulation of expression can include perturbing splice site selection of pre-mRNA processing, resulting in a change in the amount of a particular splice-variant present compared to conditions that were not perturbed. As a further example, modulation includes perturbing translation of a protein.

Biodistribution and biostability are preferably at least in part determined by a validated hybridization ligation assay adapted from Yu et al., 2002. In an embodiment, plasma or homogenized tissue samples are incubated with a specific capture oligonucleotide probe. After separation, a DIG-labeled oligonucleotide is ligated to the complex and detection followed using an anti-DIG antibody-linked peroxidase. Non-compartmental pharmacokinetic analysis is performed using WINNONLIN software package (model 200, version 5.2, Pharsight, Mountainview, CA). Levels of oligonucleotide (µg) per mL plasma or mg tissue are monitored over time to assess area under the curve (AUC), peak concentration (Cₘₐₓ), time to peak concentration (Tₘₐₓ), terminal half life and absorption lag time (t_{lag}). Such a preferred assay has been disclosed in the experimental part.

Accordingly, a preferred oligonucleotide of the invention has an improved parameter, such as an acceptable or a decreased immunogenicity and/or a better biodistribution and/or acceptable or improved RNA binding kinetics and/or thermodynamic properties by comparison to a corresponding oligonucleotide that differs only from the oligonucleotide of the invention through omission of BNA scaffold modifications, i.e. by comparison to an oligonucleotide of the same sequence, also comprising 2'-O-methyl substituted monomers, 5'-methylcytosine and/or 5'-methyluracil, optionally phosphorothioate, yet without a BNA modified scaffold. Each of these parameters could be assessed using assays known to the skilled person or preferably as disclosed herein.

### Further chemical modifications of the oligonucleotide

Below other chemistries and modifications of the oligonucleotide of the invention are defined. These additional chemistries and modifications may be present in combination with the chemistry already defined for said oligonucleotide, i.e. the presence of at least 1 BNA scaffold modification with or without a 5-methylcytosine and/or a 5-methyluracil, and/or the oligonucleotide comprising or consisting of a 2'-O-methyl monomer with optional phosphorothioate backbone linkages.

A preferred oligonucleotide of the invention comprises or consists of an RNA molecule or a modified RNA molecule. In a preferred embodiment, an oligonucleotide is single stranded. The skilled person will understand that it is however possible that a single stranded oligonucleotide may form an internal double stranded structure. However, this oligonucleotide is still named a single stranded oligonucleotide in the context of this invention.

In addition to the modifications described above, the oligonucleotide of the invention may comprise further modifications such as different types of nucleic acid monomers or nucleotides as described below. Different types of nucleic acid monomers may be used to generate an oligonucleotide of the invention. Said oligonucleotide may have at least one backbone, and/or scaffold modification and/or at least one base modification compared to an RNA-based oligonucleotide.

A base modification can include a modified version of the natural purine and pyrimidine bases (e.g. adenine, uracil, guanine, cytosine, and thymine), such as hypoxanthine, pseudouracil, pseudocytosine, 1-methylpseudouracil, orotic acid, agmatidine, lysidine, 2-thiopyrimidine (e.g. 2-thiouracil, 2-thiothymine), G-clamp and its derivatives, 5-substituted pyrimidine (e.g. 5-halouracil, 5-halomethyluracil, 5-trifluoromethyluracil, 5-propynyluracil, 5-propynylcytosine, 5-aminomethyluracil, 5-hydroxymethyluracil, 5-aminomethylcytosine, 5-hydroxymethylcytosine, Super T, or as described in e.g. Kumar et al. J. Org. Chem. 2014, 79, 5047; Leszczynska et al. Org. Biol. Chem. 2014, 12, 1052), pyrazolo[1,5-a]-1,3,5-triazine C-nucleoside (as in e.g. Lefoix et al. J. Org. Chem. 2014, 79, 3221), 7-deazaguanine, 7-deazaadenine, 7-aza-2,6-diaminopurine, 8-aza-7-deazaguanine, 8-aza-7-deazaadenine, 8-aza-7-deaza-2,6-diaminopurine, Super G, Super A, boronated cytosine (as in e.g. Niziot et al. Bioorg. Med. Chem. 2014, 22, 3906), pseudoisocytidine, C(Pyc) (as in *e.g*. Yamada et al. Org. Biomol. Chem. 2014, 12, 2255) and N4-ethylcytosine, or derivatives thereof; *N²*-cyclopentylguanine (cPent-G), *N²*-cyclopentyl-2-aminopurine (cPent-AP), and *N²*-propyl-2-aminopurine (Pr-AP), carbohydrate-modified uracil (as in e.g. Kaura et al. Org. Lett. 2014, 16, 3308), amino acid modified uracil (as in e.g. Guenther et al. Chem. Commun. 2014, 50, 9007); or derivatives thereof; and degenerate or universal bases, like 2,6-difluorotoluene or absent bases like abasic sites (*e.g.* 1-deoxyribose, 1,2-dideoxyribose, 1-deoxy-2-O-methylribose; or pyrrolidine derivatives in which the ring oxygen has been replaced with nitrogen (azaribose)). Examples of derivatives of Super A, Super G and Super T can be found in US patent 6,683,173 (Epoch Biosciences), which is incorporated here entirely by reference. cPent-G, cPent-AP and Pr-AP were shown to reduce immunostimulatory effects when incorporated in siRNA (Peacock H. et al. J. Am. Chem. Soc. 2011, 133, 9200). Examples of modified bases are described in *e.g*. WO2014/093924 (ModeRNA).

Depending on its length an oligonucleotide of the invention may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, or 34 base modifications. It is also encompassed by the invention to introduce more than one distinct base modification in said oligonucleotide.

In addition to BNA scaffold modifications already described, a scaffold modification can include a modified version of the ribosyl moiety, such as 2'-*O*-modified RNA such as 2'-*O*-alkyl or 2'-*O*-(substituted)alkyl *e.g.* 2'-*O*-methyl, 2'-*O*-(2-cyanoethyl), 2'-*O*-(2-methoxy)ethyl (2'-MOE), 2'-*O*-(2-thiomethyl)ethyl, 2'-*O*-butyryl, 2'-*O*-propargyl, 2'-*O*-acetalester (such as *e.g.* Biscans et al. Bioorg. Med. Chem. 2015, 23, 5360), 2'-*O*-allyl, 2'-*O*-(2S-methoxypropyl), 2'-*O-*(*N*-(aminoethyl)carbamoyl)methyl)(2'-AECM), 2'-*O*-(2-carboxyethyl) and carbamoyl derivatives (Yamada et al. Org. Biomol. Chem. 2014, 12, 6457), 2'-*O*-(2-amino)propyl, 2'-*O*-(2-(dimethylamino)propyl), 2'-*O*-(2-amino)ethyl, 2'-*O*-(2-(dimethylamino)ethyl); 2'-deoxy (DNA); 2'-*O*-(haloalkoxy)methyl (Arai K. et al. Bioorg. Med. Chem. 2011, 21, 6285) e.g. 2'-*O*-(2-chloroethoxy)methyl (MCEM), 2'-*O*-(2,2-dichloroethoxy)methyl (DCEM); 2'-*O*-alkoxycarbonyl *e.g.* 2'-*O*-[2-(methoxycarbonyl)ethyl] (MOCE), 2'-*O*-[2-(N-methylcarbamoyl)ethyl] (MCE), 2'-*O-*[2-(*N,N-*dimethylcarbamoyl)ethyl] (DCME), 2'-*O*-[2-(methylthio)ethyl] (2'-MTE), 2'-(ω-*O-*serinol); 2'-halo *e.g.* 2'-F, FANA (2'-F arabinosyl nucleic acid); 2',4'-difluoro-2'-deoxy; carbasugar and azasugar modifications; 3'-*O*-substituted *e.g.* 3'-*O*-methyl, 3'-*O*-butyryl, 3'-O-propargyl; 4'-substituted *e.g.* 4'-aminomethyl-2'-*O*-methyl or 4'-aminomethyl-2'-fluoro; 5'-subtituted *e.g.* 5'-methyl or CNA (Østergaard et al. ACS Chem. Biol. 2014, 22, 6227); and their derivatives.

Depending on its length an oligonucleotide of the invention may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, or 32, or 33 scaffold modifications additional to the at least 1 BNA scaffold modification. It is also encompassed by the invention to introduce more than one distinct scaffold modification in said oligonucleotide.

Other modifications include unlocked nucleic acid (UNA); cyclohexenyl nucleic acid (CeNA), F-CeNA, cyclohexanyl nucleic acid (CNA), ribo-cyclohexanyl nucleic acid (r-CNA), altritol nucleic acid (ANA), hexitol nucleic acid (HNA), fluorinated HNA (F-HNA), pyranosyl-RNA (p-RNA), 3'-deoxypyranosyl-DNA (p-DNA); and their derivatives. Examples of fluorinated nucleic acid analogues with furanose and non-furanose sugar rings are also encompassed and are described in *e.g.* Østergaard et al. J. Org. Chem. 2014, 79, 8877.

Depending on its length an oligonucleotide of the invention may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, or 34 scaffold modifications in addition to the at least 1 BNA scaffold modification. In a preferred embodiment, an oligonucleotide of the invention is fully 2'-*O*-methyl modified and contains 1, 2, 3, 4, 5, or 6 BNA scaffold modifications.

Oligonucleotides according to the invention can comprise backbone linkage modifications. A backbone linkage modification can be, but is not limited to, a modified version of the phosphodiester present in RNA, such as phosphorothioate (PS), chirally pure phosphorothioate, (*R*)-phosphorothioate, (*S*)-phopshorothioate, phosphorodithioate (PS2), phosphonoacetate (PACE), phosphonoacetamide (PACA), thiophosphonoacetate (thioPACE), thiophosphonoacetamide, phosphorothioate prodrug, H-phosphonate, methyl phosphonate, methyl phosphonothioate, methyl phosphate, methyl phosphorothioate, ethyl phosphate, ethyl phosphorothioate, boranophosphate, boranophosphorothioate, methyl boranophosphate, methyl boranophosphorothioate, methyl boranophosphonate, methyl boranophosphonothioate, phosphate, phosphotriester, aminoalkylphosphotriester, and their derivatives. Another modification includes phosphoryl guanidine, phosphoramidite, phosphoramidate, N3'→P5' phosphoramidate, phosphordiamidate, phosphorothiodiamidate, sulfamate, dimethylenesulfoxide, amide, sulfonate, siloxane, sulfide, sulfone, formacetyl, thioformacetyl, methylene formacetyl, alkenyl, methylenehydrazino, sulfonamide, amide, triazole, oxalyl, carbamate, methyleneimino (MMI), and thioacetamido nucleic acid (TANA); and their derivatives. Examples of chirally pure phosphorothioate linkages are described in e.g. WO2014/010250 or WO2017/062862 (WaVe Life Sciences). Examples of phosphoryl guanidine linkages are described in WO2016/028187 (Noogen). Various salts, mixed salts and free acid forms are also included, as well as 3'→3' and 2'→5' linkages.

Depending on its length, an oligonucleotide of the invention may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, or 33 backbone linkage modifications. It is also encompassed by the invention to introduce more than one distinct backbone modification in said oligonucleotide.

In a preferred embodiment, an oligonucleotide of the invention comprises at least one phosphorothioate modification. In a more preferred embodiment, an oligonucleotide of the invention is fully phosphorothioate modified. In another preferred embodiment, an oligonucleotide of the invention comprises at least one phosphate.

Other chemical modifications of an oligonucleotide of the invention include the substitution of one or more than one of any of the hydrogen atoms with deuterium or tritium, examples of which can be found in *e.g.* WO2014/022566 (Ased) or WO2015/011694 (Celgene).

With the advent of nucleic acid mimicking technology it has become possible to generate molecules that have a similar, preferably the same hybridization characteristics in kind not necessarily in amount as nucleic acid itself. Such functional equivalents are of course also suitable for use in the invention.

The skilled person will understand that not each scaffold, base, and/or backbone may be modified the same way. Several distinct modified scaffolds, bases and/or backbones may be combined into one single oligonucleotide of the invention.

In one embodiment, the oligonucleotides according to the invention are 10 to 33 nucleotides in length, wherein:
a) at least one monomer has formula I: 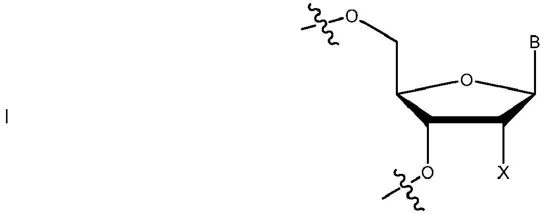 wherein:
   B is a nucleobase;
   X is F, -NR¹R², or-OR;
   R is alkenyl or optionally substituted alkyl, where the optional substituents, when present, are halo, OR¹, NR¹R², or SR¹;
   R¹ is H, alkyl, cycloalkyl, aryl, heterocycloalkyl, or heteroaryl, each independently optionally further substituted with halo, hydroxy, or alkyl;
   R² is H or alkyl; and 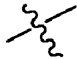
   indicates the point of attachment to the remainder of the oligonucleotide;
b) wherein at least one monomer comprises a BNA scaffold modification and has formula II: 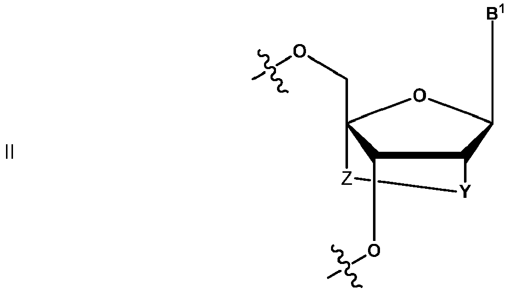 wherein:
   B¹ is a nucleobase;
   Z-Y is a divalent group selected from -(CH₂)ₙO-, -C(CH₂CH₂)O-, -CH₂WCH₂-, -(CH₂)ₙNR³-, -CH₂S(Oₘ)-, -CH(CH₃)O-, -CH(CH₂OCH₃)O-, -CH₂N(R³)O-, -CH₂CH₂-, -C(O)NR³-, -CH=CHO-, -CH₂SO₂NR³-, and -NHC(O)NH-;
   n is 1 or 2;
   m is 0, 1 or 2;
   W is O, S or NR³;
   R³ is H, -C(O)R⁴, -C(=NH)NR⁵R⁵, benzyl, or optionally substituted alkyl, where the optional substituents, when present, are selected from halo and alkoxy;
   R⁴ is alkyl, cycloalkyl or aryl;
   R⁵ is H or alkyl; and 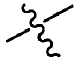
   indicates the point of attachment to the remainder of the oligonucleotide;
c) wherein the monomers are linked by phosphorothioate backbone linkages and/or by phosphodiester backbone linkages; and
d) wherein at least one nucleobase in the oligonucleotide is a 5-methylcytosine or a 5-methyluracil base.

The term "the remainder of the oligonucleotide", as used herein, generally relates to neighbouring monomers. As a skilled person will understand, when a monomer of formula I or of formula II is a terminal monomer, a remainder of the oligonucleotide can be -H. For any monomer of formula I or of formula II, both remainders can be neighbouring monomers. In contrast, for any monomer of formula I or of formula II at most a single remainder can constitute a terminus and thus for example be -H. As is understood by a skilled person, an entire oligonucleotide has only two termini.

Z-Y is a divalent group. It is preferred that such a divalent group is connected to the 4'-position of the scaffold (near Z) with the bond on the left side of its textual representation, and to the 2'-position of the scaffold (near Y) with the bond on the right side of its textual representation. For example, when the divalent group is said to be -CH₂-O-, it is preferred that -CH₂- is connected to the 4'-position of the scaffold, and that -O- is connected to the 2'-position of the scaffold. This would form an LNA monomer.

Said oligonucleotide is preferably for use in methods or compositions according to the invention. Said nucleobase under d) may be B or B¹ or another nucleobase.

In another embodiment, X is F or -OR. In another embodiment, X is F. In another embodiment, X is -OR. In another embodiment, X is F, -OCH₃ or -O-CH₂CH₂OCH₃. In another embodiment, X is -OCH₃ or -O-CH₂CH₂OCH₃. In another embodiment, X is -OCH₃. In another embodiment, X is F or -OCH₃. In another embodiment, X is For -O-CH₂CH₂OCH₃.

In another embodiment, R is unsubstituted alkyl. In another embodiment, R is CH₃ or ethyl. In another embodiment, R is CH₃. In another embodiment, R is ethyl. In another embodiment, R is alkyl substituted with halo, OR¹, NR¹R² or SR¹. In another embodiment, R is alkyl substituted with OR¹ or NR¹R². In another embodiment, R is alkyl substituted with OR¹.

In another embodiment, R¹ is H or unsubstituted alkyl. In another embodiment, R¹ is unsubstituted alkyl. In another embodiment, R¹ is CH₃. In another embodiment, R² is H. In another embodiment, R² is alkyl. In another embodiment, R² is CH₃.

In another embodiment, Z-Y is a divalent group selected from -(CH₂)ₙO-, -C(CHzCHz)O-, -CH₂WCH₂-, -CH₂NR³-, -CH₂S(Oₘ)-, -CH(CH₃)O-, -CH(CH₂OCH₃)O-, -CH₂CH₂-, -C(O)NR³-, -CH=CHO-, -CH₂SO₂NR³- and -NHC(O)NH-. In another embodiment, Z-Y is a divalent group selected from -(CH₂)ₙO-, -CH₂WCH₂-, -CH₂NR³-, -CH(CH₃)O-, -CH(CH₂OCH₃)O-, -CH₂CH₂- and -C(O)NR³-. In another embodiment, Z-Y is selected from -(CH₂)ₙO-, -CH₂NR³-, -CH(CH₃)O-, -CH(CH₂OCH₃)O-, -CH₂CH₂- and -C(O)NR³-. In another embodiment, Z-Y is selected from-(CH₂)ₙO-, -CH₂OCH₂-, -CH₂NR³CH₂-, and -CH₂NR³-. In another embodiment, Z-Y is selected from -(CH₂)ₙO-, -CH₂OCH₂-, -CH₂NHCH₂-, -CH₂NH-, -CH₂N(CH₃)CH₂-, and -CH₂N(CH₃)-. In another embodiment, Z-Y is selected from -CH₂O-, -CH₂OCH₂-, -CH₂NHCH₂-, and -CH₂NH-. In another embodiment, Z-Y is selected from -CH₂O-, -CH₂OCH₂-, or -CH₂NH-. In another embodiment, Z-Y is selected from -(CH₂)ₙO-, -CH(CH₃)O- and -CH(CH₂OCH₃)O-. In another embodiment, Z-Y is -(CH₂)ₙO-. In another embodiment, Z-Y is -CH₂CH₂O-. In another embodiment, Z-Y is -CH₂O-. In another embodiment, Z-Y is -CH₂NH-.

In another embodiment, W is O, S or NH. In another embodiment, W is O. In another embodiment, W is S. In another embodiment, W is O, NH, or NCH₃. In another embodiment, W is O or NH. In another embodiment, W is NH.

In another embodiment, R³ is H, -C(O)R⁴ or unsubstituted alkyl. In another embodiment, R³ is H, -C(O)R⁴ or CH₃. In another embodiment, R³ is H, -C(O)CH₃ or CH₃.

In another embodiment, R⁴ is alkyl. In another embodiment, R⁴ is CH₃.

In another embodiment, R⁵ is H. In another embodiment, R⁵ is alkyl.

In another embodiment, X is F, -OCH₃ or -O-CH₂CH₂OCH₃; and Z-Y is -(CH₂)ₙO-, -CH₂OCH₂-, -CH₂NR³CH₂-, or -CH₂NR³-. In another embodiment, X is F, -OCH₃ or -O-CH₂CH₂OCH₃; and Z-Y is -(CH₂)ₙO-, -CH₂OCH₂-, -CH₂NHCH₂-, or -CH₂NH, -CH₂N(CH₃)CH₂-, or -CH₂N(CH₃)-. In another embodiment, X is F, -OCH₃ or -O-CH₂CH₂OCH₃; and Z-Y is -CH₂O-, -CH₂OCH₂-, -CH₂NHCH₂-, or -CH₂NH-. In another embodiment, X is F, -OCH₃ or -O-CH₂CH₂OCH₃; and Z-Y is selected from -CH₂O-, -CH₂OCH₂-, and -CH₂NH-. In another embodiment, X is F or -OCH₃; and Z-Y is selected from -CH₂O-, -CH₂OCH₂-, and -CH₂NH-. In another embodiment, X is F or OCH₃; and Z-Y is -CH₂O-.

In another embodiment, at least one B in the oligonucleotide is a 5-methylcytosine or a 5-methyluracil base. In another embodiment, at least one B¹ in the oligonucleotide is a 5-methylcytosine or a 5-methyluracil base. In another embodiment, all of the cytosine nucleobases in the oligonucleotide are 5-methylcytosine. In another embodiment, all of the uracil nucleobases in the oligonucleotide are 5-methyluracil.

In another embodiment, the oligonucleotides are 10 to 33 nucleotides in length, wherein:
a) at least one monomer has formula I, wherein
   B is a nucleobase;
   X is F, -NR¹R², or-OR;
   R is optionally substituted alkyl, where the optional substituents, when present, are halo, OR¹, NR¹R², or SR¹;
   R¹ is H, alkyl, cycloalkyl, aryl, heterocycloalkyl, or heteroaryl, each independently optionally further substituted with halo, hydroxy or alkyl;
   R² is H or alkyl; and 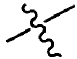
   indicates the point of attachment to the remainder of the oligonucleotide;
b) wherein at least one monomer comprises a BNA scaffold modification;
c) wherein the monomers are linked by phosphorothioate backbone linkages and/or by phosphodiester backbone linkages; and
d) wherein at least one nucleobase in the oligonucleotide is a 5-methylcytosine or a 5-methyluracil base.

In another embodiment, the oligonucleotides are 10 to 33 nucleotides in length, wherein:
a) at least one monomer has formula I, wherein
   B is a nucleobase;
   X is F, -NR¹R², or-OR;
   R is optionally substituted alkyl, where the optional substituents, when present, are halo, OR¹, NR¹R², or SR¹;
   R¹ is H, alkyl, cycloalkyl, aryl, heterocycloalkyl, or heteroaryl, each independently optionally further substituted with halo, hydroxy or alkyl;
   R² is H or alkyl; and 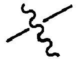
   indicates the point of attachment to the remainder of the oligonucleotide;
b) wherein at least one monomer comprises a BNA scaffold modification and has formula II, wherein B¹ and Z-Y are as defined above;
c) wherein the monomers are linked by phosphorothioate backbone linkages and/or by phosphodiester backbone linkages; and
d) wherein at least one nucleobase in the oligonucleotide is a 5-methylcytosine or a 5-methyluracil base.

In another embodiment, the BNA scaffold modification results in a monomer that is CRN, LNA, Xylo-LNA, α-LNA, α-L-LNA, β-D-LNA, 2'-amino-LNA, 2'-(alkylamino)-LNA, 2'-(acylamino)-LNA, 2'-thio-LNA, cEt BNA, cMOE BNA, cLNA, amido-bridged LNA; 2',4'-BNA^{NC}(N-H); 2',4'-BNA^{NC}(N-Me); 2',4'-BNA^{NC}(N-Bn), CBBN, ENA, DpNA, sulfonamide-bridged BNA, urea-bridged BNA, bicyclic carbocyclic nucleotide, TriNA, α-L-TriNA, bcDNA, tcDNA, F-bcDNA, F-tcDNA, heterocyclic-bridged BNA, locked PMO derived from 2'-amino-LNA, GuNA, or scpNA.

In another embodiment, the BNA scaffold modification results in a monomer that is CRN, LNA, Xylo-LNA, α-LNA, α-L-LNA, β-D-LNA, 2'-amino-LNA, 2'-(alkylamino)-LNA, 2'-(acylamino)-LNA, 2'-thio-LNA, cEt BNA, cMOE BNA, cLNA, amido-bridged LNA; 2',4'-BNA^{NC}(N-H); 2',4'-BNA^{NC}(N-Me); 2',4'-BNA^{NC}(N-Bn), CBBN, ENA, DpNA, sulfonamide-bridged BNA, urea-bridged BNA. In another embodiment, the BNA scaffold modification results in a monomer that is CRN, LNA, Xylo-LNA, α-LNA, α-L-LNA, β-D-LNA, 2'-amino-LNA, or 2'-(alkylamino)-LNA. In another embodiment, the BNA scaffold modification results in a monomer that is CRN, LNA, 2'-amino-LNA, or CBBN. In another embodiment, the BNA scaffold modification results in a monomer that is CRN, LNA, or 2'-amino-LNA. In another embodiment, the BNA scaffold modification results in a monomer that is LNA, Xylo-LNA, α-LNA, α-L-LNA, β-D-LNA, 2'-amino-LNA, 2'-(alkylamino)-LNA, 2'-(acylamino)-LNA, 2'-thio-LNA, cEt BNA, cMOE BNA, or cLNA. In another embodiment, the BNA scaffold modification results in a monomer that is LNA, 2'-amino-LNA, 2'-(alkylamino)-LNA, 2'-thio-LNA, cEt BNA, cMOE BNA, or cLNA. In another embodiment, the BNA scaffold modification results in a monomer that is LNA, 2'-amino-LNA, 2'-(alkylamino)-LNA, 2',4'-BNA^{NC}(N-H); 2',4'-BNA^{NC}(N-Me), CBBN, or ENA. In another embodiment, the BNA scaffold modification results in a monomer that is LNA, 2'-amino-LNA, 2',4'-BNA^{NC}(N-H), CBBN, or ENA. In another embodiment, the BNA scaffold modification results in a monomer that is LNA, CBBN, or ENA. In another embodiment, the BNA scaffold modification results in a monomer that is LNA or ENA. In another embodiment, the BNA scaffold modification results in a monomer that is LNA.

In another embodiment, the BNA scaffold modification results in a monomer that is 2',4'-BNA^{NC}(N-H); 2',4'-BNA^{NC}(N-Me); 2',4'-BNA^{NC}(N-Bn), CBBN, ENA, sulfonamide-bridged BNA, or urea-bridged BNA. In another embodiment, the BNA scaffold modification results in a monomer that is 2',4'-BNA^{NC}(N-H); 2',4'-BNA^{NC}(N-Me), CBBN, or ENA.

In another embodiment, the oligonucleotides are 10 to 33 nucleotides in length, wherein:
a) at least one monomer has formula I, wherein:
   B is a nucleobase;
   X is F, -OCH₃, or -O-CH₂CH₂OCH₃; and 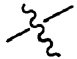
   indicates the point of attachment to the remainder of the oligonucleotide;
b) wherein at least one monomer comprises a BNA scaffold modification and has formula II, wherein
   B¹ is a nucleobase;
   Z-Y is a divalent group selected from -(CH₂)ₙO-, -C(CHzCHz)O-, -CH₂WCH₂-, -CH₂NR³-, -CH₂S-, -CH(CH₃)O-, -CH(CH₂OCH₃)O-, -CH₂CH₂-, -C(O)NR³-, -CH=CHO-, -CH₂SO₂NR³-, and -NHC(O)NH-;
   n is 1 or 2;
   W is O, S, or NR³;
   R³ is H, -C(O)R⁴, -C(=NH)NR⁵R⁵, benzyl, or optionally substituted alkyl, where the optional substituents, when present, are selected from halo and alkoxy;
   R⁴ is alkyl, cycloalkyl, or aryl;
   R⁵ is H or alkyl; and 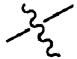
   indicates the point of attachment to the remainder of the oligonucleotide;
c) wherein the monomers are linked by phosphorothioate backbone linkages and/or by phosphodiester backbone linkages; and
d) wherein at least one nucleobase in the oligonucleotide is a 5-methylcytosine or a 5-methyluracil base.

In another embodiment, the oligonucleotides are 10 to 33 nucleotides in length, wherein:
a) at least one monomer has formula I wherein:
   B is a nucleobase;
   X is F, -OCH₃, or -O-CH₂CH₂OCH₃; and 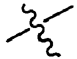
   indicates the point of attachment to the remainder of the oligonucleotide;
b) wherein one or two monomers comprise a BNA scaffold modification and have formula II, wherein:
   B¹ is a nucleobase;
   Z-Y is a divalent group selected from -(CH₂)ₙO-, -C(CHzCHz)O-, -CH₂WCH₂-, -CH₂NR³-, -CH₂S-, -CH(CH₃)O-, -CH(CH₂OCH₃)O-, -CH₂CH₂-, -C(O)NR³-, -CH=CHO-, -CH₂SO₂NR³-, and -NHC(O)NH-;
   n is 1 or 2;
   W is O, S, or NR³;
   R³ is H, -C(O)R⁴, -C(=NH)NR⁵R⁵, benzyl, or optionally substituted alkyl, where the optional substituents, when present, are selected from halo and alkoxy;
   R⁴ is alkyl, cycloalkyl, or aryl;
   R⁵ is H or alkyl; and 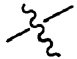
   indicates the point of attachment to the remainder of the oligonucleotide;
c) wherein the monomers are linked by phosphorothioate backbone linkages and/or by phosphodiester backbone linkages; and
d) wherein at least one nucleobase in the oligonucleotide is a 5-methylcytosine or a 5-methyluracil base.

In another embodiment, the oligonucleotides are 10 to 33 nucleotides in length, wherein all the monomers that do not comprise a BNA scaffold modification ("the non-BNA monomers") are modified monomers of formula I. In another embodiment, the oligonucleotides are 10 to 33 nucleotides in length, with one or two non-BNA monomers of formula I. In another embodiment, the oligonucleotides are 10 to 33 nucleotides in length, with one non-BNA monomer of formula I.

In another embodiment, the oligonucleotide comprises 1, 2, 3, 4, 5, 6, or 7 monomers comprising a BNA scaffold modification. In another embodiment, the oligonucleotide comprises 1, 2, 3, 4, 5, 6, or 7 monomers comprising a BNA scaffold modification and having formula II. In another embodiment, the oligonucleotide comprises 1, 2, 3, or 4 monomers comprising a BNA scaffold modification. In another embodiment, the oligonucleotide comprises 1, 2, 3, or 4 monomers comprising a BNA scaffold modification and having formula II. In another embodiment, the oligonucleotide comprises 1, 2, or 3 monomers comprising a BNA scaffold modification. In another embodiment, the oligonucleotide comprises 1, 2, or 3 monomers comprising a BNA scaffold modification and having formula II. In another embodiment, the oligonucleotide comprises 1 or 2 monomers comprising a BNA scaffold modification. In another embodiment, the oligonucleotide comprises 1 or 2 monomers comprising a BNA scaffold modification and having formula II. In another embodiment, the oligonucleotide comprises two monomers comprising a BNA scaffold modification. In another embodiment, the oligonucleotide comprises two monomers comprising a BNA scaffold modification and having formula II. In another embodiment, the oligonucleotide comprises one monomer comprising a BNA scaffold modification. In another embodiment, the oligonucleotide comprises one monomer comprising a BNA scaffold modification and having formula II.

In another embodiment, the oligonucleotide according to the invention comprises the sequence GGAAGAUGGCAU (SEQ ID NO: 6072). In another embodiment, the oligonucleotide according to the invention comprises a sequence selected from the group consisting of SEQ ID NOs: 453, 455, 456, 453, 455, 456, 459, 461, 462, 465, 467, 468, 471, 473, 474, 483, 486, 525, 531, 538, 539, 540, 543, 545, 546, and 4528-4572. In another embodiment, the oligonucleotide according to the invention comprises a sequence selected from the group consisting of SEQ ID NOs: 453, 455, 459, 4528, 4531, 4532, 4533, 4535, 4542, 4548, and 4568. In another embodiment, the oligonucleotide according to the invention comprises a sequence selected from the group consisting of SEQ ID NOs: 453, 455, and 456. In another embodiment, the oligonucleotide according to the invention comprises a sequence selected from the group consisting of SEQ ID NOs: 455 and 459. In another embodiment, the oligonucleotide according to the invention comprises a sequence selected from the group consisting of SEQ ID NOs: 4528, 4531, 4532, 4533, 4535, 4542, 4548, and 4568. In another embodiment, the oligonucleotide according to the invention comprises a sequence selected from the group consisting of SEQ ID NOs: 459, 4528, 4531, 4532, 4533, and 4542.

In another embodiment, the oligonucleotide according to the invention comprises the sequence GGAAGAUGGCAU (SEQ ID NO: 6072) and is for skipping exon 51 of pre-mRNA of dystrophin. In another embodiment, the oligonucleotide according to the invention comprises a sequence selected from the group consisting of SEQ ID NOs: 452-613 or SEQ ID NOs: 4528-4572 and is for skipping exon 51 of pre-mRNA of dystrophin. In another embodiment, the oligonucleotide according to the invention comprises a sequence selected from the group consisting of SEQ ID NOs: 453, 455, and 456 and is for skipping exon 51 of pre-mRNA of dystrophin. In another embodiment, the oligonucleotide according to the invention comprises a sequence selected from the group consisting of SEQ ID NOs: 455 and 459 and is for skipping exon 51 of pre-mRNA of dystrophin.

In another embodiment, the oligonucleotide according to the invention comprises the sequence GGUAAGUUCNGUCCAAGC (SEQ ID NO: 6073), wherein N is T or U. In another embodiment, the oligonucleotide according to the invention comprises the sequence GGUAAGUUCNGUCCAAGC (SEQ ID NO: 6073), wherein N is T or U, and is for skipping exon 51 of pre-mRNA of dystrophin. In another embodiment, the oligonucleotide according to the invention comprises a sequence selected from the group consisting of SEQ ID NOs: 4565-4571. In another embodiment, the oligonucleotide according to the invention comprises a sequence selected from the group consisting of SEQ ID NOs: 4565-4571 and is for skipping exon 51 of pre-mRNA of dystrophin.

In another embodiment, the oligonucleotide according to the invention comprises a sequence selected from the group consisting of SEQ ID NOs: 4561-4564 and SEQ ID NO:4572. In another embodiment, the oligonucleotide according to the invention comprises a sequence selected from the group consisting of SEQ ID NOs: 4561-4564 and SEQ ID NO:4572 and is for skipping exon 51 of pre-mRNA of dystrophin.

In another embodiment, the oligonucleotide according to the invention comprises the sequence CCCAAUUUUUCCUG (SEQ ID NO: 6074). In another embodiment, the oligonucleotide according to the invention comprises the sequence CCCAAUGCCAUCCUG (SEQ ID NO: 6075). In another embodiment, the oligonucleotide according to the invention comprises a sequence selected from the group consisting of SEQ ID NOs: 3185, 3573, 3855, 4198, and 4401. In another embodiment, the oligonucleotide according to the invention comprises a sequence selected from the group consisting of SEQ ID NOs: 3185, 3573, 3855, and 4401. In another embodiment, the oligonucleotide according to the invention comprises the sequence CCCAAUUUUUCCUG (SEQ ID NO: 6074) and is for skipping exon 45 of pre-mRNA of dystrophin. In another embodiment, the oligonucleotide according to the invention comprises the sequence CCCAAUGCCAUCCUG (SEQ ID NO: 6075) and is for skipping exon 45 of pre-mRNA of dystrophin. In another embodiment, the oligonucleotide according to the invention comprises a sequence selected from the group consisting of SEQ ID NOs: 3185, 3573, 3855, 4198, and 4401 and is for skipping exon 45 of pre-mRNA of dystrophin. In another embodiment, the oligonucleotide according to the invention comprises a sequence selected from the group consisting of SEQ ID NOs: 3185, 3573, 3855, and 4401 and is for skipping exon 45 of pre-mRNA of dystrophin.

In preferred embodiments, the oligonucleotide according to the invention comprises the sequence CUUCUGUUAGCC (SEQ ID NO: 6076). Such an oligonucleotide preferably has a length of 16 to 24 nucleotides, more preferably of 20 nucleotides. In this context, a preferred embodiment is the antisense oligonucleotide consisting of the nucleotide sequence represented by SEQ ID NO: 29.

In preferred embodiments, the oligonucleotide according to the invention comprises the sequence UAUUUAGCA (SEQ ID NO: 6077). Such an oligonucleotide preferably has a length of 16 to 24 nucleotides, more preferably of 23 nucleotides. In this context, a preferred embodiment is the antisense oligonucleotide consisting of the nucleotide sequence represented by SEQ ID NO: 161.

In preferred embodiments, the oligonucleotide according to the invention comprises the sequence GGAAUUUGU (SEQ ID NO: 6078). Such an oligonucleotide preferably has a length of 16 to 24 nucleotides, more preferably of 23 nucleotides. In this context, a preferred embodiment is the antisense oligonucleotide consisting of the nucleotide sequence represented by SEQ ID NO: 119.

In preferred embodiments, the oligonucleotide according to the invention comprises the sequence CUCAACAGA (SEQ ID NO: 6079). Such an oligonucleotide preferably has a length of 16 to 24 nucleotides, more preferably of 23 nucleotides. In this context, a preferred embodiment is the antisense oligonucleotide consisting of the nucleotide sequence represented by SEQ ID NO: 233.

When an oligonucleotide according to the invention is for skipping exon 44 of pre-mRNA of dystrophin, it is preferred that it comprises a sequence represented by SEQ ID NOs: 6076-6079.

In preferred embodiments, the oligonucleotide according to the invention comprises the sequence GCCCAAU (SEQ ID NO: 6080). Such an oligonucleotide preferably has a length of 16 to 26 nucleotides, more preferably of 25 nucleotides. In this context, a preferred embodiment is the antisense oligonucleotide consisting of the nucleotide sequence represented by SEQ ID NO: 3185.

In preferred embodiments, the oligonucleotide according to the invention comprises the sequence CCAAUUUU (SEQ ID NO: 6081). Such an oligonucleotide preferably has a length of 16 to 26 nucleotides, more preferably of 24 nucleotides. In this context, a preferred embodiment is the antisense oligonucleotide consisting of the nucleotide sequence represented by SEQ ID NO: 3573.

In preferred embodiments, the oligonucleotide according to the invention comprises the sequence GCCCAAU (SEQ ID NO: 6082). Such an oligonucleotide preferably has a length of 16 to 26 nucleotides, more preferably of 25 nucleotides. In this context, a preferred embodiment is the antisense oligonucleotide consisting of the nucleotide sequence represented by SEQ ID NO: 3855.

In preferred embodiments, the oligonucleotide according to the invention comprises the sequence UCUGACAACA (SEQ ID NO: 6083). Such an oligonucleotide preferably has a length of 16 to 24 nucleotides, more preferably of 22 nucleotides. In this context, a preferred embodiment is the antisense oligonucleotide consisting of the nucleotide sequence represented by SEQ ID NO: 4198.

In preferred embodiments, the oligonucleotide according to the invention comprises the sequence CAAUGCCAUCC (SEQ ID NO: 6084). Such an oligonucleotide preferably has a length of 16 to 24 nucleotides, more preferably of 21 nucleotides. In this context, a preferred embodiment is the antisense oligonucleotide consisting of the nucleotide sequence represented by SEQ ID NO: 4401.

When an oligonucleotide according to the invention is for skipping exon 45 of pre-mRNA of dystrophin, it is preferred that it comprises a sequence represented by SEQ ID NOs: 6074, 6075, or 6080-6084.

In preferred embodiments, the oligonucleotide according to the invention comprises the sequence AAGAUGGCAU (SEQ ID NO: 6085). Such an oligonucleotide preferably has a length of 16 to 24 nucleotides, more preferably of 22 nucleotides. In this context, a preferred embodiment is the antisense oligonucleotide consisting of the nucleotide sequence represented by SEQ ID NO: 459.

In preferred embodiments, the oligonucleotide according to the invention comprises the sequence UAAGUUCUGUCCAA (SEQ ID NO: 6086). Such an oligonucleotide preferably has a length of 16 to 24 nucleotides, more preferably of 18 nucleotides. In this context, a preferred embodiment is the antisense oligonucleotide consisting of the nucleotide sequence represented by SEQ ID NO: 4565.

When an oligonucleotide according to the invention is for skipping exon 51 of pre-mRNA of dystrophin, it is preferred that it comprises a sequence represented by SEQ ID NOs: 6072, 6073, 6085 or 6086.

In preferred embodiments, the oligonucleotide according to the invention comprises the sequence GUUGCCUCCGGUUC (SEQ ID NO: 6087). Such an oligonucleotide preferably has a length of 16 to 24 nucleotides, more preferably of 18 nucleotides. In this context, a preferred embodiment is the antisense oligonucleotide consisting of the nucleotide sequence represented by SEQ ID NO: 845.

In preferred embodiments, the oligonucleotide according to the invention comprises the sequence GGUUCUG (SEQ ID NO: 6088). Such an oligonucleotide preferably has a length of 16 to 26 nucleotides, more preferably of 25 nucleotides. In this context, a preferred embodiment is the antisense oligonucleotide consisting of the nucleotide sequence represented by SEQ ID NO: 863.

In preferred embodiments, the oligonucleotide according to the invention comprises the sequence GAUUCUGAAU (SEQ ID NO: 6089). Such an oligonucleotide preferably has a length of 16 to 24 nucleotides, more preferably of 22 nucleotides. In this context, a preferred embodiment is the antisense oligonucleotide consisting of the nucleotide sequence represented by SEQ ID NO: 4987.

In preferred embodiments, the oligonucleotide according to the invention comprises the sequence ACUUCAUC (SEQ ID NO: 6090). Such an oligonucleotide preferably has a length of 16 to 26 nucleotides, more preferably of 24 nucleotides. In this context, a preferred embodiment is the antisense oligonucleotide consisting of the nucleotide sequence represented by SEQ ID NO: 5174.

In preferred embodiments, the oligonucleotide according to the invention comprises the sequence UUCCAUGA (SEQ ID NO: 6091). Such an oligonucleotide preferably has a length of 16 to 26 nucleotides, more preferably of 24 nucleotides. In this context, a preferred embodiment is the antisense oligonucleotide consisting of the nucleotide sequence represented by SEQ ID NO: 5446.

In preferred embodiments, the oligonucleotide according to the invention comprises the sequence UGUUGCCU (SEQ ID NO: 6092). Such an oligonucleotide preferably has a length of 16 to 26 nucleotides, more preferably of 24 nucleotides. In this context, a preferred embodiment is the antisense oligonucleotide consisting of the nucleotide sequence represented by SEQ ID NO: 5765.

When an oligonucleotide according to the invention is for skipping exon 53 of pre-mRNA of dystrophin, it is preferred that it comprises a sequence represented by SEQ ID NOs: 6087-6092.

### Composition

In an aspect of the invention is provided a composition comprising at least one oligonucleotide according to the invention, preferably wherein said composition comprises at least one excipient, and/or wherein said oligonucleotide comprises at least one conjugated ligand, that may further aid in enhancing the targeting and/or delivery of said composition and/or said oligonucleotide to a tissue and/or cell and/or into a tissue and/or cell. Compositions as described here are herein referred to as compositions according to the invention. A composition according to the invention can comprise one or more than one oligonucleotide according to the invention. In the context of this invention, an excipient can be a distinct molecule, but it can also be a conjugated moiety. In the first case, an excipient can be a filler, such as starch. In the latter case, an excipient can for example be a targeting ligand that is linked to the oligonucleotide according to the invention.

In preferred embodiments of this aspect, such compositions can further comprise a cationic amphiphilic compound (CAC) or a cationic amphiphilic drug (CAD). A CAC is generally a lysosomotropic agent and a weak base that can buffer endosomes and lysosomes (Mae et al., Journal of Controlled Release 134:221-227, 2009). Compositions that further comprise a CAC preferably have an improved parameter for RNA modulation as compared to a similar composition that does not comprise said CAC. Without being bound to theory, it is thought that this is because a CAC can aid an oligonucleotide of the invention in reaching its site of activity, for example through facilitating endosomal escape. A preferred CAC as comprised in compositions of the invention is toremifene and its derivatives, analogues, and metabolites, such as N-desmethyl toremifene, tamoxifen, afimoxifene, clomiphene, droloxifene, idoxifene, miproxifene, and nafoxidine. These CACs share a common 1,1-diphenylethylene moiety. When a compound differs from a compound as described here only through minor substitutions or modifications, said compound is a derivative of such a compound, and can also be seen as an analogue thereof. Metabolites of a compound are a special class of derivatives. For compounds that are known in the art, metabolites are often also known. At least all of these known metabolites are referred to when a metabolite of a compound is referenced. For example, N-desmethyl toremifene is a known metabolite of toremifene.

In a preferred embodiment, said composition is for use as a medicament. Said composition is therefore a pharmaceutical composition. A pharmaceutical composition usually comprises a pharmaceutically accepted carrier, diluent and/or excipient. In a preferred embodiment, a composition of the current invention comprises a compound as defined herein and optionally further comprises a pharmaceutically acceptable formulation, filler, preservative, solubilizer, carrier, diluent, excipient, salt, adjuvant and/or solvent. Such pharmaceutically acceptable carrier, filler, preservative, solubilizer, diluent, salt, adjuvant, solvent and/or excipient may for instance be found in Remington: The Science and Practice of Pharmacy, 20th Edition. Baltimore, MD: Lippincott Williams & Wilkins, 2000. The compound as described in the invention may possess at least one ionizable group. An ionizable group may be a base or acid, and may be charged or neutral. An ionizable group may be present as ion pair with an appropriate counterion that carries opposite charge(s). Examples of cationic counterions are sodium, potassium, cesium, Tris, lithium, calcium, magnesium, trialkylammonium, triethylammonium, and tetraalkylammonium. Examples of anionic counterions are chloride, bromide, iodide, lactate, mesylate, besylate, triflate, acetate, trifluoroacetate, dichloroacetate, tartrate, lactate, and citrate. Examples of counterions have been described [e.g. Kumar, **2008]** which is incorporated here in its entirety by reference].

A pharmaceutical composition may comprise an aid in enhancing the stability, solubility, absorption, bioavailability, activity, pharmacokinetics, pharmacodynamics, cellular uptake, and intracellular trafficking of said compound, in particular an excipient capable of forming complexes, nanoparticles, microparticles, nanotubes, nanogels, hydrogels, poloxamers or pluronics, polymersomes, colloids, microbubbles, vesicles, micelles, lipoplexes, and/or liposomes. Examples of nanoparticles include polymeric nanoparticles, (mixed) metal nanoparticles, carbon nanoparticles, gold nanoparticles, magnetic nanoparticles, silica nanoparticles, lipid nanoparticles, sugar particles, protein nanoparticles and peptide nanoparticles. An example of the combination of nanoparticles and oligonucleotides is spherical nucleic acid (SNA), as in e.g. Barnaby et al. Cancer Treat. Res. 2015, 166, 23.

A preferred composition comprises at least one excipient that may further aid in enhancing the targeting and/or delivery of said composition and/or said oligonucleotide to a tissue and/or a cell and/or into a tissue and/or a cell. A preferred tissue or cell is a muscle tissue or cell.

Many of these excipients are known in the art (e.g. see Bruno, 2011) and may be categorized as a first type of excipient. Examples of first type of excipients include polymers (e.g. polyethyleneimine (PEI), polypropyleneimine (PPI), dextran derivatives, butylcyanoacrylate (PBCA), hexylcyanoacrylate (PHCA), poly(lactic-co-glycolic acid) (PLGA), polyamines (*e.g.* spermine, spermidine, putrescine, cadaverine), chitosan, poly(amido amines) (PAMAM), poly(ester amine), polyvinyl ether, polyvinyl pyrrolidone (PVP), polyethylene glycol (PEG) cyclodextrins, hyaluronic acid, colominic acid, and derivatives thereof), dendrimers (*e.g*. poly(amidoamine)), lipids {*e.g.* 1,2-dioleoyl-3-dimethylammonium propane (DODAP), dioleoyldimethylammonium chloride (DODAC), phosphatidylcholine derivatives [*e.g* 1,2-distearoyl-sn-glycero-3-phosphocholine (DSPC)], lyso-phosphatidylcholine derivaties [*e.g*. 1-stearoyl-2-lyso-sn-glycero-3-phosphocholine (S-LysoPC)], sphingomyeline, 2-{3-[Bis-(3-amino-propyl)-amino]-propylamino}-*N*-ditetracedyl carbamoyl methylacetamide (RPR209120), phosphoglycerol derivatives [*e.g.* 1,2-dipalmitoyl-sn-glycero-3-phosphoglycerol,sodium salt (DPPG-Na), phosphaticid acid derivatives [1,2-distearoyl-sn-glycero-3-phosphaticid acid, sodium salt (DSPA), phosphatidylethanolamine derivatives [*e.g*. dioleoyl-*L*-*R-*phosphatidylethanolamine (DOPE), 1,2-distearoyl-sn-glycero-3-phosphoethanolamine (DSPE),2-diphytanoyl-sn-glycero-3-phosphoethanolamine (DPhyPE),], *N*-[1-(2,3-dioleoyloxy)propyl]-*N,N,*N-trimethylammonium (DOTAP), N-[1-(2,3-dioleyloxy)propyl]-*N,N,N-*trimethylammonium (DOTMA), 1,3-di-oleoyloxy-2-(6-carboxy-spermyl)-propylamid (DOSPER), (1,2-dimyristyolxypropyl-3-dimethylhydroxy ethyl ammonium (DMRIE), (N1-cholesteryloxycarbonyl-3,7-diazanonane-1,9-diamine (CDAN), dimethyldioctadecylammonium bromide (DDAB), 1-palmitoyl-2-oleoyl-sn-glycerol-3-phosphocholine (POPC), (b-L-Arginyl-2,3-L-diaminopropionic acid-*N*-palmityl-*N*-olelyl-amide trihydrochloride (AtuFECT01), N,N-dimethyl-3-aminopropane derivatives [ *e.g*. 1,2-distearoyloxy-*N,N*-dimethyl-3-aminopropane (DSDMA), 1,2-dioleyloxy-*N,N*-dimethyl-3-aminopropane (DoDMA), 1,2-Dilinoleyloxy-*N,N*-3-dimethylaminopropane (DLinDMA), 2,2-dilinoleyl-4-dimethylaminomethyl [1,3]-dioxolane (DLin-K-DMA), phosphatidylserine derivatives [1,2-dioleyl-sn-glycero-3-phospho-L-serine, sodium salt (DOPS)], proteins (*e.g.* albumin, gelatins, atellocollagen), and linear or cyclic peptides (*e.g.* protamine, PepFects, NickFects, polyarginine, polylysine, CADY, MPG, cell-penetrating peptides (CPPs), targeting peptides, cell-translocating peptides, endosomal escape peptides). Examples of such peptides have been described, e.g. muscle targeting peptides (e.g. Jirka et al., Nucl. Acid Ther. 2014, 24, 25), CPPs (e.g. Pip series, including WO2013/030569, and oligoarginine series, e.g. US9,161,948 (Sarepta), WO2016/187425 (Sarepta), and M12 peptide in e.g. Gao et al., Mol. Ther. 2014, 22, 1333), or blood-brain barrier (BBB) crossing peptides such as (branched) ApoE derivatives (Shabanpoor et al., Nucl. Acids Ther. 2017, 27, 130). Carbohydrates and carbohydrate clusters as described below, when used as distinct compounds, are also suitable for use as a first type of excipient.

Another preferred composition may comprise at least one excipient categorized as a second type of excipient. A second type of excipient may comprise or contain a conjugate group as described herein to enhance targeting and/or delivery of the composition and/or of the oligonucleotide of the invention to a tissue and/or cell and/or into a tissue and/or cell, as for example muscle tissue or cell. The conjugate group may display one or more different or identical ligands. Examples of conjugate group ligands are e.g. peptides, vitamins, aptamers, carbohydrates or mixtures of carbohydrates (Han et al., Nature Communications, 2016, doi:10.1038/ncomms10981; Cao et al., Mol. Ther. Nucleic Acids, 2016, doi:10.1038/mtna.2016.46), proteins, small molecules, antibodies, polymers, drugs. Examples of carbohydrate conjugate group ligands are glucose, mannose, galactose, maltose, fructose, *N*-acetylgalactosamine (GaINac), glucosamine, *N*-acetylglucosamine, glucose-6-phosphate, mannose-6-phosphate, and maltotriose. Carbohydrates may be present in plurality, for example as end groups on dendritic or branched linker moieties that link the carbohydrates to the component of the composition. A carbohydrate can also be comprised in a carbohydrate cluster portion, such as a GalNAc cluster portion. A carbohydrate cluster portion can comprise a targeting moiety and, optionally, a conjugate linker. In some embodiments, the carbohydrate cluster portion comprises 1, 2, 3, 4, 5, 6, or more GalNAc groups. As used herein, "carbohydrate cluster" means a compound having one or more carbohydrate residues attached to a scaffold or linker group, (see, e.g., Maier et al., "Synthesis of Antisense Oligonucleotides Conjugated to a Multivalent Carbohydrate Cluster for Cellular Targeting," Bioconjugate Chem., 2003, (14): 18-29; Rensen et al., "Design and Synthesis of Novel N-Acetylgalactosamine-Terminated Glycolipids for Targeting of Lipoproteins to the Hepatic Asiaglycoprotein Receptor," J. Med. Chem. 2004, (47): 5798-5808). In this context, "modified carbohydrate" means any carbohydrate having one or more chemical modifications relative to naturally occurring carbohydrates. As used herein, "carbohydrate derivative" means any compound which may be synthesized using a carbohydrate as a starting material or intermediate. As used herein, "carbohydrate" means a naturally occurring carbohydrate, a modified carbohydrate, or a carbohydrate derivative. Both types of excipients may be combined together into one single composition as identified herein. An example of a trivalent N-acetylglucosamine cluster is described in WO2017/062862 (Wave Life Sciences), which also describes a cluster of sulfonamide small molecules. An example of a single conjugate of the small molecule sertraline has also been described (Ferrés-Coy et al., Mol. Psych. 2016, 21, 328), as well as conjugates of protein-binding small molecules, including ibuprofen (e.g. US 6,656,730 ISIS/lonis Pharmaceuticals), spermine (e.g. Noir et al., J. Am. Chem Soc. 2008, 130, 13500), anisamide (e.g. Nakagawa et al., J. Am. Chem. Soc. 2010, 132, 8848) and folate (e.g. Dohmen et al., Mol. Ther. Nucl. Acids 2012, 1, e7).

Conjugates of oligonucleotides with aptamers are known in the art (e.g. Zhao et al. Biomaterials 2015, 67, 42).

Antibodies and antibody fragments can also be conjugated to an oligonucleotide of the invention. In a preferred embodiment, an antibody or fragment thereof targeting tissues of specific interest, particularly muscle tissue, is conjugated to an oligonucleotide of the invention. Examples of such antibodies and/or fragments are e.g. targeted against CD71 (transferrin receptor), described in e.g. WO2016/179257 (CytoMx) and in Sugo et al. J. Control. Rel. 2016, 237, 1, or against equilibrative nucleoside transporter (ENT), such as the 3E10 antibody, as described in e.g. Weisbart et al., Mol. Cancer Ther. 2012, 11, 1.

Other oligonucleotide conjugates are known to those skilled in the art, and have been reviewed in e.g. Winkler et al., Ther. Deliv. 2013, 4, 791, Manoharan, Antisense Nucl. Acid. Dev. 2004, 12, 103 and Ming et al., Adv. Drug Deliv. Rev. 2015, 87, 81.

The skilled person may select, combine and/or adapt one or more of the above or other alternative excipients and delivery systems to formulate and deliver a compound for use in the present invention.

Such a pharmaceutical composition of the invention may be administered in an effective concentration at set times to an animal, preferably a mammal. More preferred mammal is a human being. An oligonucleotide or a composition as defined herein for use according to the invention may be suitable for direct administration to a cell, tissue and/or an organ *in vivo* of individuals affected by or at risk of developing a disease or condition as identified herein, and may be administered directly *in vivo, ex vivo* or *in vitro.* Administration may be *via* topical, systemic and/or parenteral routes, for example intravenous, subcutaneous, intraperitoneal, intrathecal, intramuscular, ocular, nasal, urogenital, intradermal, dermal, enteral, intravitreal, intracavernous, intracerebral, intrathecal, epidural or oral route.

Preferably, such a pharmaceutical composition of the invention may be encapsulated in the form of an emulsion, suspension, pill, tablet, capsule or soft-gel for oral delivery, or in the form of aerosol or dry powder for delivery to the respiratory tract and lungs.

In an embodiment an oligonucleotide of the invention may be used together with another compound already known to be used for the treatment of said disease. Such other compounds may be used for reducing inflammation, preferably for reducing muscle tissue inflammation, and/or an adjunct compound for improving muscle fiber function, integrity and/or survival and/or improve, increase or restore cardiac function.

Examples are, but not limited to, a steroid, preferably a (gluco)corticosteroid, epicatechin, an ACE inhibitor (preferably perindopril), and HDAC inhibitor, an angiotensin II type 1 receptor blocker (preferably losartan), angiotensin peptide (1-7), a tumor necrosis factor-alpha (TNFα) inhibitor, an NF-kB inhibitor, a TGFβ inhibitor (preferably decorin), human recombinant biglycan, a source of mIGF-1, a myostatin inhibitor, mannose-6-phosphate, an antioxidant, an ion channel inhibitor, dantrolene, a protease inhibitor, a phosphodiesterase inhibitor (preferably a PDE5 inhibitor, such as sildenafil or tadalafil), and/or L-arginine. Such combined use may be a sequential use: each component is administered in a distinct fashion, perhaps as a distinct composition. Alternatively each compound may be used together in a single composition.

Compounds that are comprised in a composition according to the invention can also be provided separately, for example to allow sequential administration of the active components of the composition according to the invention. In such a case, the composition according to the invention is a combination of compounds comprising at least an oligonucleotide according to the invention with or without a conjugated ligand, at least one excipient, and optionally a CAC as described above.

### Use

In a further aspect, there is provided the use of a composition or an oligonucleotide as described in the previous sections for use as a medicament or part of therapy, or applications in which said oligonucleotide exerts its activity intracellularly.

Preferably, an oligonucleotide or composition of the invention is for use as a medicament or part of a therapy for preventing, delaying, curing, ameliorating and/or treating DMD or BMD or SMA.

In an embodiment of this aspect of the invention is provided the oligonucleotide according to the invention, or the composition according to the invention, for use as a medicament, preferably for treating, preventing, and/or delaying Duchenne Muscular Dystrophy (DMD), Becker Muscular Dystrophy (BMD), or Spinal Muscular Atrophy (SMA).

### Method

In a further aspect, there is provided a method for preventing, treating, curing, ameliorating and/or delaying a condition or disease as defined in the previous section in an individual, in a cell, tissue or organ of said individual. The method comprises administering an oligonucleotide or a composition of the invention to said individual or a subject in the need thereof.

The method according to the invention wherein an oligonucleotide or a composition as defined herein may be suitable for administration to a cell, tissue and/or an organ *in vivo* of individuals affected by any of the herein defined diseases or at risk of developing an inflammatory disorder, and may be administered *in vivo, ex vivo* or *in vitro.* An individual or a subject in need is preferably a mammal, more preferably a human being. Alternately, a subject is not a human. Administration may be via topical, systemic and/or parenteral routes, for example intravenous, subcutaneous, nasal, ocular, intraperitoneal, intrathecal, intramuscular, intracavernous, urogenital, intradermal, dermal, enteral, intravitreal, intracerebral, intrathecal, epidural or oral route.

In an embodiment, in a method of the invention, a concentration of an oligonucleotide or composition is ranged from 0.01 nM to 1 µM. More preferably, the concentration used is from 0.05 to 500 nM, or from 0.1 to 500 nM, or from 0.02 to 500 nM, or from 0.05 to 500 nM, even more preferably from 1 to 200 nM.

Dose ranges of an oligonucleotide or composition according to the invention are preferably designed on the basis of rising dose studies in clinical trials (*in vivo* use) for which rigorous protocol requirements exist. An oligonucleotide as defined herein may be used at a dose which is ranged from 0.01 to 200 mg/kg or 0.05 to 100 mg/kg or 0.1 to 50 mg/kg or 0.1 to 20 mg/kg, preferably from 0.5 to 10 mg/kg.

The ranges of concentration or dose of oligonucleotide or composition as given above are preferred concentrations or doses for *in vitro* or *ex vivo* uses. The skilled person will understand that depending on the identity of the oligonucleotide used, the target cell to be treated, the gene target and its expression levels, the medium used and the transfection and incubation conditions, the concentration or dose of oligonucleotide used may further vary and may need to be optimised any further.

In an embodiment of this aspect of the invention is provided a method for preventing, treating, and/or delaying Duchenne Muscular Dystrophy (DMD), Becker Muscular Dystrophy (BMD), or Spinal Muscular Atrophy (SMA), comprising administering to a subject an oligonucleotide according to the invention, or a composition according to the invention.

### Particular embodiments of the invention

1. An oligonucleotide comprising:
   i)
      Ia) at least one 2'-substituted monomer and optionally a phosphorothioate backbone linkage, or
      Ib) only 2'-substituted monomers linked by phosphorothioate backbone linkages and/or by phosphodiester linkages,
   ii) a 5-methylcytosine and/or a 5-methyluracil base and
   iii) at least one monomer comprising a bicyclic nucleic acid (BNA) scaffold modification.
2. An oligonucleotide according to embodiment 1, wherein said oligonucleotide comprises 1, 2, 3, or 4 monomers that comprise a bicyclic nucleic acid (BNA) scaffold modification, preferably a bridged nucleic acid scaffold modification.
3. An oligonucleotide according to embodiment 1 or 2, wherein at least one bicyclic nucleic acid (BNA) scaffold modification is comprised in a terminal monomer of said oligonucleotide, preferably in the 5'-terminal monomer of said oligonucleotide, more preferably in both terminal monomers of said oligonucleotide.
4. An oligonucleotide according to any one of embodiments 1 to 3, wherein each occurrence of said bicyclic nucleic acid (BNA) scaffold modification results in a monomer that is independently chosen from the group consisting of a conformationally restrained nucleotide (CRN) monomer, a locked nucleic acid (LNA) monomer, a xylo-LNA monomer, an α-L-LNA monomer, a β-D-LNA monomer, a 2'-amino-LNA monomer, a 2'-(alkylamino)-LNA monomer, a 2'-(acylamino)-LNA monomer, a 2'-N-substituted-2'-amino-LNA monomer, a (2'-O,4'-C) constrained ethyl (cEt) LNA monomer, a (2'-O,4'-C) constrained methoxyethyl (cMOE) BNA monomer, a 2',4'-BNA^{NC}(N-H) monomer, a 2',4'-BNA^{NC}(N-Me) monomer, an ethylene-bridged nucleic acid (ENA) monomer, a 2'-C-bridged bicyclic nucleotide (CBBN) monomer, and derivatives thereof.
5. An oligonucleotide according to any one of embodiments 1 to 4, wherein said 2'-substituted monomer is a 2'-substituted RNA monomer, a 2'-F monomer, a 2'-amino monomer, a 2'-O-substituted monomer, a 2'-O-methyl monomer, or a 2'-O-(2-methoxyethyl) monomer, preferably a 2'-O-methyl monomer.
6. An oligonucleotide according to any one of embodiments 1 to 5, wherein all cytosine bases are 5-methylcytosine bases, and/or wherein all uracil bases are 5-methyluracil bases.
7. An oligonucleotide according to any one of embodiments 1 to 6, wherein the length of said oligonucleotide is less than 34 nucleotides.
8. An oligonucleotide according to any one of embodiments 1 to 7, wherein said oligonucleotide is complementary to or binds to or targets or hybridizes with at least a part of an exon and/or non-exon region, preferably wherein said oligonucleotide comprises or consists of a sequence which is complementary to or binds or targets or hybridizes at least a part of an exon recognition sequence (ERS), an exonic splicing silencer (ESS), an intronic splicing silencer (ISS), an SR protein binding site, or another splicing element, signal, or structure.
9. An oligonucleotide according to embodiment 8, wherein said at least part of an exon and/or non-exon region has a length of 10 to 33 nucleotides.
10. An oligonucleotide according to embodiment 8 or 9, wherein said exon and/or non-exon region is in a DMD gene or in an SMN gene.
11. An oligonucleotide according to any one of embodiments 1 to 10, wherein said oligonucleotide is represented by a nucleotide sequence comprising or consisting of SEQ ID NO: 8-1580, or by a nucleotide sequence comprising or consisting of a fragment of SEQ ID NO: 8-1580, preferably wherein said oligonucleotide is represented by a nucleotide sequence comprising or consisting of SEQ ID NO: 453, 455, 456, 459, 461, 462, 465, 467, 468, 471, 473, 474, 483, or 486, or by a nucleotide sequence comprising or consisting of a fragment of SEQ ID NO: 453, 455, 456, 459, 461, 462, 465, 467, 468, 471, 473, 474, 483, or 486.
12. An oligonucleotide according to any one of embodiments 1 to 11, wherein said oligonucleotide induces pre-mRNA splicing modulation, preferably said pre-mRNA splicing modulation alters production or composition of protein, which preferably comprises exon skipping or exon inclusion, wherein said RNA modulation most preferably comprises exon skipping.
13. An oligonucleotide according to any one of embodiments 1 to 12, wherein said oligonucleotide induces pre-mRNA splicing modulation, wherein said pre-mRNA splicing modulation alters production of protein that is related to a disease or a condition, preferably wherein said disease or condition is Duchenne Muscular Dystrophy (DMD), Becker Muscular Dystrophy (BMD), or Spinal Muscular Atrophy (SMA).
14. An oligonucleotide according to any one of embodiments 1 to 13, wherein said oligonucleotide has an improved parameter by comparison to a corresponding oligonucleotide that does not comprise a bicyclic nucleic acid (BNA) scaffold modification.
15. A composition comprising an oligonucleotide as defined in any one of embodiments 1 to 14, preferably wherein said composition comprises at least one excipient that may further aid in enhancing the targeting and/or delivery of said composition and/or said oligonucleotide to a tissue and/or cell and/or into a tissue and/or cell.
16. An oligonucleotide according to any one of embodiments 1 to 14, or a composition according to claim 15, for use as a medicament, preferably for treating, preventing, and/or delaying Duchenne Muscular Dystrophy (DMD), Becker Muscular Dystrophy (BMD), or Spinal Muscular Atrophy (SMA).
17. A method for preventing, treating, and/or delaying Duchenne Muscular Dystrophy (DMD), Becker Muscular Dystrophy (BMD), or Spinal Muscular Atrophy (SMA), comprising administering to a subject an oligonucleotide as defined in any one of embodiments 1 to 14, or a composition as defined in claim 15.
18. An oligonucleotide of 10 to 33 nucleotides in length, wherein:
   a) at least one monomer has formula I: 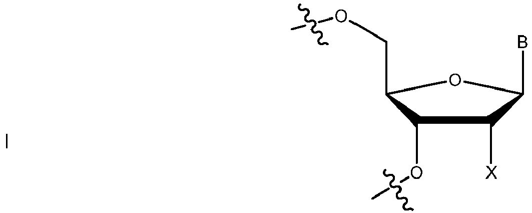 wherein:
      B is a nucleobase;
      X is F, -NR¹R², or-OR;
      R is alkenyl or optionally substituted alkyl, where the optional substituents, when present, are halo, OR¹, NR¹R², or SR¹;
      R¹ is H, alkyl, cycloalkyl, aryl, heterocycloalkyl, or heteroaryl, each independently optionally further substituted with halo, hydroxy, or alkyl;
      R² is H or alkyl; and 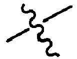
      indicates the point of attachment to the remainder of the oligonucleotide;
   b) wherein at least one monomer comprises a BNA scaffold modification and has formula II: 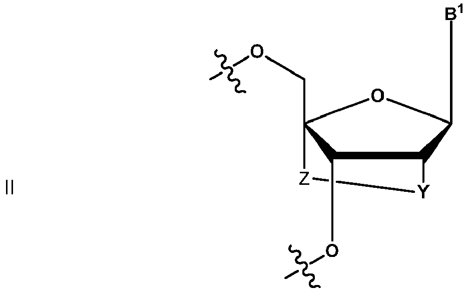 wherein:
      B¹ is a nucleobase;
      Z-Y is a divalent group selected from -(CH₂)ₙO-, -C(CHzCHz)O-, -CH₂WCH₂-, -(CH₂)ₙNR³-, -CH₂S(Oₘ)-, -CH(CH₃)O-, -CH(CH₂OCH₃)O-, -CH₂N(R³)O-, -CH₂CH₂-, -C(O)NR³-, -CH=CHO-, -CH₂SO₂NR³-, and -NHC(O)NH-;
      n is 1 or 2;
      m is 0, 1 or 2;
      W is O, S, or NR³;
      R³ is H, -C(O)R⁴, -C(=NH)NR⁵R⁵, benzyl, or optionally substituted alkyl, where the optional substituents, when present, are selected from halo and alkoxy;
      R⁴ is alkyl, cycloalkyl or aryl;
      R⁵ is H or alkyl; and 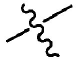
      indicates the point of attachment to the remainder of the oligonucleotide;
   c) wherein the monomers are linked by phosphorothioate backbone linkages and/or by phosphodiester backbone linkages; and
   d) wherein at least one nucleobase in the oligonucleotide is a 5-methylcytosine or a 5-methyluracil base.
19. The oligonucleotide according to embodiment 18, wherein R is unsubstituted alkyl, or -CH₃, or -CH₂CH₃.
20. The oligonucleotide according to embodiment 18 or 19, wherein R¹ is CH₃.
21. The oligonucleotide according to any of embodiments 18-20, wherein X is F or -OR.
22. The oligonucleotide according to any of embodiments 18-21, wherein X is F or -OCH₃.
23. The oligonucleotide of any of embodiments 18-22, wherein Z-Y is a divalent group selected from the group consisting of -(CH₂)ₙO-, -CH(CH₃)O-, and -CH(CH₂OCH₃)O-.
24. The oligonucleotide according to any of embodiments 18-23, wherein Z-Y is -CH₂O-. Preferably, Z is -CH₂- and Y is -O-.
25. The oligonucleotide according to any of embodiments 18-24, wherein the oligonucleotide comprises the sequence GGAAGAUGGCAU (SEQ ID NO: 6072).
26. The oligonucleotide according to any of embodiments 18-25, wherein the oligonucleotide has a length of 16, 17, 18, 19, 20, 21, or 22 nucleotides.
27. The oligonucleotide according to any of embodiments 18-26, wherein the oligonucleotide has a length of 19, 20 or 22 nucleotides.
28. The oligonucleotide according to any of embodiments 18-27, wherein the oligonucleotide has a length of 20 or 22 nucleotides.
29. The oligonucleotide according to any of embodiments 18-28, wherein the oligonucleotide has a length of 20 nucleotides.
30. The oligonucleotide according to any of embodiments 18-28, wherein the oligonucleotide has a length of 22 nucleotides.
31. The oligonucleotide according to any of embodiments 18-27, wherein the oligonucleotide has a length of 19 nucleotides.
30. The oligonucleotide according to any of embodiments 18-27, wherein the oligonucleotide is represented by a sequence comprising or consisting of a sequence selected from SEQ ID NO: 453, 455, 459, 4528, 4531, 4532, 4533, 4535, 4542, 4548, and 4568.
31. The oligonucleotide according to any of embodiments 18-26, wherein the oligonucleotide is represented by a sequence consisting of a sequence selected from SEQ ID NO: 453, 455, 459, 4528, 4531, 4532, 4533, 4535, 4542, 4548, and 4568.
32. The oligonucleotide according to any of embodiments 18-31, wherein said oligonucleotide induces pre-mRNA splicing modulation, preferably said pre-mRNA splicing modulation alters production or composition of protein, which preferably comprises exon skipping or exon inclusion, wherein said RNA modulation most preferably comprises exon skipping.
33. The oligonucleotide according to any of embodiments 18-32, wherein said oligonucleotide induces pre-mRNA splicing modulation, wherein said pre-mRNA splicing modulation alters production of protein that is related to a disease or a condition, preferably wherein said disease or condition is Duchenne Muscular Dystrophy (DMD), Becker Muscular Dystrophy (BMD), or Spinal Muscular Atrophy (SMA).
34. The oligonucleotide according to any of embodiments 18-33, wherein said oligonucleotide induces pre-mRNA splicing modulation, wherein said pre-mRNA splicing modulation alters production of protein that is related to Duchenne Muscular Dystrophy (DMD).
35. The oligonucleotide according to any of embodiments 18-33, wherein said oligonucleotide induces pre-mRNA splicing modulation, wherein said pre-mRNA splicing modulation alters production of protein that is related to Becker Muscular Dystrophy (BMD).
36. The oligonucleotide according to any of embodiments 18-33, wherein said oligonucleotide induces pre-mRNA splicing modulation, wherein said pre-mRNA splicing modulation alters production of protein that is related to Spinal Muscular Atrophy (SMA).
37. The oligonucleotide according to any of embodiments 18-36, wherein said oligonucleotide has an improved parameter by comparison to a corresponding oligonucleotide that does not comprise a bicyclic nucleic acid (BNA) scaffold modification.

### Definitions

In this document and in its claims, the verb "to comprise" and its conjugations is used in its non-limiting sense to mean that items following the word are included, but items not specifically mentioned are not excluded. In addition the verb "to consist" may be replaced by "to consist essentially of" meaning that an oligonucleotide or a composition as defined herein may comprise additional component(s) than the ones specifically identified, said additional component(s) not altering the unique characteristic of the invention. In addition, reference to an element by the indefinite article "a" or "an" does not exclude the possibility that more than one of the element is present, unless the context clearly requires that there be one and only one of the elements. The indefinite article "a" or "an" thus usually means "at least one".

Each embodiment as identified herein may be combined together unless otherwise indicated. All patent and literature references cited in the present specification are hereby incorporated by reference in their entirety.

Throughout the application, the word "binds", "targets", "hybridizes" could be used interchangeably when used in the context of an antisense oligonucleotide which is complementary, preferably reverse complementary, to a part of a pre-mRNA as identified herein. In the context of the invention, "hybridizes" is used under physiological conditions in a cell, preferably a muscular cell unless otherwise indicated.

When a structural formula or chemical name is understood by the skilled person to have chiral centers, yet no chirality is indicated, for each chiral center individual reference is made to all three of either the racemic mixture, the pure R enantiomer, and the pure S enantiomer.

Whenever a parameter of a substance is discussed in the context of this invention, it is assumed that unless otherwise specified, the parameter is determined, measured, or manifested under physiological conditions. Physiological conditions are known to a person skilled in the art, and comprise aqueous solvent systems, atmospheric pressure, pH-values between 6 and 8, a temperature ranging from room temperature to about 37° C (from about 20° C to about 40° C), and a suitable concentration of buffer salts or other components. It is understood that charge is often associated with equilibrium. A moiety that is said to carry or bear a charge is a moiety that will be found in a state where it bears or carries such a charge more often than that it does not bear or carry such a charge. As such, an atom that is indicated in this disclosure to be charged could be non-charged under specific conditions, and a neutral moiety could be charged under specific conditions, as is understood by a person skilled in the art.

Generally, a substitution replaces one moiety, which might be hydrogen, by another moiety. When considering the carbon skeleton of organic molecules, an RNA monomer is inherently 2'-substituted because it has a hydroxyl moiety at its 2'-position. A DNA monomer would therefore not be 2'-substituted, and an RNA monomer can be seen as a 2'-substituted DNA monomer. When an RNA monomer in turn is 2'-substituted, this substitution can have replaced either the 2'-OH or the 2'-H. When an RNA monomer is 2'-O-substituted, this substitution replaces the H of the 2'-OH moiety. As a non-limiting example, 2'-O-methyl RNA is a 2'-substituted monomer (-OMe substitutes -H) and a 2'-substituted RNA monomer (-OMe substitutes -OH) and a 2'-O-substituted RNA monomer (-Me substitutes -H), while 2'-F RNA is a 2'-substituted RNA monomer (-F substitutes -OH or -H) yet not a 2'-O-substituted RNA monomer (2'-O is either no longer present, or is not substituted). 2'-F RNA where F substituted 2'-OH is 2'-F-2'-deoxy RNA, which is also 2'-F DNA.

"Alkenyl" means a straight or branched hydrocarbon radical having from 2 to 8 carbon atoms and at least one double bond. In certain embodiments, alkenyl includes ethenyl, propenyl, 1 but-3-enyl, 1 pent-3-enyl, and 1-hex-5-enyl.

"Alkoxy" means a group of the formula -OR, where R is alkyl. In certain embodiments, alkoxy includes methoxy, ethoxy, propoxy, 2-propoxy, butoxy, tert-butoxy, pentyloxy, and hexyloxy.

"Alkyl" means a straight or branched saturated hydrocarbon radical containing from 1-20 carbon atoms, and in certain embodiments includes 1-6 carbon atoms. In certain embodiments, alkyl includes 1-4 carbon atoms, and in certain embodiments includes 1-3 carbon atoms.₌In certain embodiments, alkyl includes methyl, ethyl, n-propyl, iso-propyl, n-butyl, sec-butyl, tert-butyl, n-pentyl, isopentyl, neopentyl, n-hexyl, 3-methylhexyl, 2,2-dimethylpentyl, 2,3-dimethylhexyl, n-heptyl, n-octyl, n-nonyl, and n-decyl.

"Aryl" means a monovalent six- to fourteen-membered, mono-, bi-, or tri-carbocyclic ring, wherein the monocyclic ring is aromatic and at least one of the rings in the bicyclic or tricyclic ring is aromatic. In certain embodiments, aryl includes phenyl, naphthyl, indanyl, and anthracenyl.

"Cycloalkyl" means a monocyclic or bicyclic, saturated or partially unsaturated (but not aromatic), hydrocarbon radical of three to ten carbon ring atoms. In certain embodiments, the cycloalkyl group contains from 5-6 carbon atoms, it may be referred to herein as C₅₋₆ cycloalkyl. Cycloalkyl groups include fused, bridged and spirocycloalkyl bicyclic rings. For example, when fused, the cycloalkyl group may comprise two rings that share adjacent atoms (e.g., one covalent bond). When bridged, the cycloalkyl group may comprise two rings that share three or more atoms, separating the two bridgehead atoms by a bridge containing at least one atom. When spiro, the cycloalkyl group may comprise two rings that share only one single atom, the spiro atom, which may be, for example, a quaternary carbon. In certain embodiments, cycloalkyl groups include cyclopropyl, cyclobutyl, cyclopentyl, and cyclohexyl. In certain embodiments, cycloalkyl groups include:

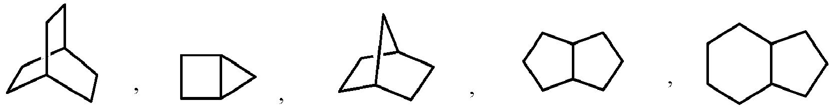

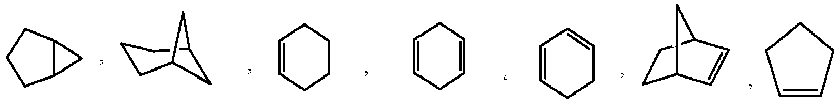

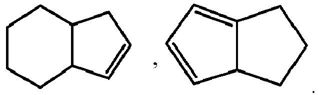

"Halo" means a fluoro, chloro, bromo, or iodo group. "Halo" preferably means fluoro. "Halo" can be replaced by a halogen. Preferred halogens are fluorine, chlorine, bromine, or iodine. A most preferred halogen is fluorine.

"Heteroaryl" means monocyclic, fused bicyclic, or fused tricyclic, radical of 5 to 14 ring atoms containing one or more, in another example one, two, three, or four ring heteroatoms independently selected from -O-, -S(O)ₙ- (n is 0, 1, or 2), -N= (trivalent nitrogen), -N(H)-, and >N-oxide, and the remaining ring atoms being carbon, wherein the ring comprising a monocyclic radical is aromatic and wherein at least one of the fused rings comprising a bicyclic or tricyclic radical is aromatic (but does not have to be a ring which contains a heteroatom, e.g. 2,3-dihydrobenzo[b][1,4]dioxin-6-yl). Fused bicyclic radical includes bridged ring systems. Unless stated otherwise, the valency may be located on any atom of any ring of the heteroaryl group, valency rules permitting.

In certain embodiments, heteroaryl includes, but is not limited to, triazolyl, tetrazolyl, pyrrolyl, imidazolyl, thienyl, furanyl, pyrazolyl, oxazolyl, isoxazolyl, oxadiazolyl, thiadiazolyl, indolyl, indazolyl, phthalimidyl, benzimidazolyl, benzoxazolyl, benzofuranyl, benzothienyl, benzopyranyl, benzothiazolyl, pyridinyl, pyrazinyl, pyrimidinyl, pyridazinyl, quinolinyl, isoquinolinyl, and tetrahydroisoquinolinyl.

"Heterocycloalkyl" means a saturated or partially unsaturated (but not aromatic) monovalent monocyclic group of 3 to 9 ring atoms or a saturated or partially unsaturated (but not aromatic) monovalent bicyclic group of 5 to 12 ring atoms in which one or more ring atoms is a heteroatom independently selected from -O-, -S(O)ₙ- (n is 0, 1, or 2), -N= (trivalent nitrogen), or -NH-, and the remaining ring atoms are carbon. Heterocycloalkyl groups include fused, bridged and spiro heterocycloalkyl bicyclic rings. For example, when fused, the heterocycloalkyl group may comprise two rings that share adjacent atoms (e.g., one covalent bond). When bridged, the heterocycloalkyl group may comprise two rings that share three or more atoms, separating the two bridgehead atoms by a bridge containing at least one atom. When spiro, the heterocycloalkyl group may comprise two rings that share only one single atom, the spiro atom, which may be, for example, a quaternary carbon. In certain embodiments, the heterocycloalkyl group comprises one, two, three, or four ring heteroatoms, independently selected from -O-, -S(O)ₙ- (n is 0, 1, or 2), -N= (trivalent nitrogen), or -NH-.

In certain embodiments, the heterocycloalkyl group contains from 5 or 6 ring atoms. In certain embodiments, heterocycloalkyl includes, but is not limited to, azetidinyl, pyrrolidinyl, piperidinyl, morpholinyl, piperazinyl, pyranyl, tetrahydropyranyl, tetrahydrothiopyranyl, dioxinyl, thiomorpholinyl, pyrazolidinyl, imidazolinyl, imidazolidinyl, oxazolinyl, oxazolidinyl, isoxazolidinyl, thiazolinyl, thiazolidinyl, and tetrahydrofuryl.

In the context of this invention, a decrease or increase of a parameter to be assessed means a change of at least 5% of the value corresponding to that parameter. More preferably, a decrease or increase of the value means a change of at least 10%, even more preferably at least 20%, at least 30%, at least 40%, at least 50%, at least 70%, at least 90%, or 100%. In this latter case, it can be the case that there is no longer a detectable value associated with the parameter.

The use of a substance as a medicament as described in this document can also be interpreted as the use of said substance in the manufacture of a medicament. Similarly, whenever a substance is used for treatment or as a medicament, it can also be used for the manufacture of a medicament for treatment.

The word "about" or "approximately" when used in association with a numerical value (e.g. about 10) preferably means that the value may be the given value (of 10) more or less 0.1% of the value.

Compounds or compositions according to this invention are preferably for use in methods or uses according to this invention.

As will be understood by a skilled person, throughout this application, the terms "BNA", "BNA scaffold", "BNA nucleotide", "BNA nucleoside", "BNA modification", or "BNA scaffold modification" may be replaced by conformationally restricted scaffold modification, locked scaffold modification, locked nucleotide, locked nucleoside, locked monomer, or Tm enhancing scaffold modification, or high-affinity modification and the like, as appropriately.

The following examples are offered for illustrative purposes only, and are not intended to limit the scope of the present invention in any way.

### Legends to the figures

**Figure 1****.**
   The effect of implementation of a 5' and/or 3' BNA scaffold-modified nucleotide on AON-induced DMD exon 51 skipping (**A**) and dystrophin production (**B**) in a DMD patient (del. exon 48-50) muscle cell culture, compared to an iso-sequential AON without BNA modification based on SEQ ID NO:452. The BNA modification in this case is LNA (SEQ ID NOs shown in the figure relate to SEQ ID NOs where the BNA scaffold modifications as indicated result in an LNA monomer). (**A**): Average exon 51 skipping percentages were determined by RT-ddPCR analysis of triplicate RNA samples, error bars indicate standard deviation, AON (800 nM or 4 µM). (**B**): Average chemiluminescence values (area under curve (AUC) from electropherogram) were measured by Simple Western Capillary Immunoassay; high to low protein concentrations were loaded HC= healthy control muscle sample, NT= non-treated sample, AON concentration 800 nM.
**Figure 2****.**
   The effect of implementation of a 5' and/or 3' BNA scaffold-modified nucleotide on AON-induced DMD exon 51 skipping in a DMD patient (del. exon 48-50) muscle cell culture, compared to an iso-sequential AON without BNA modification based on SEQ ID NO:452. The BNA modification in **A** is CRN (SEQ ID NOs: 453C and 455C shown in the figure relate to SEQ ID NO: 453 and 455 where the BNA scaffold modifications as indicated result in a CRN monomer). The BNA modification in **B** is 2'-amino-2'-deoxy LNA, which in this application is referred to as 2'-amino-LNA (SEQ ID NO: 456A shown in the figure relate to SEQ ID NO: 456 where the BNA scaffold modification as indicated result in a 2'-amino-LNA monomer). Average exon 51 skipping percentages were determined by RT-ddPCR analysis of triplicate RNA samples, error bars indicate standard deviation, AON concentration 800 nM (B) or 4 µM (A,B).
**Figure 3****.**
   The effect of implementation of a 5' and/or 3' BNA scaffold-modified nucleotide on AON-induced DMD exon 51 skipping in the hDMD mouse model after 12 wks of IV treatment (100 mg/kg AON weekly). The BNA modification in this case is LNA (SEQ ID NOs shown in the figure relate to SEQ ID NOs where the BNA scaffold modifications as indicated result in an LNA monomer). Average exon 51 skipping percentages were determined by RT-ddPCR analysis of muscle RNA samples.
**Figure 4****.**
   The effect of implementation of at least one BNA scaffold-modified nucleotide on AON-induced DMD exon 51 skipping in a DMD patient (del. exon 48-50) muscle cell culture, compared to an iso-sequential AON without BNA modification based on SEQ ID NO: 452 (all at 800 nM). The BNA modification in this case results in LNA (SEQ ID NOs shown in the figure relate to SEQ ID NOs where the BNA scaffold modifications as indicated result in an LNA monomer). Average fold increase of exon 51 skipping levels over SEQ ID NO: 452 are based on RT-ddPCR analysis of triplicate RNA samples.
**Figure 5****.**
   The effect of implementation of a 5' and 3' BNA scaffold-modified nucleotide on AON-induced DMD exon 44 skipping (A, SEQ ID NO: 29), exon 45 skipping (B, SEQ ID NO: 3185), and exon 53 skipping (C, SEQ ID NO: 863) in healthy human muscle cells, compared to iso-sequential AONs without BNA scaffold modifications (SEQ ID NO: 26 (exon 44), SEQ ID NO: 6049 (exon 45), and SEQ ID NO: 860 (exon 53). The BNA modification in this case results in LNA (SEQ ID NOs shown in the figure relate to SEQ ID NOs where the BNA scaffold modifications as indicated result in an LNA monomer). The average exon skipping percentages were determined by RT-ddPCR analysis of RNA samples (n=6), error bars indicate standard deviation, AON (800 nM or 4 µM).

### Examples

### EXAMPLE 1 (IN VITRO)

### Material and Methods

### AONs

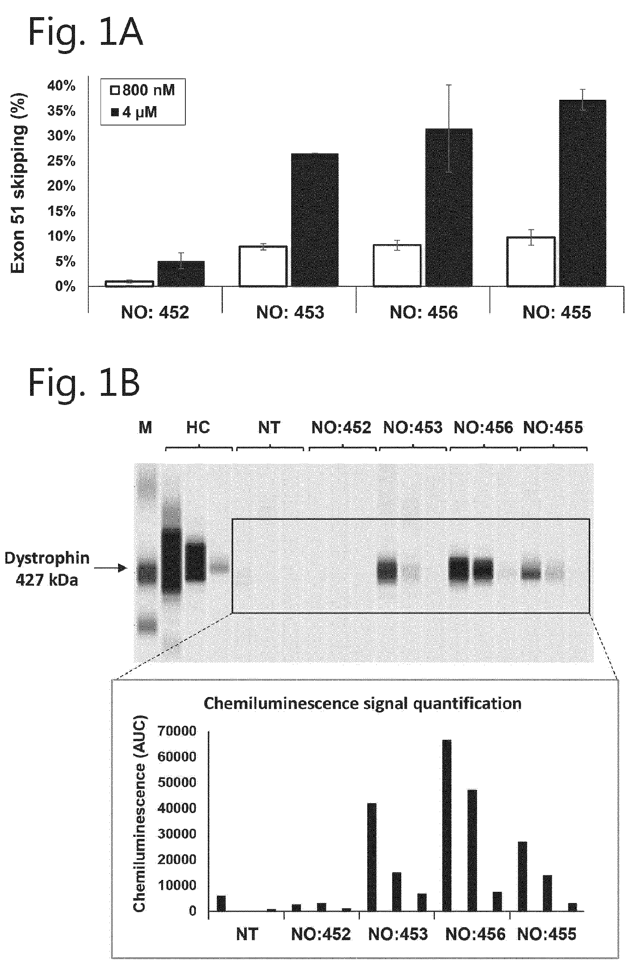

Antisense oligonucleotides (AONs) (Table 1, Figure 1-3) had a phosphorothioate backbone with 2'-O-methyl monomers and either LNA (SEQ ID NO:452, 453, 455, and 456), or 2'-amino-LNA (SEQ ID NO:456A), or CRN (SEQ ID NO:453C and 455C) scaffold modification. The AON with SEQ ID NO:452 featured cytosines, the other SEQ ID NOs featured 5-methylcytosines. 2'-amino-LNA refers to the scaffold modification that is also sometimes named 2'-amino-2'-deoxy LNA The AONs were synthesized in 10 µmol scale using an OP-10 synthesizer (GE/ÄKTA Oligopilot), through standard phosphoramidite protocols. The AONs were cleaved and deprotected in a two-step sequence (diethylamine followed by conc. NH₄OH treatment), purified by HPLC and dissolved in water and an excess of NaCl was added to exchange ions. After evaporation, the AONs were redissolved in water, desalted by FPLC and lyophilized. Mass spectrometry confirmed the identity of all AONs, and purity (determined by UPLC) was found acceptable for all AONs (>80%).

**Table 1**

| **AON ID** | **Chemical Modification** | **Sequence** | **Length** | **SEQ ID NO** |
|---|---|---|---|---|
| NO:452 | 20Me/PS | UCAAGGAAGAUGGCAUUUCU | 20 | 452 |
| NO:453(C) | 2OMe/BNA/PS | T*C*AAGGAAGAUGGCAUUU*C*U | 20 | 453 |
| NO:455(C) | 2OMe/BNA/PS | T*C*AAGGAAGAUGGCAUUU*C*T | 20 | 455 |
| NO:456 (A) | 2OMe/BNA/PS | T*C*AAGGAAGAUGGCAUUU*C*U | 20 | 456 |

A=adenosine, G=guanine, U=uracil, T=thymine, C=cytosine, C=5-methylcytosine, and T=BNA nucleotide; 2OMe details that the AON comprises 2'-O-methyl substitutions in all non-BNA monomers; PS details that the AON exclusively comprises phosphorothioate backbone linkages

### Gymnotic uptake and cDNA synthesis

Immortalized myoblasts, derived from a DMD patient with a deletion of exons 48-50 (Δ48-50), were cultured to confluency in 6-wells plates. To induce the formation of myotubes, proliferation medium was replaced by low-serum differentiation medium for 5 days, supplemented with 800 nM or 4 µM of AON (in triplicate) according to non-GLP standard operating procedures. Total RNA was then isolated and 1000ng of RNA was used as input for the cDNA synthesis using random hexamer primers.

### Digital droplet (dd)PCR analysis

Specific Taqman minor groove binder (MGB) assays were designed to detect the dystrophin transcript products with and without exon 51 (Table 2) and were purchased from Applied Biosystems. Digital droplet PCR analysis was performed on 1µl (for non-skipped transcript) or 4µl (for skipped transcript) of cDNA in a 20µl reaction volume using an annealing/extension temperature of 60° C according to the manufacturer's instructions (BioRad). Data was presented as percentage exon skip [N₀ skipped/(N₀ skipped + N₀ non-skipped)*100].

**Table 2**

| **Assay** | **Target Exons** | | **Sequences** | **Amplicon length** | **SEQ ID NO** |
|---|---|---|---|---|---|
| | | **Forward primer** | TGAAAATAAGCTCA AGCAGACAAATC GACGCCTCTGTTC CAAATCC CAGTGGATAAAGG CAACA GTGATGGTGGGTG ACCTTGAG GACGCCTCTGTTC CAAATCC CAAGCAGAAGGCA ACAA | | 1586 |
| DMD_47-52 | 47/52 | **Reverse primer** | | 71 | 1587 |
| | | **Probe** | | | 1588 |
| | | **Forward primer** | | | 1589 |
| DMD_51-52.2 | 51/52 | **Reverse primer** | | 82 | 1590 |
| | | **Probe sequence** | | | 1591 |

### Simple Western Capillary Immunoassay

Total protein was extracted in protein loading buffer (6% 1.25 M Tris-HCl pH 6.8, 20% glycerol, 15% SDS, 0.0016% bromophenol blue, 5% β-mercaptoethanol (all Sigma) with protease inhibitor (Roche)). Protein concentration was measured using a Compat-Able Protein Assay Preparation Reagent Set (Thermo scientific) and a Pierce BCA Protein Assay Kit (Thermo scientific). For the healthy human control cell sample 50, 5 or 0.5 µg/mL was applied, for the DMD patient cell samples 100, 50, or 10 µg/mL. Dystrophin protein levels were quantified using Simple Western Capillary Immunoassay (Protein Simple) and WES 66-440 kDa Rabbit Master Kit (Protein Simple cat. # PSMK20) according to manufacturer's protocols. The WES plate was loaded with biotinylated ladder, samples, primary rabbit polyclonal anti-dystrophin antibody (Abcam, cat.# ab15277, 1:50 diluted in supplied antibody diluent), streptavidin-HRP for ladder detection, secondary anti-rabbit antibody, luminol-peroxide mix and wash buffer (all supplied in the WES master kit). The plate was centrifuged for 5 minutes at 2500 rpm at room temperature and loaded in the WES equipment together with the corresponding capillary cartridge. After running the assay, data was analyzed using Compass software.

### Results

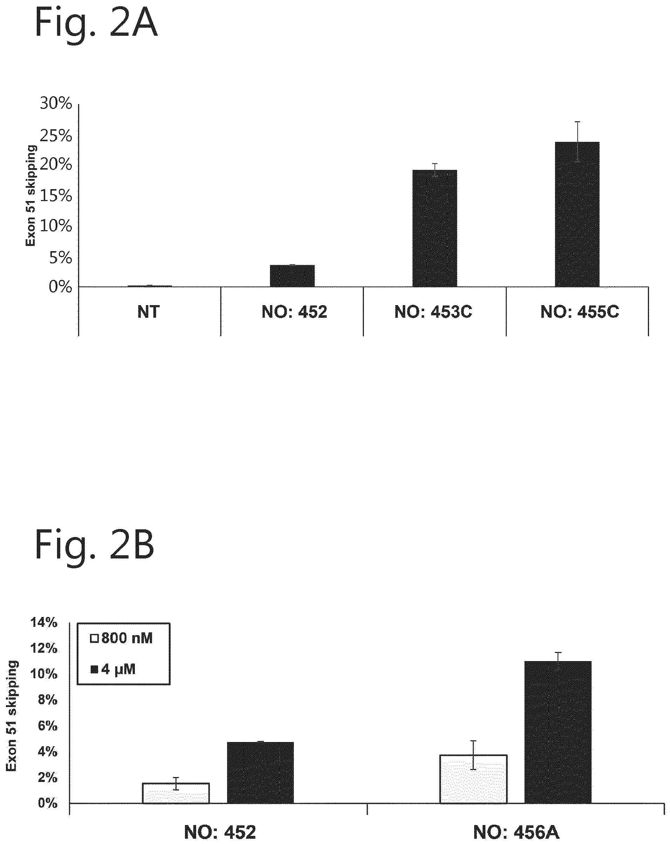

Implementation of at least one 5' and/or 3' BNA scaffold-modified nucleotide in an AON improves exon 51 skipping levels when compared to an iso-sequential AON without BNA scaffold-modified nucleotide (based on SEQ ID NO:452). **Figure 1A** shows this effect for three AONs (SEQ ID NO: 453, NO:455, and NO:456) at low (800 nM) and high (4 µM) concentration in DMD patient muscle cells *in vitro.* The BNA scaffold modification in this case is LNA. Compared to the AON without BNA scaffold modifications based on SEQ ID NO:452, the AONs with LNA nucleotides induced 4- to 8-fold higher exon 51 skipping levels. This was associated with an even higher improvement in dystrophin levels (up to 20-fold with SEQ ID NO:456) (**Figure 1B**). A similar effect was obtained with implementation of a 5' and/or 3' CRN scaffold-modified (SEQ ID NO: 453C and 455C) or 2'-amino-LNA scaffold-modified (SEQ ID NO: 456A) nucleotide when compared to the AON without BNA scaffold modifications based on SEQ ID NO:452 at 800 nM and/or 4 µM (**Figure 2A****, B**).

### EXAMPLE 2 (IN VIVO)

### Material and Methods

### AONs

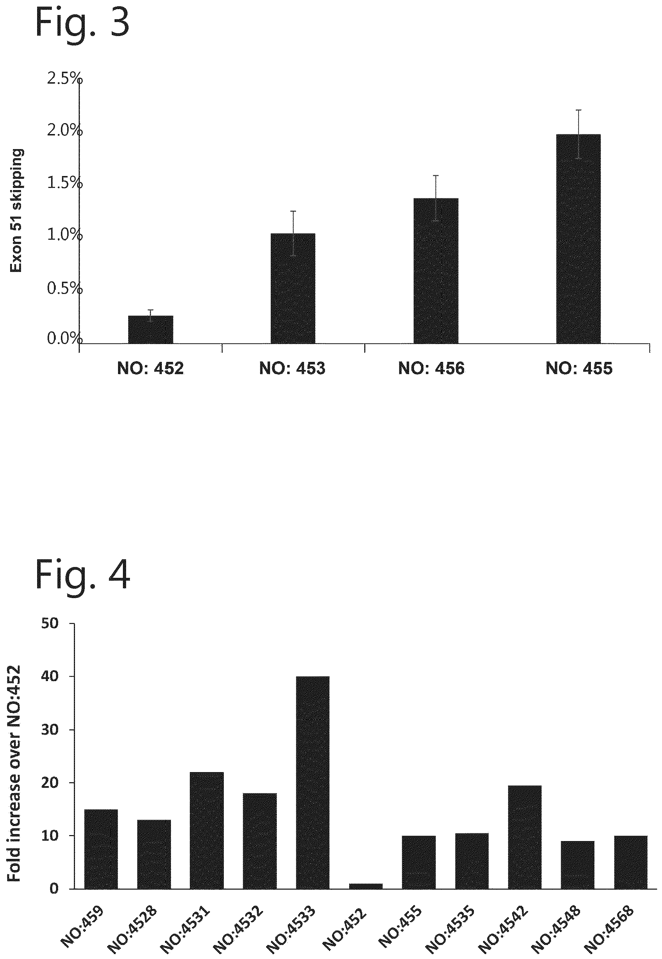

Antisense oligonucleotides (AONs) (Table 1, Figure 3) had a full phosphorothioate backbone with 2'-O-methyl substitutions in all non-BNA monomers, 5-methylcytosines and BNA scaffold modifications resulting in LNA monomers (SEQ ID NOs: 453, 455, and 456). The control AON with SEQ ID NO: 452 had a phosphorothioate backbone with 2'-O-methyl monomers, cytosines, and no BNA scaffold modifications. The AONs were synthesized on a 1 mmol scale using an OP-100 synthesizer (GE/ÄKTA Oligopilot), through standard phosphoramidite protocols. The AONs were cleaved and deprotected in a two-step sequence (diethylamine followed by conc. NH₄OH treatment), purified by anion-exchange chromatography, desalted by ultrafiltration/diafiltration, and lyophilized. Mass spectrometry confirmed the identity of all AONs, and purity (determined by UPLC) was found acceptable for all AONs (>85%).

### Mouse experiment

This mouse experiment was carried out according to the National Institute of Health (NIH) guidelines for the care and use of laboratory animals. hDMD mice were bred and genotyped by JAX Labs (USA). Mice were randomized into groups (n=15) taking into account baseline weight and male-female distribution. Mice received 1x weekly an intravenous tail vein injection with 100 mg/kg of each of the AONs with SEQ ID NO: 452, 453, 455, or 456, and starting at 5-6 weeks of age for a total of 12 weeks (SEQ ID NO: 452 does not comprise any BNA scaffold modifications in this case). Four days after the last AON injection the animals were sacrificed and tissue samples collected (after transcardial perfusion with PBS in order to remove blood from the tissues). Muscle tissues samples were snap frozen and stored at -80° C.

### RNA isolation and cDNA synthesis

Tissues were homogenized in 1 ml RNA-Bee (Bio-Connect) by grinding in a MagNa Lyser using MagNA Lyser Green Beads (Roche). Total RNA was extracted from the homogenate based on the manufacturer's instructions. For cDNA synthesis 1000ng of total RNA was used as input. cDNA was generated in 20µl reactions using random hexamer primers and Transcriptor Reverse transcriptase according to the manufacturer's instructions (Roche), with the exception that incubation took place for 40 minutes at 50° C instead of 30 minutes at 55° C.

### Digital droplet PCR analysis

Specific Taqman minor groove binder (MGB) assays were designed (using Primer Express 3.0.1 software; Applied Biosystems) to detect the dystrophin transcript products with and without exon 51 (Table 2) and purchased from Applied Biosystems. Digital droplet PCR analysis was performed on 2 or 4 µl of cDNA in a 20µl reaction volume using an annealing/extension temperature of 60° C according to the manufacturer's instructions (BioRad). Data was presented as percentage exon skip [N₀ skipped/(N₀ skipped + N₀ non-skipped)*100].

### Results

Transgenic hDMD mice expressing full length human dystrophin allow *in vivo* screening of human-specific AONs in a mouse experimental background. Note that this model is not dystrophin-deficient and has no muscle pathology. Therefore the uptake of AONs by muscle tissue is typically lower than that in the *mdx* mouse model. In this experiment, three AONs with 5' and/or 3' BNA-scaffold modified nucleotides (SEQ ID NO: 453, 455, and 456, using LNA scaffold modifications) were compared to the iso-sequential AON without BNA scaffold modified nucleotides (SEQ ID NO:452) in a 12 wks systemic (IV) hDMD study. **Figure 3** shows improved *in vivo* exon 51 skipping levels for all LNA-containing AONs, up to 8-fold with AON of SEQ ID NO:455 when compared to AON of SEQ ID NO:452.

### EXAMPLE 3 (IN VITRO)

### AONs

The antisense oligonucleotides (AONs) of the invention (Table 3, Figure 4) comprised a full phosphorothioate backbone with 2'-O-methyl substitutions in all non-BNA monomers, 5-methylcytosines, and at least one BNA scaffold modification resulting in an LNA monomer (SEQ ID NOs: 455, 459, 4528, 4531, 4532, 4533, 4535, 4542, 4548, and 4568). The control AON with SEQ ID NO: 452 comprised a phosphorothioate backbone with 2'-O-methyl monomers, cytosines and no BNA scaffold modifications. The AONs were synthesized in 5 µmol scale using an OP-10 synthesizer (GE/ÄKTA Oligopilot), through standard phosphoramidite protocols. The AONs were cleaved and deprotected in a two-step sequence (DEA followed by conc. NH₄OH treatment), purified by anion-exchange chromatography, desalted by size exclusion chromatography and lyophilized. Mass spectrometry confirmed the identity of all AONs, and purity (determined by UPLC) was found acceptable for all AONs (>80%).

**Table 3**

| **SEQ ID NO:** | **Chemical Modification** | **Sequence** |
|---|---|---|
| 452 | 2OMe/PS | UCAAGGAAGAUGGCAUUUCU |
| 455 | 2OMe/PS/LNA | T*C*AAGGAAGAUGG*C*AUUU*C*T |
| 459 | 2OMe/PS/LNA | T*C*AAGGAAGAUGG*C*AUUU*C*UAG |
| 4528 | 2OMe/PS/LNA | T*C*AAGGAAGAUGG*C*AUUU*C*UAG |
| 4531 | 2OMe/PS/LNA | T*C*AAGGAAGAUGG*C*AUUU*C*UAG |
| 4532 | 2OMe/PS/LNA | TCAAGGAAGAUGG*C*AUUU*C*UAG |
| 4533 | 2OMe/PS/LNA | T*C*AAGGAAGAUGG*C*AUUU*C*UAG |
| 4535 | 2OMe/PS/LNA | T*C*AAGGAAGAUGGCAUUUCT |
| 4542 | 2OMe/PS/LNA | T*C*AAGGAAGAUGGCAUUUCT |
| 4548 | 2OMe/PS/LNA | *C*AAGGAAGAUGGCAUUUCT |
| 4568 | 2OMe/PS/LNA | GGUAAGUUCUGU*CC*AAG*C* |

A=adenosine, G=guanine, U=uracil, T=thymine, C=cytosine, C=5-methylcytosine, and T=LNA nucleotide; 2OMe details that the AON comprises 2'-O-methyl substitutions in all non-BNA monomers; PS details that the AON exclusively comprises phosphorothioate backbone linkages

### Gymnotic uptake and cDNA synthesis

Immortalized myoblasts, derived from a DMD patient with a deletion of exons 48-50 (Δ48-50), were cultured to confluency in 6-wells plates. To induce the formation of myotubes, proliferation medium was replaced by low-serum differentiation medium for 7 days, supplemented with 800 nM AON (in triplicate) according to non-GLP standard operating procedures. Total RNA was then isolated and 1000ng of RNA was used as input for the cDNA synthesis using random hexamer primers.

### Digital droplet (dd)PCR analysis

Specific Taqman minor groove binder (MGB) assays were designed to detect the dystrophin transcript products with and without exon 51 (Table 2) and were purchased from Applied Biosystems. Digital droplet PCR analysis was performed on 1µl (for transcript without exon skip) or 4µl (for transcript with exon skip) of cDNA in a 20µl reaction volume using an annealing/extension temperature of 60° C according to the manufacturer's instructions (BioRad). Data was presented as percentage exon skip [N₀ skipped/(N₀ skipped + N₀ non-skipped)*100].

### Results

Implementation of at least one BNA scaffold-modified nucleotide (resulting in an LNA monomer) in an AON improves exon 51 skipping levels when compared to an iso-sequential AON without a BNA scaffold modification (based on SEQ ID NO: 452). Figure 4 shows this effect for ten AONs (SEQ ID NOs: 455, 459, 4528, 4531, 4532, 4533, 4535, 4542, 4548, and 4568) at 800 nM concentration in DMD patient muscle cells in vitro. Compared to the AON without a BNA scaffold modification (SEQ ID NO: 452), the AONs with LNA nucleotides induced 10- to 40-fold higher exon 51 skipping levels.

### EXAMPLE 4 (IN VITRO)

### Material and Methods

### AONs

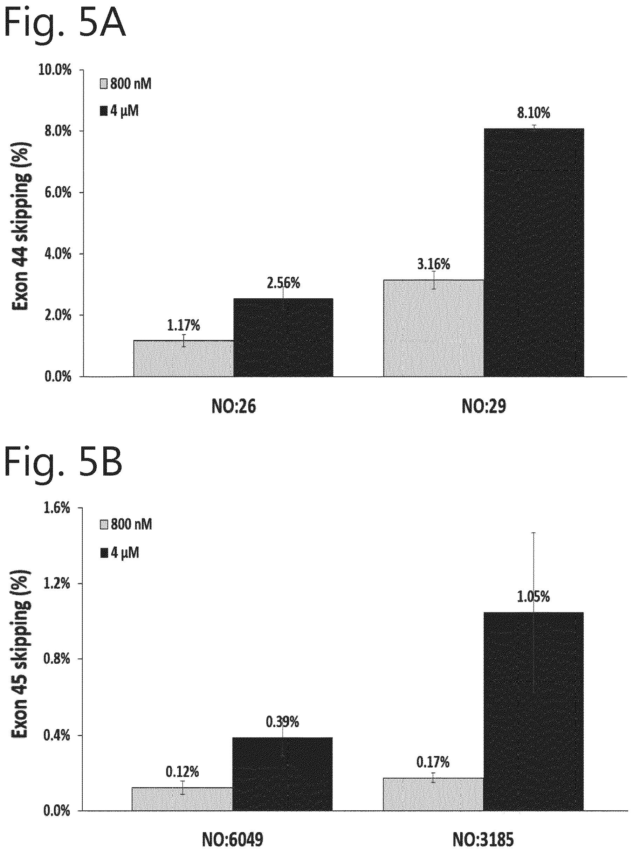

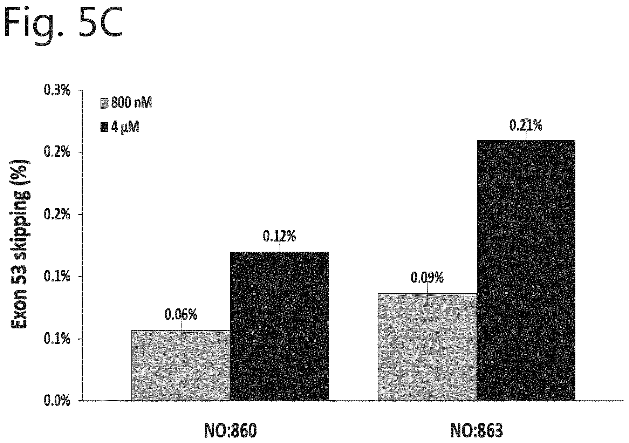

The antisense oligonucleotides (AONs) of the invention (Table 4, Figure 5) comprised a full phosphorothioate backbone with 2'-O-methyl substitutions, 5-methylcytosines, and at least one BNA scaffold modification resulting in an LNA monomer (SEQ ID NOs: 29, 3185, and 863). The control AONs comprised a full phosphorothioate backbone with only 2'-O-methyl substituted monomers, 5-methylcytosines, but no BNA scaffold modification (SEQ ID NO: 26, 6049, and 860; for SEQ ID NO: 6049, these modifications make it identical to SEQ ID NO: 6071). The AONs were synthesized in 5 µmol scale using an OP-10 synthesizer (GE/ÄKTA Oligopilot), through standard phosphoramidite protocols. The AONs were cleaved and deprotected in a two-step sequence (DEA followed by conc. NH₄OH treatment), purified by anion-exchange chromatography, desalted by size exclusion chromatography and lyophilized. Mass spectrometry confirmed the identity of all AONs, and purity (determined by UPLC) was found acceptable for all AONs (>80%).

**Table 4**

| **SEQ ID NO:** | **Chemical Modification** | **Sequence** |
|---|---|---|
| 26 | 2OMe/PS | U*C*AG*C*UU*C*UGUUAG*CC*A*C*UG |
| 29 | 2OMe/PS/LNA | TCAG*C*UUCUGUUAG*CC*ACUG |
| 6049 | 2OMe/PS | UUUG*CC*G*C*UG*CCC*AAUG*CC*AU*CC*UG |
| 3185 | 2OMe/PS/LNA | TUUGCCGCUG*CCC*AAUG*CC*AU*CC*UG |
| 860 | 2OMe/PS | GUUG*CC*U*CC*GGUU*C*UGAAGGUGUUC |
| 863 | 2OMe/PS/LNA | GUUG*CC*U*CC*GGUU*C*UGAAGGUGUU*C* |

A=adenosine, G=guanine, U=uracil, T=thymine, C=5-methylcytosine, and T=LNA nucleotide; 2OMe details that the AON comprises 2'-O-methyl substitutions in all non-BNA monomers; PS details that the AON exclusively comprises phosphorothioate backbone linkages

### Gymnotic uptake and cDNA synthesis

Immortalized myoblasts, derived from a healthy donor, were cultured to confluency in 12-wells plates. To induce the formation of myotubes, proliferation medium was replaced by low-serum differentiation medium for 7 days, supplemented with 800 nM or 4 µM of AON (n=6) according to non-GLP standard operating procedures. Total RNA was then isolated and 1000ng of RNA was used as input for the cDNA synthesis using random hexamer primers.

### Digital droplet (dd)PCR analysis

Specific Taqman minor groove binder (MGB) assays were designed to detect the dystrophin transcript products with and without exon 44, 45, or 53 (Table 5) and were purchased from Applied Biosystems. Digital droplet PCR analysis was performed on 1µl (for transcript without exon skip) or 4µl (for transcript with exon skip) of cDNA in a 20µl reaction volume using an annealing/extension temperature of 60° C according to the manufacturer's instructions (BioRad). Data was presented as percentage exon skip [N₀ skipped/(N₀ skipped + N₀ non-skipped)*100].

**Table 5**

| **Assay** | | **Primer/Probe Sequences (5'-3')** | **Amplicon length** | **SEQ ID NO:** |
|---|---|---|---|---|
| DMD 43-45 | **Forward** | CCCAGCTTGATTTCCAATGG | | 6050 |
| | **Reverse** | GCCGCTGCCCAATGC | 83 | 6051 |
| | **Probe** | ACCGACAAGGGAACTC | | 6052 |
| DMD 44-45 | **Forward** | TGGGAACATGCTAAATACAAATGG | | 6053 |
| | **Reverse** | TGCCGCTGCCCAATG | 62 | 6054 |
| | **Probe** | ATCTTAAGGAACTCCAGGATG | | 6055 |
| DMD 44-46.2 | **Forward** | GAGAATTGGGAACATGCTAAATAC AA | | 6056 |
| | **Reverse** | CCTCCAACCATAAAACAAATTCATT | 110 | 6057 |
| | **Probe** | TATCTTAAGGCTAGAAGAACAA | | 6058 |
| DMD 52-54 | **Forward** | CCAGCAATCAAGAGGCTAGAACA | | 6059 |
| | **Reverse** | TCATTTGCCACATCTACATTTGTCT | 92 | 6060 |
| | **Probe** | ATTACGGATCGAACAGTTG | | 6061 |
| DMD 53-54 | **Forward** | GTCCCTATACAGTAGATGCAATCC AA | | 6062 |
| | **Reverse** | GCCACTGGCGGAGGTCTT | 75 | 6063 |
| | **Probe** | ACAGAAACCAAGCAGTTG | | 6064 |

### Results

Implementation of BNA scaffold modifications resulting in LNA monomers at both the 5' and 3' end of AONs targeting DMD exon 44, exon 45, or exon 53, improved exon skipping levels in healthy human control myotubes *in vitro* 2- to 3-fold when compared to iso-sequential AONs without BNA scaffold-modifications. **Figure 5** shows this effect for three AONs (SEQ ID NO: 29 (targeting exon 44), SEQ ID NO: 3185 (targeting exon45), and SEQ ID NO:863 (targeting exon 53), at low (800 nM) and high (4 µM) concentrations. Note that exon skip levels in healthy human myotubes are typically lower than those obtained in DMD patient muscle cells (as used in Example 1). This is explained by the nonsense-mediated decay of out-of-frame transcripts resulting from AON-induced exon skipping in healthy muscle cells.

### List of references

Dominski and Kole, PNAS 1993, 90(18):8673-8677
Friedman et al., J Biol Chem 1999, 274(51):36193-36199
Uchikawa et al., J Hum Genet 2007, 52(11):891-897
Williams et al., Oligonucleotides 2006, 16(2):186-95
Vickers et al., J Immunol 2006, 176(6):3652-61
Karras et al., Biochemistry 2001, 40(26):7853-9
Vetrini et al., Hum Mutat 2006, 27(5):420-6
Du et al., PNAS 2007, 104(14):6007-12
Rincon et al., Am J Hum Genet 2007, 81(6):1262-1270
Tyson-Capper et al., Mol Pharmacol 2006, 69(3):796-804
Khoo et al., BMC Mol Biol 2007, 8;3
Renshaw et al., Mol Cancer Ther 2004, 3(11):1467-84
Giles et al., Antisense Nucleic Acid Drug Dev 1999, 9(2):213-20
Goto et al., J Invest Dermatol 2006, 126(12):2614-20
Disterer et al., Mol Ther 2013, 21(3):602-609
Uehara et al., FASEB J 2013, 27(1):76-85
Gedicke-Hornung et al., EMBO Mol Med 2013, 5(7):1060-77
Lentz et al., Nat Med 2013, 19(3):345-350
Taniguchi-Ikeda et al., Nature 2011, 478(7367):127-31
Owen et al., PLoS One 2012, 7(3):e33576
Zammarchi et al., PNAS 2011, 108(43):17779-84
Mercatante et al., J Biol Chem 2002, 277(51):49374-82
Osorio et al., Sci Transl Med 2011, 3(106):106ra107
Wein et al., Hum Mut 2010, 31(2):136-42
Gao et al., Cell Transplant 2008, 17(7):723-34
Peacey et al., NAR 2012, 40(19):9836-49
Wheeler et al., J Clin Invest 2007, 117(12):3952-7
Evers et al., Nucleic Acid Ther 2014, 24(1):4-12
van Ommen, van Deutekom, Aartsma-Rus, Curr Opin Mol Ther. 2008; 10(2):140-9.
Yokota, Duddy, Partidge, Acta Myol. 2007; 26(3):179-84.
van Deutekom et al., N Engl J Med. 2007; 357(26):2677-86.
Goemans et al., N Engl J Med. 2011; 364(16):1513-22.
Cirak et al., Lancet 2011; 378: 595-605.
Voit et al., Lancet Neurol 2014, 13(10):987-96
Heemskerk et al., Mol Ther 2010; 18(6):1210-7.
Evers et al. PLoS ONE 2011, 6 (9) e24308
Gao et al., Mol Ther Nucleic Acids 2015, 4:e255
Goyenvalle et al., Nat Med 2015, 21(3):270-5
Khoo and Krainer, Curr Opin Mol Ther 2009, 11(2):108-15
Singh et al., Mol Cell Biol 2006, 26(4):1333-46
Hua et al., Am J Hum Genet 2008, 82(4): 834-48
Hua et al., Genes Dev 2010, 24(15):1634-44
Hua et al., Nature 2011, 478(7367):123-6
Passini et al., Sci Transl Med 2011, 3(72):72ra18
Swoboda et al., J Clin Invest 2014, 124(2): 487-90
Chiriboga et al., Neurology 2016, 86(10):890-7
Braida C., et al, Human Molecular Genetics, 2010, vol 9: 1399-1412
Aartsma-Rus et al., Hum Mol Gen 2003; 12(8):907-14.
Yu RZ., Anal Biochem 2002; 304: 19-25.
Dorn and Kippenberger, Curr Opin Mol Ther 2008; 10(1): 10-20
Krieg AM. et al., Nature 1995; 374: 546-549.
Krieg, A. M., Curr. Opin. Immunol. 2000; 12: 35-43.
Han et al., Nature Communications, 2016, doi:10.1038/ncomms10981
Wagner, H., Adv. Immunol. 1999; 73: 329-368.
Popovic PJ. et al. J of Immunol 2006; 177: 8701-8707.
Diebold S.S., et.al., Eur J Immunol. 2006; Dec;36(12):3256-67.
Peacock H et al. J. Am. Chem. Soc. 2011, 133, 9200
Arai K et al.Bioorg. Med. Chem. 2011, 21, 6285
Ehmsen J. et al, J. Cell Sci. 2002, 115 (Pt14): 2801-2803.
Monaco A.P., et al. , Genomics 1988; 2: 90-95.
Manzur A.Y. et al., Wiley publishers, 2008. The Cochrane collaboration.
Hodgetts S., et al, Neuromuscular Disorders 2006; 16: 591-602.
Zuker M., et al, Nucleic Acids Res. 2003; 31(13):3406-15.
Cartegni L, et al, Nat Rev Genet 2002;3(4):285-98.
Cartegni L, et al, Nucleic Acids Res 2003;31(13):3568-71
Remington: The Science and Practice of Pharmacy, 20th Edition.
Baltimore, MD: Lippincott Williams & Wilkins, 2000
Kumar L, Pharm. Technol. 2008, 3, 128
Bruno, K., Advanced Drug Delivery Reviews 2011; 63: 1210.
doi: 10.1021/ja710342q
Seth et al., J. Org. Chem. 2010, 75, 1569-1581
doi: 10.1093/nass/1.1.241
doi: 10.1021/jo100170g
Osawa et al., J. Org. Chem., 2015, 80 (21), pp 10474-10481
WO 2014/145356 (MiRagen Therapeutics)
WO 2014/126229 (Mitsuoka Y et al.)
Yamamoto etal. Org. Biomol. Chem. 2015, 13, 3757
Nishida et al. Chem. Commun. 2010, 46, 5283
WO 2014/112463 (Obika S et al.)
WO 2015/142910 (lonis Pharmaceuticals)
Hanessian et al., J. Org. Chem., 2013, 78 (18), pp 9064-9075
Bolli et al., Chem Biol. 1996 Mar;3(3):197-206
DOI: 10.1021/jo402690j
Murray et al., Nucl. Acids Res., 2012, Vol. 40, No. 13 6135-6143
doi: 10.1021/acs.joc.5b00184
Nucleic Acids Res. 2004, 32, 5791-5799
WO 2011/097641 (ISIS/lonis Pharmaceuticals)
WO2016/017422 (Osaka University)
Cao et al., Mol. Ther. Nucleic Acids, 2016, doi:10.1038/mtna.2016.46
Spitali et al., FASEB J 2013, 27(12): 4909-4916
Horiba et al., J. Org. Chem. 2016, doi: 10.1021/acs.joc.6b02036
Shrestha et al., Chem. Commun. 2014, doi: 10.1039/C3CC46017G
WO2017/062862 (WaVe Life Sciences)
WO2016/028187 (Noogen)
Jirka et al., Nucl. Acid Ther. 2014, 24, 25
WO2013/030569
US9,161,948 (Sarepta)
WO2016/187425 (Sarepta)
Gao et al., Mol. Ther. 2014, 22, 1333
Shabanpoor et al., Nucl. Acids Ther. 2017, 27, 130
WO2017/062862 (Wave Life Sciences)
Ferrés-Coy et al. Mol. Psych. 2016, 21, 328
US 6,656,730 (ISIS/lonis Pharmaceuticals)
Noir et al., J. Am. Chem Soc. 2008, 130, 13500
Nakagawa et al., J. Am. Chem. Soc. 2010, 132, 8848
Dohmen et al., Mol. Ther. Nucl. Acids 2012, 1, e7
Zhao et al. Biomaterials 2015, 67, 42
WO2016/179257 (CytoMx)
Sugo et al., J. Control. Rel. 2016, 237, 1
Weisbart et al., Mol. Cancer Ther. 2012, 11, 1
Winkler et al., Ther. Deliv. 2013, 4, 791
Manoharan, Antisense Nucl. Acid. Dev. 2004, 12, 103
Ming et al., Adv. Drug Deliv. Rev. 2015, 87, 81

The following paragraphs form part of the description and not the claims. The paragraphs describe preferred features of the invention.
1. An oligonucleotide having a length from 10 to 33 nucleotides comprising:
   i) only 2'-substituted monomers linked by phosphorothioate backbone linkages and/or by phosphodiester linkages,
   ii) a 5-methylcytosine base, and
   iii) at least one monomer comprising a bicyclic nucleic acid (BNA) scaffold modification,
   wherein said oligonucleotide is complementary to or binds to or targets or hybridizes with at least a part of dystrophin pre-mRNA exons 2 to 78.
2. An oligonucleotide according to feature 1, wherein said oligonucleotide comprises a continuous stretch of at least 10 and up to 33 nucleotides of at least one of the nucleotide sequences selected from SEQ ID NO: 6065 to 6070, preferably of SEQ ID NO: 6067.
3. An oligonucleotide according to feature 1 or 2, wherein said oligonucleotide comprises 1, 2, 3, or 4 monomers that comprise a bicyclic nucleic acid (BNA) scaffold modification, preferably a bridged nucleic acid scaffold modification.
4. An oligonucleotide according to any one of features 1 to 3, wherein at least one bicyclic nucleic acid (BNA) scaffold modification is comprised in a terminal monomer of said oligonucleotide, preferably in the 5'-terminal monomer of said oligonucleotide, more preferably in both terminal monomers of said oligonucleotide.
5. An oligonucleotide according to any one of features 1 to 4, wherein each occurrence of said bicyclic nucleic acid (BNA) scaffold modification results in a monomer that is independently chosen from the group consisting of
   a locked nucleic acid (LNA) monomer, a conformationally restrained nucleotide (CRN) monomer, a xylo-LNA monomer, an α-L-LNA monomer, a β-D-LNA monomer, a 2'-amino-LNA monomer, a 2'-(alkylamino)-LNA monomer, a 2'-(acylamino)-LNA monomer, a 2'-N-substituted-2'-amino-LNA monomer, a (2'-O,4'-C) constrained ethyl (cEt) LNA monomer, a (2'-O,4'-C) constrained methoxyethyl (cMOE) BNA monomer, a 2',4'-BNA^{NC}(N-H) monomer, a 2',4'-BNA^{NC}(N-Me) monomer, an ethylene-bridged nucleic acid (ENA) monomer, a 2'-C-bridged bicyclic nucleotide (CBBN) monomer, and derivatives thereof,
   preferably chosen from the group consisiting of an LNA monomer, an ENA monomer, a cEt BNA monomer, an oxo-CBBN monomer, a 2'-amino-LNA monomer, and a cMOE BNA monomer,
   more preferably chosen from the group consisting of an LNA monomer, an ENA monomer, a cEt BNA monomer, or a cMOE BNA monomer,
   most preferably the BNA scaffold modification results in an LNA monomer.
6. An oligonucleotide according to any one of features 1 to 5, wherein said 2'-substituted monomer is a 2'-substituted RNA monomer, a 2'-F monomer, a 2'-amino monomer, a 2'-O-substituted monomer, a 2'-O-methyl monomer, or a 2'-O-(2-methoxyethyl) monomer, preferably a 2'-O-methyl monomer.
7. An oligonucleotide according to any one of features 1 to 6, wherein all cytosine bases are 5-methylcytosine bases, and/or wherein all uracil bases are 5-methyluracil bases.
8. An oligonucleotide according to any one of features 1 to 7, wherein the length of said oligonucleotide is less than 34 nucleotides.
9. An oligonucleotide according to any one of features 1 to 8, wherein said oligonucleotide comprises or consists of a sequence which is complementary to or binds or targets or hybridizes at least a part of an exon recognition sequence (ERS), an exonic splicing silencer (ESS), an intronic splicing silencer (ISS), an SR protein binding site, or another splicing element, signal, or structure.
10. An oligonucleotide according to any one of features 1 to 9, wherein said oligonucleotide induces pre-mRNA splicing modulation, preferably said pre-mRNA splicing modulation alters production or composition of protein, which preferably comprises exon skipping or exon inclusion, wherein said RNA modulation most preferably comprises exon skipping.
11. An oligonucleotide according to any one of features 1 to 10, wherein said oligonucleotide has an improved parameter by comparison to a corresponding oligonucleotide that does not comprise a bicyclic nucleic acid (BNA) scaffold modification.
12. An oligonucleotide according to any one of features 1 to 11, wherein:
   a) at least one monomer has formula I: 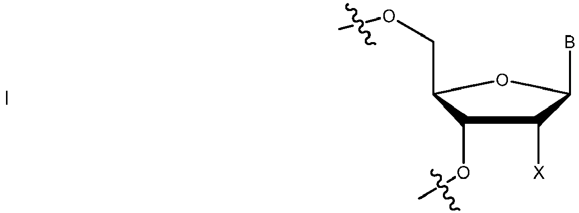 wherein:
      B is a nucleobase;
      X is F, -NR¹R², or-OR;
      R is alkenyl or optionally substituted alkyl, where the optional substituents, when present, are halo, OR¹, NR¹R², or SR¹;
      R¹ is H, alkyl, cycloalkyl, aryl, heterocycloalkyl, or heteroaryl, each independently optionally further substituted with halo, hydroxy, or alkyl;
      R² is H or alkyl; and 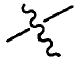
      indicates the point of attachment to the remainder of the oligonucleotide;
   b) wherein at least one monomer comprises a BNA scaffold modification and has formula II: 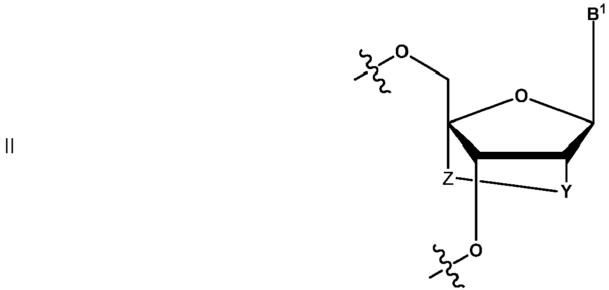 wherein:
      B¹ is a nucleobase;
      Z-Y is a divalent group selected from -(CH₂)ₙO-, -C(CHzCHz)O-, -CH₂WCH₂-, -(CH₂)ₙNR³-, -CH₂S(Oₘ)-, -CH(CH₃)O-, -CH(CH₂OCH₃)O-, -CH₂N(R³)O-, -CH₂CH₂-, -C(O)NR³-, -CH=CHO-, -CH₂SO₂NR³-, and -NHC(O)NH-;
      n is 1 or 2;
      m is 0, 1 or 2;
      W is O, S or NR³;
      R³ is H, -C(O)R⁴, -C(=NH)NR⁵R⁵, benzyl, or optionally substituted alkyl, where the optional substituents, when present, are selected from halo and alkoxy;
      R⁴ is alkyl, cycloalkyl or aryl;
      R⁵ is H or alkyl; and 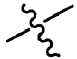
      indicates the point of attachment to the remainder of the oligonucleotide
13. A composition comprising an oligonucleotide as defined in any one of features 1 to 12, preferably wherein said composition comprises at least one excipient that may further aid in enhancing the targeting and/or delivery of said composition and/or said oligonucleotide to a tissue and/or cell and/or into a tissue and/or cell.
14. An oligonucleotide according to any one of features 1 to 12, or a composition according to feature 13, for use as a medicament, preferably for treating, preventing, and/or delaying Duchenne Muscular Dystrophy (DMD).
15. A method for preventing, treating, and/or delaying Duchenne Muscular Dystrophy (DMD), comprising administering to a subject an oligonucleotide as defined in any one of features 1 to 12, or a composition as defined in feature 13.

## Claims

1. An antisense oligonucleotide comprising the base sequence of any one of SEQ ID NOs: 8-452, 458, 464, 470, 476, 482, 488, 494, 500, 506, 512, 518, 524, 530, 536, 542, 548-1399, 1608-4529, 4561-6049, 6071, 6073-6084 and 6086-6092, wherein the antisense oligonucleotide has a fully phosphorothioate backbone, wherein the antisense oligonucleotide contains at least one 5-methylcytosine or 5-methyluracil base, and wherein the antisense oligonucleotide contains at least one BNA monomer.

2. The antisense oligonucleotide of claim 1, comprising the base sequence of any one of SEQ ID NOs: 452, 458, 464, 470, 476, 482, 488, 494, 500, 506, 512, 518, 524, 530, 536, 542, 548-613, 4561-4572, 6073 and 6086.

3. The antisense oligonucleotide of claim 2, wherein all cytosines are replaced by 5-methylcytosine.

4. The antisense oligonucleotide of claim 3, wherein the oligonucleotide has a length of 18, 19, 20, 21, or 22 nucleotides.

5. The antisense oligonucleotide of claim 4, wherein the oligonucleotide has a length of 18, 20, or 22 nucleotides.

6. The antisense oligonucleotide of claim 5, wherein said oligonucleotide comprises 1, 2, 3, or 4 LNA monomers.

7. The antisense oligonucleotide of claim 6, wherein the 5'-terminal position of the oligonucleotide is an LNA monomer.

8. The antisense oligonucleotide of claim 6, wherein the 3'-terminal position of the oligonucleotide is an LNA monomer.

9. The antisense oligonucleotide of claim 6, wherein the two monomers closest to the 5'-position of the oligonucleotide are LNA monomers.

10. The antisense oligonucleotide of claim 9 comprising the base sequence of any one of SEQ ID NOs: 591-601, 4565-4571, 6073 or 6086.

11. The antisense oligonucleotide of claim 10 comprising the base sequence of SEQ ID NO: 4568.

12. The antisense oligonucleotide of claim 1 comprising the base sequence GGUAAGUU*C*UGU*CC*AAG*C*, wherein *C*=5-methylcytosine, *C*=5-methylcytosinse LNA nucleotide and G=guanine LNA nucleotide.

13. A pharmaceutical composition, comprising the antisense oligonucleotide of any one of claims 1-12 and a pharmaceutically acceptable carrier.

14. A method of preventing, treating, and/or delaying the onset of Duchenne Muscular Dystrophy in a subject, comprising administering to the subject the antisense oligonucleotide of any one of claims 1-12 or the pharmaceutical composition of claim 13.
